(19) Europäisches Patentamt / European Patent Office / Office européen des brevets

(11) EP 2 513 339 B1

(12) **EUROPEAN PATENT SPECIFICATION**

(45) Date of publication and mention of the grant of the patent:
**09.09.2015 Bulletin 2015/37**

(21) Application number: **10844163.5**

(22) Date of filing: **01.12.2010**

(51) Int Cl.:
*C12Q 1/68* $^{(2006.01)}$ *C12P 19/34* $^{(2006.01)}$

(86) International application number:
**PCT/US2010/058606**

(87) International publication number:
**WO 2011/090556 (28.07.2011 Gazette 2011/30)**

(54) **METHODS FOR DETERMINING FRACTION OF FETAL NUCLEIC ACID IN MATERNAL SAMPLES**

VERFAHREN ZUR BESTIMMUNG DER FRAKTION EINER FETALEN NUCLEINSÄURE IN MATERIALPROBEN

PROCÉDÉS POUR DÉTERMINER UNE FRACTION D'ACIDE NUCLÉIQUE F TAL DANS DES ÉCHANTILLONS MATERNELS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.10.2010 US 455849 P**
**19.01.2010 US 296358 P**
**01.07.2010 US 360837 P**
**26.10.2010 US 407017 P**

(43) Date of publication of application:
**24.10.2012 Bulletin 2012/43**

(73) Proprietor: **Verinata Health, Inc.**
**Redwood City CA 94063 (US)**

(72) Inventors:
- **RAVA, Richard, P.**
  **Redwood City, CA 94062 (US)**
- **CHUU, Yue-Jen**
  **Cupertino, CA 95014 (US)**
- **CHINNAPPA, Manjula**
  **Foster City, CA 94404 (US)**
- **COMSTOCK, David, A.**
  **San Francisco, CA 94107 (US)**
- **HEILEK, Gabrielle**
  **Mountain View, CA 94043 (US)**
- **HUNKAPILLER, Michael**
  **San Carlos, CA 94070 (US)**

(74) Representative: **Chapman, Desmond Mark et al**
**Carpmaels & Ransford LLP**
**One Southampton Row**
**London WC1B 5HA (GB)**

(56) References cited:

| | |
|---|---|
| **EP-A1- 2 496 717** | **WO-A2-2007/100911** |
| **WO-A2-2007/147074** | **US-A1- 2007 134 658** |
| **US-A1- 2008 020 390** | **US-A1- 2008 050 739** |
| **US-A1- 2008 064 098** | **US-A1- 2008 070 792** |

- **LI YING ET AL: "Size separation of circulatory DNA in maternal plasma permits ready detection of fetal DNA polymorphisms", CLINICAL CHEMISTRY, AMERICAN ASSOCIATION FOR CLINICAL CHEMISTRY, WASHINGTON, DC, vol. 50, no. 6, 1 June 2004 (2004-06-01), pages 1002-1011, XP002510472, ISSN: 0009-9147, DOI: 10.1373/CLINCHEM.2003.029835**
- **CHIU R W K ET AL: "Non-invasive prenatal diagnosis by single molecule counting technologies", TRENDS IN GENETICS, ELSEVIER SCIENCE PUBLISHERS B.V. AMSTERDAM, NL, vol. 25, no. 7, 1 July 2009 (2009-07-01), pages 324-331, XP026301457, ISSN: 0168-9525, DOI: 10.1016/J.TIG.2009.05.004 [retrieved on 2009-06-18]**
- **FAN H C ET AL: "Noninvasive diagnosis of fetal aneuploidy by shotgun sequencing DNA from maternal blood", PROCEEDINGS OF THE NATIONAL ACADEMY OF SCIENCES, NATIONAL ACADEMY OF SCIENCES, US, vol. 105, no. 42, 21 October 2008 (2008-10-21), pages 16266-16271, XP002613056, ISSN: 0027-8424, DOI: 10.1073/PNAS.0808319105 [retrieved on 2008-10-06]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 513 339 B1

- T. CHU ET AL: "Statistical model for whole genome sequencing and its application to minimally invasive diagnosis of fetal genetic disease", BIOINFORMATICS, vol. 25, no. 10, 23 March 2009 (2009-03-23), pages 1244-1250, XP055018601, ISSN: 1367-4803, DOI: 10.1093/bioinformatics/btp156
- NICKLAS ET AL.: ’A Real-Time Multiplex SNP Melting Assay to Discriminate Individuals.’ J FORENSIC SCI vol. 53, no. 6, November 2008, pages 1316 - 1324

**Description**

## FIELD OF THE INVENTION

The present invention relates to methods and compositions for detecting fetal nucleic acids in a maternal sample and determining the fraction of cell-free fetal nucleic acid circulating in a maternal sample.

## BACKGROUND OF THE INVENTION

Invasive prenatal tests are potentially harmful to the mother and to the fetus. Therefore, there is a need for the development of noninvasive prenatal tests. Maternal blood can contain fetal cells (see e.g., U.S. Patent Application Publication No. 20080070792) and cell-free fetal DNA (see e.g., Huang et al. (2008), Methods in Molecular Biology, 444:203-208). While circulating fetal cells present an attractive target for non invasive prenatal diagnostics, particularly for the diagnosis of fetal sex and chromosomal abnormalities by simple karyotyping, the scarcity of intact fetal cells in the maternal circulation (around one cell per ml of maternal blood), low efficiency of enrichment *(*Bianchi et al., Am J Hum Genet 61:822-829 [1997]) and difficulties with chromosomal analysis associated with abnormally dense nuclei in some cells (Babochkina et al., Haematologica 90:740-745 [2005]), have favored research on cell-free DNA.

The establishment of the concentrations of cell-free fetal DNA (cfDNA) in maternal plasma in healthy pregnant women has formed the platform on which fetal DNA abnormalities in pregnancy-associated disorders can be studied. The finding of a gradual increase in fetal DNA concentration in maternal serum as gestation progresses has been shown to precede complications associated with preterm labor. A five-fold increase in fetal DNA concentration has also been found in the serum obtained from women affected by preeclampsia. Other pregnancy-related disorders that have been linked to an elevated concentration of cfDNA include hyperemesis gravidarum (severe morning sickness), invasive placentation (in which the placenta contacts the maternal bloodstream), intrauterine growth restriction, feto-maternal haemorrhage and polyhydramnios. (Wright C.F. and Burton H., Human Reproduction Update 15(1):139-151 [2009]).

Quantitative analysis of cell free DNA by real-time PCR strategies has also indicated that the concentrations of circulatory fetal DNA are increased in pregnancies with fetal aneuploidies, most notably trisomy 21 (Lo et al., Clin Chem 45:1747-1751 [1999]). However, the fraction of fetal DNA in maternal cell-free plasma DNA is usually determined by comparing the amount of fetal-specific locus (such as the SRY locus on chromosome Y in male pregnancies) to that of a locus on any autosome that is common to both the mother and the fetus by using quantitative real-time PCR (Dahllan et al., Lancet 369:474-481 [2007]; Li et al., Clin Chem 1002-1011 [2004]; Fan et al., Proc Natl Acad Sci 105:16266-16271 [2008]).

Thus, there is a need for additional methods that would enable the determination of the fraction of fetal nucleic acid in both male and female pregnancies.

The method of the invention fulfills the need in providing the means to determine fetal fraction that is independent of the gender of the fetus. The method can be applied for determining simultaneously the presence or absence of a chromosomal aneuploidy or other copy number variation, and may be used in conjunction with nay known methods that are used to determine aneuploidies in maternal sample.

## SUMMARY OF THE INVENTION

The invention relates to compositions and methods for determining the fraction of fetal nucleic acids in a maternal sample comprising a mixture of fetal and maternal nucleic acids. The fraction of fetal nucleic acids can be used in determining the presence or absence of fetal aneuploidy.

The invention provides a method for determining the fraction of fetal nucleic acids in a maternal blood sample comprising a mixture of fetal and maternal genomic DNA, wherein said fetal and maternal genomic DNA is cell-free DNA (cfDNA), said method comprising: (a) amplifying a plurality of polymorphic target nucleic acids in said mixture, wherein each amplified polymorphic target nucleic acid comprises at least one polymorphic site, using primer pairs each capable of amplifying a target nucleic acid sequence comprising a polymorphic site in a multiplex PCR reaction, to generate a panel of amplified polymorphic sites that contains a sufficient number of polymorphic sites such that at least 25 are informative polymorphic sites; (b) performing massively parallel sequencing of at least a portion of the amplified product obtained in (a), wherein said sequencing comprises providing a plurality of sequence tags for said panel of amplified polymorphic sites; and (c) using said plurality of sequence tags to: (i) identify at least one informative polymorphic site in said panel of amplified polymorphic sites, wherein said at least one informative polymorphic site is identified by the difference in the allelic sequences at each polymorphic site and the number of sequence tags mapped to each of the possible alleles at each polymorphic site, and (ii) determine said fraction of fetal nucleic acids using the total number of tags that map to a first allele at an identified polymorphic site and the total number of tags that map to a second allele at the identified informative polymorphic site to calculate said fraction of fetal nucleic acids.

In one embodiment, there is disclosed herein a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction. The plurality of polymorphic nucleic acids are located on a plurality of different chromosomes. In one embodiment, the plurality of polymorphic nucleic acids can be located on chromosomes 1-22. In another embodiment, the plurality of polymorphic nucleic acids can be located on different autosomes other than chromosomes 13, 18 and 21. The maternal sample is selected from blood, plasma, serum, urine and saliva. Preferably, the maternal sample is a plasma sample.

In one embodiment, there is disclosed herein a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22 For example, the plurality of polymorphic nucleic acids can be located on a plurality of different chromosomes other than chromosomes 13, 18,21, X or Y. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality

of polymorphic nucleic acids can be located on chromosomes 1-22. For example, the plurality of polymorphic nucleic acids can be located on a plurality of different chromosomes other than chromosomes 13, 18,21, X or Y. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In one embodiment, there is disclosed herein a method for determining the fraction of fetal nucleic acids in a maternal sample comprising a mixture of fetal and maternal genomic DNA *e.g*. cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18,21, X or Y. The maternal sample is selected from blood, plasma, serum, urine and saliva. Preferably, the maternal sample is a plasma sample.

In one embodiment, there is disclosed herein a method for determining the fraction of fetal nucleic acids in a maternal sample comprising a mixture of fetal and maternal genomic DNA *e.g*. cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13,18, 21, X or Y. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13,18,21,XorY. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA *e.g*. cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes.

In one embodiment, the plurality of polymorphic nucleic acids can be located on chromosomes 1-22. For example, the plurality of polymorphic nucleic acids can be located on a plurality of different chromosomes other than chromosomes 13, 18,21, X or Y. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In one embodiment, there is disclosed herein a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In one embodiment, there is disclosed herein a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18,21, X or Y. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference

genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In one embodiment, there is disclosed herein a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In one embodiment, there is disclosed herein a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal

sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18,21, X or Y. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). The massively parallel sequencing is sequencing-by-synthesis with reversible dye terminators. Alternatively, the massively parallel sequencing is sequencing-by-ligation or single molecule sequencing. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13,18, 21, X or Y. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA *e.g*. cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in

(a), wherein sequencing comprises providing a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989- rs13047336; rs987980- rs987981; rs4143392- rs4143391; rs1691324- rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102- rs458848; rs961301-rs2830208; rs2174536-rs458076; rs1088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989- rs13047336; rs987980- rs987981; rs4143392- rs4143391; rs1691324- rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102- rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal

genomic DNA; (b) performing massively parallel sequencing of at least a portion of the amplified product obtained in (a), wherein sequencing comprises an amplification and provides a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980-rs987981; rs4143392-rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102- rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980-rs987981; rs4143392-rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102- rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In one embodiment, there is disclosed herein a method for determining the fraction of fetal nucleic acids in a

maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980- rs987981; rs4143392- rs4143391; rs1691324- rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102- rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; ra11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSFIPO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980- rs987981; rs4143392- rs4143391; rs1691324- rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703;

rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989- rs13047336; rs987980- rs987981; rs4143392- rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102- rs458848; rs961301-rs2830208; rs2174536-rs458076; rs11088023-rs11088024; rs10111734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989- rs13047336; rs987980- rs987981; rs4143392- rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848;

rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088;rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In one embodiment, there is disclosed herein a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid.. The maternal sample is selected from blood, plasma, serum, urine and saliva. Preferably, the maternal sample is a plasma sample.

In one embodiment, there is disclosed herein a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989- rs13047336; rs987980- rs987981; rs4143392- rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D,

Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises providing a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989- rs13047336; rs987980- rs987981; rs4143392- rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989- rs13047336; rs987980- rs987981; rs4143392- rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102- rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316;

rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The maternal sample is selected from blood, plasma, serum, urine and saliva. Preferably, the maternal sample is a plasma sample.

In another embodiment, there is disclosed herein a method for determining the fraction of fetal nucleic acids in a maternal sample comprising a mixture of fetal and maternal genomic DNA *e.g.* cell-free DNA, wherein the method comprises the steps of: (a) amplifying a plurality of polymorphic target nucleic acids in the mixture of fetal and maternal genomic DNA; (b) performing massively parallel sequencing of at least a portion of the amplified product obtained in (a), wherein sequencing comprises an amplification and provides a plurality of sequence tags; and (c) based on the sequencing, determining the fraction, wherein determining the fraction comprises determining the number of fetal and maternal sequence tags mapped to a reference genome comprising of at least one polymorphic nucleic acid.. The plurality of polymorphic target nucleic acids comprises at least one single nucleotide polymorphism (SNP). Alternatively, the plurality of polymorphic target nucleic acids comprises at least one short tandem repeat (STR). In some embodiments, the at least one SNP is a single SNP selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989- rs13047336; rs987980- rs987981; rs4143392- rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. The massively parallel sequencing is sequencing-by-synthesis with reversible dye terminators. Alternatively, the massively parallel sequencing is sequencing-by-ligation or single molecule sequencing. The maternal sample is selected from blood, plasma, serum, urine and saliva. Preferably, the maternal sample is a plasma sample.

In another embodiment, there is disclosed herein a method for determining the fraction of fetal nucleic acids in a maternal plasma sample comprising a mixture of fetal and maternal genomic DNA, wherein the method comprises the steps of: (a) amplifying a plurality of polymorphic nucleic acids in said mixture of fetal and maternal nucleic acids, wherein each of said at least one polymorphic nucleic acid comprises an STR; (b) determining the amount of fetal and maternal STR alleles at least one polymorphic nucleic acid; and (c)determining said fraction using said amount of fetal and maternal STR alleles. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The method is a fetal gender-independent method.

In another embodiment, there is disclosed herein a method for determining the fraction of fetal nucleic acids in a maternal plasma sample comprising a mixture of fetal and maternal genomic DNA, wherein the method comprises the steps of: (a) amplifying a plurality of polymorphic nucleic acids in said mixture of fetal and maternal nucleic acids, wherein each of said at least one polymorphic nucleic acid comprises an STR; (b) determining the amount of fetal and maternal STR alleles at least one polymorphic nucleic acid; and (c)determining said fraction using said amount of fetal and maternal STR alleles. The plurality of polymorphic nucleic acids are located on a plurality of different chromosomes. In one embodiment, the plurality of polymorphic nucleic acids can be located on chromosomes 1-22. For example, the plurality of polymorphic nucleic acids are located on a plurality of different chromosomes other than chromosomes 13,18,

21, X or Y. The method further comprises preamplifying the mixture of fetal and maternal nucleic acids. The method is a fetal gender-independent method.

In another embodiment, there is disclosed herein a method for determining the fraction of fetal nucleic acids in a maternal plasma sample comprising a mixture of fetal and maternal genomic DNA, wherein the method comprises the steps of: (a) amplifying a plurality of polymorphic nucleic acids in said mixture of fetal and maternal nucleic acids, wherein each of said at least one polymorphic nucleic acid comprises an STR; (b) determining the amount of fetal and maternal STR alleles at least one polymorphic nucleic acid; and (c)determining said fraction using said amount of fetal and maternal STR alleles. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The method further comprises resolving the size of the STRs using capillary electrophoresis. The method is a fetal gender-independent method.

In another embodiment, there is disclosed herein a method for determining the fraction of fetal nucleic acids in a maternal plasma sample comprising a mixture of fetal and maternal genomic DNA, wherein the method comprises the steps of: (a) amplifying a plurality of polymorphic nucleic acids in said mixture of fetal and maternal nucleic acids, wherein each of said at least one polymorphic nucleic acid comprises an STR; (b) determining the amount of fetal and maternal STR alleles at least one polymorphic nucleic acid; and (c)determining said fraction using said amount of fetal and maternal STR alleles. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The method further comprises preamplifying the mixture of fetal and maternal nucleic acids, and resolving the size of the STRs using capillary electrophoresis. The method is a fetal gender-independent method.

In another embodiment, there is disclosed herein a method for determining the fraction of fetal nucleic acids in a maternal plasma sample comprising a mixture of fetal and maternal genomic DNA, wherein the method comprises the steps of: (a) amplifying a plurality of polymorphic nucleic acids in said mixture of fetal and maternal nucleic acids, wherein each of said at least one polymorphic nucleic acid comprises an STR; (b) determining the amount of fetal and maternal STR alleles at least one polymorphic nucleic acid; and (c)determining said fraction using said amount of fetal and maternal STR alleles. The plurality of polymorphic nucleic acids can be located on a plurality of different chromosomes. For example, the plurality of polymorphic nucleic acids are located on a plurality of different chromosomes other than chromosomes 13, 18, 21, X or Y. The at least one STR is selected from CSF1PO, FGA, TH01, vWA, D3S1358, D5S818, D7S820, D8S1179, D13S317, D16S539, D18S51, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. Optionally, the at least one STR can be the panel of STRs comprising CSF1PO, D13S317, D16S539, D18S51, D21S11, D2S1338, D7S820 and FGA. The method is a fetal gender-independent method.

In another embodiment, there is disclosed herein a method for determining the fraction of fetal nucleic acids in a maternal plasma sample comprising a mixture of fetal and maternal genomic DNA, wherein the method comprises the steps of: (a) amplifying a plurality of polymorphic nucleic acids in said mixture of fetal and maternal nucleic acids, wherein each of said at least one polymorphic nucleic acid comprises an STR; (b) determining the amount of fetal and maternal STR alleles at least one polymorphic nucleic acid; and (c)determining said fraction using said amount of fetal and maternal STR alleles. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The method further comprises preamplifying the mixture of fetal and maternal nucleic acids. The at least one STR is selected from CSF1PO, FGA, TH01, vWA, D3S1358, D5S818, D7S820, D8S1179, D13S317, D16S539, D18S51, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. Optionally, the at least one STR can be the panel of STRs comprising CSF1PO, D13S317, D16S539, D18S51, D21S11, D2S1338, D7S820 and FGA. The method is a fetal gender-independent method.

In another embodiment, there is disclosed herein a method for determining the fraction of fetal nucleic acids in a maternal plasma sample comprising a mixture of fetal and maternal genomic DNA, wherein the method comprises the steps of: (a) amplifying a plurality of polymorphic nucleic acids in said mixture of fetal and maternal nucleic acids, wherein each of said at least one polymorphic nucleic acid comprises an STR; (b) determining the amount of fetal and maternal STR alleles at least one polymorphic nucleic acid; and (c)determining said fraction using said amount of fetal and maternal STR alleles. The plurality of polymorphic nucleic acids are located on a plurality of different chromosomes. In one embodiment, the plurality of polymorphic nucleic acids can be located on chromosomes 1-22. For example, the plurality of polymorphic nucleic acids are located on a plurality of different chromosomes other than chromosomes 13,

18, 21, X or Y. The method further comprises resolving the size of the STRs using capillary electrophoresis. The at least one STR is selected from CSF1PO, FGA, TH01, vWA, D3S1358, D5S818, D7S820, D8S1179, D13S317, D16S539, D18S51, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. Optionally, the at least one STR can be the panel of STRs comprising CSF1PO, D13S317, D16S539, D18S51, D21S11, D2S1338, D7S820 and FGA.

In another embodiment, there is disclosed herein a method for determining the fraction of fetal nucleic acids in a maternal plasma sample comprising a mixture of fetal and maternal genomic DNA, wherein the method comprises the steps of: (a) amplifying a plurality of polymorphic nucleic acids in said mixture of fetal and maternal nucleic acids, wherein each of said at least one polymorphic nucleic acid comprises an STR; (b) determining the amount of fetal and maternal STR alleles at least one polymorphic nucleic acid; and (c)determining said fraction using said amount of fetal and maternal STR alleles. The plurality of polymorphic nucleic acids are located on a plurality of different chromosomes. In one embodiment, the plurality of polymorphic nucleic acids can be located on chromosomes 1-22. For example, the plurality of polymorphic nucleic acids can be located on a plurality of different chromosomes other than chromosomes 13, 18, 21, X or Y. The method further comprises preamplifying the mixture of fetal and maternal nucleic acids, and resolving the size of the STRs using capillary electrophoresis. The at least one STR is selected from CSF1PO, FGA, TH01, vWA, D3S1358, D5S818, D7S820, D8S1179, D13S317, D16S539, D18S51, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. Optionally, the at least one STR can be the panel of STRs comprising CSF1PO, D13S317, D16S539, D18S51, D21S11, D2S1338, D7S820 and FGA.

## BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

**Figure 1** is a flowchart of a method 100 for determining the fetal fraction in a maternal test sample comprising a mixture of fetal and maternal nucleic acids using massively parallel sequencing methods or size separation of polymorphic nucleic acid sequences.

**Figure 2** is a bar diagram showing the identification of fetal and maternal polymorphic sequences (SNPs) used to determine fetal fraction in a test sample. The total number of sequence reads (Y-axis) mapped to the SNP sequences identified by rs numbers (X-axis), and the relative level of fetal nucleic acids (*) are shown.

**Figure 3** is a flowchart outlining alternative embodiments of the method for determining fetal fraction by massively parallel sequencing shown in **Figure 1.**

**Figure 4** illustrates STR markers used in the AmpFℓSTR® Identifiler® PCR Amplification Kit.

**Figure 5** illustrates STR markers used in the AmpFℓSTR® MiniFiler® PCR Amplification Kit.

**Figure 6** illustrates the correlation of fetal fraction determined by massively parallel sequencing size separation of polymorphic sequences comprising SNPs and STRs.

**Figure 7** illustrates an embodiment of use of fetal fraction for determining cutoff thresholds for aneuploidy detection.

## DETAILED DESCRIPTION OF THE INVENTION

The invention relates to methods for determining the fraction of fetal nucleic acids in a maternal sample comprising a mixture of fetal and maternal nucleic acids. The fraction of fetal nucleic acids can be used in determining the presence or absence of fetal aneuploidy.

Unless otherwise indicated, the practice of the present invention involves conventional techniques commonly used in molecular biology, microbiology, protein purification, protein engineering, protein and DNA sequencing, and recombinant DNA fields, which are within the skill of the art. Such techniques are known to those of skill in the art and are described in numerous standard texts and reference works.

Numeric ranges are inclusive of the numbers defining the range. It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader

numerical range, as if such narrower numerical ranges were all expressly written herein.

The headings provided herein are not limitations of the various aspects or embodiments of the invention which can be had by reference to the Specification as a whole. Accordingly, as indicated above, the terms defined immediately below are more fully defined by reference to the specification as a whole.

Unless defined otherwise herein, all technical and scientific terms used herein have the same meaning as commonly understood by one of ordinary skill in the art to which this invention belongs. Various scientific dictionaries that include the terms included herein are well known and available to those in the art. Although any methods and materials similar or equivalent to those described herein find use in the practice or testing of the present invention, some preferred methods and materials are described. Accordingly, the terms defined immediately below are more fully described by reference to the Specification as a whole. It is to be understood that this invention is not limited to the particular methodology, protocols, and reagents described, as these may vary, depending upon the context they are used by those of skill in the art.

## DEFINITIONS

As used herein, the singular terms "a", "an," and "the" include the plural reference unless the context clearly indicates otherwise. Unless otherwise indicated, nucleic acids are written left to right in 5' to 3' orientation and amino acid sequences are written left to right in amino to carboxy orientation, respectively.

As used herein, the singular terms "a", "an," and "the" include the plural reference unless the context clearly indicates otherwise. Unless otherwise indicated, nucleic acids are written left to right in 5' to 3' orientation and amino acid sequences are written left to right in amino to carboxy orientation, respectively.

The term "portion" when used in reference to the amount of sequence information of fetal and maternal nucleic acid molecules in a biological sample herein refers to the amount of sequence information of fetal and maternal nucleic acid molecules in a biological sample that in sum amount to less than the sequence information of <1 human genome.

The terms "polynucleotide", "nucleic acid" and "nucleic acid molecules" are used interchangeably and refer to a covalently linked sequence of nucleotides (i.e., ribonucleotides for RNA and deoxyribonucleotides for DNA) in which the 3' position of the pentose of one nucleotide is joined by a phosphodiester group to the 5' position of the pentose of the next, include sequences of any form of nucleic acid, including, but not limited to RNA, DNA and cfDNA molecules. The term "polynucleotide" includes, without limitation, single- and doublestranded polynucleotide.

The term "copy number variation" herein refers to variation in the number of copies of a nucleic acid sequence that is 1 kb or larger present in a test sample in comparison with the copy number of the nucleic acid sequence present in a qualified sample. A "copy number variant" refers to the 1 kb or larger sequence of nucleic acid in which copy-number differences are found by comparison of a sequence of interest in test sample with that present in a qualified sample. Copy number variants/variations include deletions, including microdeletions, insertions, including microinsertions, duplications, multiplications, inversions, translocations and complex multisite variants. CNV encompass chromosomal aneuploidies and partial aneuplodies.

As used herein, the term "fetal fraction" is used interchangeably with "fraction of fetal nucleic acid", which refers to the fraction of fetal nucleic acid in a sample comprising fetal and maternal nucleic acid. Similarly, the term "minor fraction" or "minor component" herein refers to the lesser fraction of the total genetic material that is present in a sample containing genetic material derived from separate sources *e.g.* individuals.

As used herein the term "allele" refers to one specific form of a genetic sequence (such as a gene) within a cell, a sample, an individual or within a population, the specific form differing from other forms of the same gene in the sequence of at least one, and frequently more than one, variant sites within the sequence of the gene. The sequences at these variant sites that differ between different alleles are termed "variants", "polymorphisms", or "mutations". In general, polymorphism is used to refer to variants that have a frequency of at least 1% in a population, while the term mutation is generally used for variants that occur at a frequency of less than 1 % in a population. In diploid organisms such as humans, at each autosomal specific chromosomal location or "locus" an individual possesses two alleles, a first inherited from one parent and a second inherited from the other parent, for example one from the mother and one from the father. An individual is "heterozygous" at a locus if it has two different alleles at the locus. An individual is "homozygous" at a locus if it has two identical alleles at that locus.

The term "enrich" herein refers to the process of amplifying polymorphic target nucleic acids contained in a portion of a maternal sample, and combining the amplified product with the remainder of the maternal sample from which the portion was removed.

As used herein, the term "genotyping" refers to the determination of the genetic information an individual carries at one or more positions in the genome. For example, genotyping may comprise the determination of which allele or alleles an individual carries for a single SNP or the determination of which allele or alleles an individual carries for a plurality of SNPs. For example, a particular nucleotide in a genome may be a T in some individuals and a C in other individuals. Those individuals who have a T at the position have the T allele and those who have a C have the C allele.

In a diploid organism the individual will have two copies of the sequence containing the polymorphic position so the individual may have a T allele and a C allele or alternatively two copies of the T allele or two copies of the C allele. Those individuals who have two copies of the C allele are homozygous for the C allele, those individuals who have two copies of the T allele are homozygous for the T allele, and those individuals who have one copy of each allele are heterozygous. The alleles are often referred to as the A allele, often the major allele, and the B allele, often the minor allele. The genotypes may be AA (homozygous A), BB (homozygous B) or AB (heterozygous). Genotyping methods generally provide for identification of the sample as AA, BB or AB.

As used herein the term "chromosome" refers to the heredity-bearing gene carrier of a living cell which is derived from chromatin and which comprises DNA and protein components (especially histones). The conventional internationally recognized individual human genome chromosome numbering system is employed herein. The size of an individual chromosome can vary from one type to another with a given multi-chromosomal genome and from one genome to another. In the case of the human genome, the entire DNA mass of a given chromosome is usually greater than about 100,000,000 bp. For example, the size of the entire human genome is about 3.times.10.sup.9 bp. The largest chromosome, chromosome no. 1, contains about 2.4.times.10.sup.8 by while the smallest chromosome, chromosome no. 22, contains about 5.3.times.10.sup.7 bp.

The term "aneuploidy" herein refers to the occurrence of one or more extra or missing chromosomes.

As used herein the term "chromosomal region" is a portion of a chromosome. The actual physical size or extent of any individual chromosomal region can vary greatly. The term "region" is not necessarily definitive of a particular one or more genes because a region need not take into specific account the particular coding segments (exons) of an individual gene.

As used herein the term "genetic marker" refers to a sequence of DNA that has a specific location on a chromosome that can be measured in a laboratory. The term "genetic marker" can also be used to refer to, e.g., a cDNA and/or an mRNA encoded by a genomic sequence, as well as to that genomic sequence. To be useful, a marker needs to have two or more alleles or variants. Markers can be either direct, that is, located within the gene or locus of interest (i.e., candidate gene), or indirect, that is closely linked with the gene or locus of interest (presumably due to a location which is proximate to, but not inside the gene or locus of interest). Moreover, markers can also include sequences which either do or do not modify the amino acid sequence of a gene.

As used herein, the term "maternal sample" refers to a biological sample obtained from a pregnant subject, and comprises a mixture of fetal and maternal nucleic acids. A "pregnant subject" is not limited to a human being, but may also include other organisms including but not limited to mammals, plants, bacteria or cells derived from any of the above.

The term "whole genome amplification" or "WGA" as used herein generally refers to a method for amplification of a limited DNA sample in a non-specific manner, in order to generate a new sample that is indistinguishable from the original but with a higher DNA concentration. The ideal whole genome amplification technique would amplify a sample up to a microgram level while maintaining the original sequence representation. The DNA of the sample may include an entire genome or a portion thereof. Degenerate oligonucleotide-primed PCR (DOP), primer extension PCR technique (PEP) including modified improved primer extension preamplification (mIPEP), and multiple displacement amplification (MDA), are examples of whole genome amplification methods.

The term "short tandem repeat" or "STR" as used herein refers to a class of polymorphisms that occurs when a pattern of two or more nucleotides are repeated and the repeated sequences are directly adjacent to each other. The pattern can range in length from 2 to 10 base pairs (bp) (for example (CATG)n in a genomic region) and is typically in the non-coding intron region. By examining several STR loci and counting how many repeats of a specific STR sequence there are at a given locus, it is possible to create a unique genetic profile of an individual.

The term "primer," as used herein refers to an isolated oligonucleotide which is capable of acting as a point of initiation of synthesis when placed under conditions in which synthesis of a primer extension product which is complementary to a nucleic acid strand is induced, (i.e., in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer, use of the method, and the parameters used for primer design, as disclosed herein.

The term "primer pair" or "primer set" refers to a set of primers including a 5' "upstream primer" or "forward primer" that hybridizes with the complement of the 5' end of the DNA sequence to be amplified and a 3' "downstream primer" or "reverse primer" that hybridizes with the 3' end of the sequence to be amplified. As will be recognized by those of skill in the art, the terms "upstream" and "downstream" or "forward" and "reverse" are not intended to be limiting, but rather provide illustrative orientation in particular embodiments. A primer pair is said to be "unique" if it can be employed to specifically amplify a particular target nucleotide sequence in a given amplification mixture.

A "polymorphic marker" or "polymorphic site" is a locus at which nucleotide sequence divergence occurs. The locus may be as small as one base pair. Illustrative markers have at least two alleles, each occurring at frequency of greater than 1%, and more typically greater than 10% or 20% of a selected population. A polymorphic site may be as small as one base pair. Polymorphic markers include restriction fragment length polymorphism (RFLPs), variable number of tandem repeats (VNTR's), hypervariable regions, minisatellites, dinucleotide repeats, trinucleotide repeats, tetranucleotide repeats, simple sequence repeats, deletions, and insertion elements such as Alu. The first identified allelic form is arbitrarily designated as the reference form and other allelic forms are designated as alternative or variant alleles. The allelic form occurring most frequently in a selected population is sometimes referred to as the wild type form. Diploid organisms may be homozygous or heterozygous for allelic forms. A diallelic polymorphism has two forms. A triallelic polymorphism has three forms. A polymorphism between two nucleic acids can occur naturally, or be caused by exposure to or contact with chemicals, enzymes, or other agents, or exposure to agents that cause damage to nucleic acids, for example, ultraviolet radiation, mutagens or carcinogens. The terms "polymorphic locus" and "polymorphic site" are herein used interchangeably.

The terms "polymorphic target nucleic acid", "polymorphic sequence", "polymorphic target nucleic acid sequence" and "polymorphic nucleic acid" are used interchangeably herein to refer to a nucleic acid sequence *e.g.* a DNA sequence, that comprises one or more polymorphic sites *e.g* one SNP or a tandem SNP. Polymorphic sequences according to the present technology can be used to specifically differentiate between maternal and non-maternal alleles in the maternal sample comprising a mixture of fetal and maternal nucleic acids.

A "single nucleotide polymorphism" (SNP) occurs at a polymorphic site occupied by a single nucleotide, which is the site of variation between allelic sequences. The site is usually preceded by and followed by highly conserved sequences of the allele (e.g., sequences that vary in less than 1/100 or 1/1000 members of the populations). A SNP usually arises due to substitution of one nucleotide for another at the polymorphic site. A transition is the replacement of one purine by another purine or one pyrimidine by another pyrimidine. A transversion is the replacement of a purine by a pyrimidine or vice versa. SNPs can also arise from a deletion of a nucleotide or an insertion of a nucleotide relative to a reference allele. Single nucleotide polymorphisms (SNPs) are positions at which two alternative bases occur at appreciable frequency (>1%) in the human population, and are the most common type of human genetic variation.

As used herein, the term "short tandem repeat" or "STR" as used herein refers to a class of polymorphisms that occurs when a pattern of two or more nucleotides are repeated and the repeated sequences are directly adjacent to each other. The pattern can range in length from 2 to 10 base pairs (bp) (for example (CATG)n in a genomic region) and is typically in the non-coding intron region. By examining several STR loci and counting how many repeats of a specific STR sequence there are at a given locus, it is possible to create a unique genetic profile of an individual.

As used herein, the term "miniSTR" herein refers to tandem repeat of four or more base pairs that spans less than about 300 base pairs, less than about 250 base airs, less than about 200 base pairs, less than about 150 base pairs, less than about 100 base pairs, less than about 50 base pairs, or less than about 25 base pairs. "miniSTRs" are STRs that are amplifiable from cfDNA templates.

The term "tandem SNPs" herein refers to two or more SNPs that are present within a polymorphic target nucleic acid sequence.

The terms "plurality of polymorphic target nucleic acids", "polymorphic nucleic acids" and "polymorphic sequences" are used interchangeably herein and refer to a number of nucleic acid sequences each comprising at least one polymorphic site *e.g.* one SNP, such that at least 1, 2, 3, 4, 5, 6, 7, 8,9, 10, 15, 20, 25, 30, or 40 or more different polymorphic sites are amplified from the polymorphic target nucleic acids to identify and/or quantify fetal alleles present in maternal samples comprising fetal and maternal nucleic acids.

As used herein, the term "substantially cell free" encompasses preparations of the desired sample from which components that are normally associated with it are removed. For example, a plasma sample is rendered essentially cell free by removing blood cells *e.g.* red cells, that are normally associated with it. In some embodiments, substantially free samples are processed to remove cells that would otherwise contribute to the desired genetic material that is to be tested for an abnormality

As used herein the term "chromosome" refers to the heredity-bearing gene carrier of a living cell which is derived from chromatin and which comprises DNA and protein components (especially histones). The conventional internationally recognized individual human genome chromosome numbering system is employed herein. The size of an individual chromosome can vary from one type to another with a given multi-chromosomal genome and from one genome to another. In the case of the human genome, the entire DNA mass of a given chromosome is usually greater than about 100,000,000 bp. For example, the size of the entire human genome is about 3.times.10.sup.9 bp. The largest chromosome, chromosome no. 1, contains about 2.4.times.10.sup.8 by while the smallest chromosome, chromosome no. 22, contains about 5.3.times.10.sup.7 bp.

The term "oligonucleotide" is used to refer to a nucleic acid that is relatively short, generally shorter than 200 nucleotides, more particularly, shorter than 100 nucleotides, most particularly, shorter than 50 nucleotides. Typically, oligonucleotides are single-stranded DNA molecules.

The term "primer" refers to an oligonucleotide that is capable of hybridizing (also termed "annealing") with a nucleic acid and serving as an initiation site for nucleotide (RNA or DNA) polymerization under appropriate conditions (i.e., in the presence of four different nucleoside triphosphates and an agent for polymerization, such as DNA or RNA polymerase or reverse transcriptase) in an appropriate buffer and at a suitable temperature. The appropriate length of a primer depends on the intended use of the primer, but primers are typically at least 7 nucleotides long and, more typically range from 10 to 30 nucleotides, or even more typically from 15 to 30 nucleotides, in length. Other primers can be somewhat longer, e.g., 30 to 50 nucleotides long.

The term "allele call" as used herein, refers to successful characterization of an allele by a given analysis method. If the analysis provides successful characterization of both alleles of a gene locus of a DNA sample, it is said that two allele calls are made. If one allele is characterized while the other allele is not characterized, it is said that one allele call is made. If neither of the two alleles is successfully characterized, no allele calls are made.

The term "allele" as used herein, is any one of a number of viable DNA codings occupying a given locus (position) on a chromosome. Usually alleles are DNA (deoxyribonucleic acid) sequences that code for a gene, but sometimes the term is used to refer to a non-gene sequence. An individual's genotype for that gene is the set of alleles it happens to possess. In a diploid organism, one that has two copies of each chromosome, two alleles make up the individual's genotype.

The term "reaction mixture" as used herein refers to a mixture containing sufficient components to carry out an amplification reaction.

The term "sequence tag density" herein refers to the number of sequence reads that are mapped to a reference genome sequence *e.g.* the sequence tag density for chromosome 21 is the number of sequence reads generated by the sequencing method that are mapped to chromosome 21 of the reference genome. The term "sequence tag density ratio" herein refers to the ratio of the number of sequence tags that are mapped to a chromosome of the reference genome *e.g.* chromosome 21, to the length of the reference genome chromosome 21.

The terms "threshold value" and "qualified threshold value" herein refer to any number that is calculated using a qualifying data set and serves as a limit of diagnosis of a copy number variation *e.g.* an aneuploidy, in an organism. If a threshold is exceeded by results obtained from practicing the invention, a subject can be diagnosed with a copy number variation *e.g.* trisomy 21.

The term "read" refers to a DNA sequence of sufficient length (*e.g.,* at least about 30 bp) that can be used to identify a larger sequence or region, e.g.that can be aligned and specifically assigned to a chromosome or genomic region or gene.

The term "sequence tag" is herein used interchangeably with the term "mapped sequence tag" to refer to a sequence read that has been specifically assigned *i.e.* mapped, to a larger sequence *e.g.* a reference genome, by alignment. Mapped sequence tags are uniquely mapped to a reference genome *i.e.* they are assigned to a single location to the reference genome. Tags that can be mapped to more than one location on a reference genome *i.e.* tags that do not map uniquely, are not included in the analysis.

The terms "aligned", "alignment", or "aligning" refer to one or more sequences that are identified as a match in terms of the order of their nucleic acid molecules to a known sequence from a reference genome. Such alignment can be done manually or by a computer algorithm, examples including the Efficient Local Alignment of Nucleotide Data (ELAND) computer program distributed as part of the Illumina Genomics Analysis pipeline. The matching of a sequence read in aligning can be a 100% sequence match or less than 100% (non-perfect match).

The term "reference genome" refers to any particular known genome sequence, whether partial or complete, of any organism or virus which may be used to reference identified sequences from a subject. For example, a reference genome used for human subjects as well as many other organisms is found at the National Center for Biotechnology Information at www.ncbi.nlm.nih.gov. A "genome" refers to the complete genetic information of an organism or virus, expressed in nucleic acid sequences.

The term "artificial target sequences genome" herein refers to a grouping of known sequences that encompass alleles of known polymorphic sites. For example, a "SNP reference genome" is an artificial target sequences genome comprising a grouping of sequences that encompass alleles of known SNPs.

The term "clinically-relevant sequence" herein refers to a nucleic acid sequence that is known or is suspected to be associated or implicated with a genetic or disease condition. Determining the absence or presence of a clinically-relevant sequence can be useful in determining a diagnosis or confirming a diagnosis of a medical condition, or providing a prognosis for the development of a disease.

The term "mixed sample" herein refers to a sample containing a mixture of nucleic acids, which are derived from different genomes.

The term "original maternal sample" herein refers to a biological sample obtained from a pregnant subject *e.g.* a woman, who serves as the source from which a portion is removed to amplify polymorphic target nucleic acids. The "original sample" can be any sample obtained from a pregnant subject, and the processed fractions thereof *e.g.* a purified cfDNA sample extracted from a maternal plasma sample. The term "original maternal sample" herein refers to a biological

sample obtained from a pregnant subject *e.g.* a woman, who serves as the source from which a portion is removed to amplify polymorphic target nucleic acids. The "original sample" can be any sample obtained from a pregnant subject, and the processed fractions thereof *e.g.* a purified cfDNA sample extracted from a maternal plasma sample.

The term "biological fluid" herein refers to a liquid taken from a biological source and includes, for example, blood, serum, plasma, sputum, lavage fluid, cerebrospinal fluid, urine, semen, sweat, tears, saliva, and the like. As used herein, the terms "blood," "plasma" and "serum" expressly encompass fractions or processed portions thereof. Similarly, where a sample is taken from a biopsy, swab, smear, etc., the "sample" expressly encompasses a processed fraction or portion derived from the biopsy, swab, smear, etc.

The terms "maternal nucleic acids" and "fetal nucleic acids" herein refer to the nucleic acids of a pregnant female subject and the nucleic acids of the fetus being carried by the pregnant female, respectively.

The term "corresponding to" herein refers to a nucleic acid sequence *e.g.* a gene or a chromosome, that is present in the genome of different subjects, and which does not necessarily have the same sequence in all genomes, but serves to provide the identity rather than the genetic information of a sequence of interest *e.g.* a gene or chromosome.

The term "group of chromosomes" herein refers to two or more chromosomes.

The term "subject" herein refers to a human subject as well as a non-human subject such as a mammal, an invertebrate, a vertebrate, a fungus, a yeast, a bacteria, and a virus. Although the examples herein concern human cells and the language is primarily directed to human concerns, the concept of this invention is applicable to genomes from any plant or animal, and is useful in the fields of veterinary medicine, animal sciences, research laboratories and such.

## DESCRIPTION

The methods described herein enable the determination of the fraction of the minor fetal nucleic acid component in a sample comprising a mixture of fetal and maternal nucleic acids. In particular, the method enables the determination of the fraction of cfDNA contributed by a fetus to the mixture of fetal and maternal cfDNA. in a maternal sample *e.g.* a plasma sample. The difference between the maternal and fetal fraction is determined by the relative contribution of a polymorphic allele derived from the fetal genome to the contribution of the corresponding polymorphic allele derived from the maternal genome. Polymorphic sequences can be used in conjunction with clinically-relevant diagnostic tests as a positive control for the presence of cfDNA in order to highlight false-negative or false-positive results stemming from low levels of cfDNA below the identification limit. The methods described are independent of the gender of the fetus, and are useful across a range of gestational ages.

**Figure 1** provides a flow diagram of an embodiment of method of the invention **100** for determining the fraction of fetal nucleic acids in a maternal biological sample by massively parallel sequencing of PCR-amplified polymorphic target nucleic acids. In step **110** a maternal sample comprising a mixture of fetal and maternal nucleic acids is obtained from a subject. The sample is a maternal sample that is obtained from a pregnant female, for example a pregnant woman. Other maternal samples can be from mammals, for example, cow, horse, dog, or cat. If the subject is a human, the sample can be taken in the first or second trimester of pregnancy. Any maternal biological sample can be used a source of fetal and maternal nucleic acids which are contained in cells or that are "cell-free". In some embodiments, it is advantageous to obtain a maternal sample that comprises cell-free nucleic acids *e.g.* cfDNA. Preferably, the maternal biological sample is a biological fluid sample. A biological fluid includes, as non-limiting examples, blood, plasma, serum, sweat, tears, sputum, urine, sputum, ear flow, lymph, saliva, cerebrospinal fluid, ravages, bone marrow suspension, vaginal flow, transcervical lavage, brain fluid, ascites, milk, secretions of the respiratory, intestinal and genitourinary tracts, and leukophoresis samples. In some embodiments, the biological fluid sample is a sample that is easily obtainable by non-invasive procedures *e.g.* blood, plasma, serum, sweat, tears, sputum, urine, sputum, ear flow, and saliva. In some embodiments, the biological sample is a peripheral blood sample, or the plasma and/or the serum fractions thereof. In another embodiment, the sample is a mixture of two or more biological samples *e.g.* a biological sample can comprise two or more of a biological fluid samples. As used herein, the terms "blood," "plasma" and "serum" expressly encompass fractions or processed portions thereof. In some embodiments, the biological sample is processed to obtain a sample fraction *e.g.* plasma, that contains the mixture of fetal and maternal nucleic acids. A sample that can be used to determine the genotype of one or more fetal alleles can be any sample that contains fetal cells or fetal nucleic acid. For example, maternal serum or plasma sample comprising fetal and maternal cell-free nucleic acids (e.g., DNA or RNA) can be used to determine the genotypes of fetal alleles. In one embodiment, the sample can comprise a fetal cell, e.g., a fetal nucleated red blood cell or a trophoblast.

In step **120,** the mixture of fetal and maternal nucleic acids is further processed from the sample fraction *e.g.* plasma, to obtain a sample comprising a purified mixture of fetal and maternal nucleic acids *e.g.* cfDNA. Cell-free nucleic acids, including cell-free DNA, can be obtained by various methods known in the art from biological samples including but not limited to plasma, serum and urine (Fan et al., Proc Natl Acad Sci 105:16266-16271 [2008]; Koide et al., Prenatal Diagnosis 25:604-607 [2005]; Chen et al., Nature Med. 2: 1033-1035 [1996]; Lo et al., Lancet 350: 485-487 [1997). To separate cfDNA from cells, fractionation, centrifugation (e.g., density gradient centrifugation), DNA-specific precipitation,

or high-throughput cell sorting and/or separation methods can be used. Examples of methods for processing fluid samples have been previously disclosed, e.g., U.S. Patent Application Nos. 20050282293, 20050224351, and 20050065735. Commercially available kits for manual and automated separation of cfDNA are available (Roche Diagnostics, Indianapolis, IN, Qiagen,Valencia, CA, Macherey-Nagel, Duren, DE). In some instances, it can be advantageous to fragment the nucleic acid molecules in the nucleic acid sample. Fragmentation can be random, or it can be specific, as achieved, for example, using restriction endonuclease digestion. Methods for random fragmentation are well known in the art, and include, for example, limited DNAse digestion, alkali treatment and physical shearing. In one embodiment, sample nucleic acids are obtained as cfDNA., which is not subjected to fragmentation. In other embodiments, the sample nucleic acids are obtained as genomic DNA, which is subjected to fragmentation into fragments of approximately 500 or more base pairs, and to which NGS methods can be readily applied.

In step **130,** a portion of the purified mixture of fetal and maternal cfDNA is used for amplifying a plurality of polymorphic target nucleic acids each comprising a polymorphic site. In some embodiments, the target nucleic acids each comprise a SNP. In other embodiments, each of the target nucleic acids comprises a pair of tandem SNPs. In yet other embodiments, each the target nucleic acids comprises an STR. Polymorphic sites that are contained in the target nucleic acids include without limitation single nucleotide polymorphisms (SNPs), tandem SNPs, small-scale multi-base deletions or insertions, called IN-DELS (also called deletion insertion polymorphisms or DIPs), Multi-Nucleotide Polymorphisms (MNPs) Short Tandem Repeats (STRs), restriction fragment length polymorphism (RFLP), or a polymorphism comprising any other change of sequence in a chromosome. In some embodiments, the polymorphic sites that are encompassed by the method of the invention are located on autosomal chromosomes, thereby enabling the determination of fetal fraction independently of sex of the fetus. Polymorphisms associated with chromosomes other than chromosome 13, 18, 21 and Y can also be used in the methods described herein.

Polymorphisms can be indicative, informative, or both. Indicative polymorphisms indicate the presence of fetal cell-free DNA in a maternal sample. For example, the more there is of a particular genetic sequence, *e.g.* a SNP, the more a method will translate its presence into a particular color intensity, density of color, or some other property which is detectable and measurable and indicative of the presence, absence, and quantity of a particular fragment of DNA and/or particular polymorphism *e.g.* SNP of the embryo. Informative polymorphisms yield information about the fetus - for example, the presence or absence of a disease, genetic abnormality, or any other biological information such as the stage of gestation or gender. With regard to the present invention, the methods are not conducted using all possible SNPs in a genome, but use those which are said to be "informative". "Informative SNPs" in this instance are those which identify differences in the sequence of the mother and the fetus. Any polymorphic site that can be encompassed by the reads generated by the sequencing methods described herein can be used to determine the fetal fraction.

In one embodiment, a portion of the mixture of fetal and maternal nucleic acids in the sample *e.g.* cfDNA, is used as template for amplifying target nucleic acids that comprise at least one SNP. In some embodiments, each target nucleic acid comprises a single *i.e.* one SNP. Target nucleic acid sequences comprising SNPs are available from publicly accessible databases including, but not limited to Human SNP Database at world wide web address wi.mit.edu, NCBI dbSNP Home Page at world wide web address ncbi.nlm.nih.gov, world wide web address lifesciences.perkinelmer.com, Applied Biosystems by Life Technologies ™ (Carlsbad, CA) at world wide web address appliedbiosystems.com, Celera Human SNP database at world wide web address celera.com, the SNP Database of the Genome Analysis Group (GAN) at world wide web address gan.iarc.fr. In one embodiment, the SNPs chosen for enriching the fetal and maternal cfDNA are selected from the group of 92 individual identification SNPs (IISNPs) described by Pakstis *el al.* (Pakstis et el. Hum Genet 127:315-324 [2010]), which have been shown to have a very small variation in frequency across populations ($F_{st}$ <0.06), and to be highly informative around the world having an average heterozygosity ≥0.4. SNPs that are encompassed by the method of the invention include linked and unlinked SNPs. Other useful SNPs applicable or useful for the methods described herein are disclosed in U.S. Pat. Application Nos. 20080070792, 20090280492, 20080113358, 20080026390, 20080050739, 20080220422, and 20080138809. Each target nucleic acid comprises at least one polymorphic site *e.g.* a single SNP, that differs from that present on another target nucleic acid to generate a panel of polymorphic sites *e.g.* SNPs, that contain a sufficient number of polymorphic sites of which at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 40 or more are informative. For example, a panel of SNPs can be configured to comprise at least one informative SNP. In one embodiment, the SNPs that are targeted for amplification are selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In one embodiment, the panel of SNPs comprises at least 3, at least 5, at least 10, at least 13, at least 15, at least 20, at least 25, at least 30 or mare SNPs. In one embodiment, the panel of SNPs comprises rs560681, rs1109037, rs9866013, rs13182883, rs13218440, rs7041158, rs740598, rs10773760, rs4530059, rs7205345, rs8078417, rs576261, and rs2567608. The polymorphic nucleic acids comprising the SNPs can be amplified using exemplary primer pairs provided in Example 3, and disclosed as SEQ ID NOs:57-112.

In other embodiments, each target nucleic acid comprises two or more SNPs *i.e.* each target nucleic acid comprises tandem SNPs. Preferably, each target nucleic acid comprises two tandem SNPs. The tandem SNPs are analyzed as a single unit as short haplotypes, and are provided herein as sets of two SNPs. To identify suitable tandem SNP sequences, the International HapMap Consortium database can be searched (The International HapMap Project, Nature 426:789-796 [2003]). The database is available on the world wide web at hapmap.org. In one embodiment, tandem SNPs that are targeted for amplification are selected from the following sets of tandem pairs of SNPS rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980- rs987981; rs4143392- rs4143391; rsl691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The polymorphic nucleic acids comprising the tandem SNPs can be amplified using primer pairs that amplify polymorphic sequences comprising the tandem SNPs. Examples of primer pairs that can be used to amplify the tandem SNPs disclosed herein are SEQ ID Nos: 197-310 as provided in Example 8.

In one embodiment, a portion of the mixture of fetal and maternal nucleic acids in the sample e.g. cfDNA, is used as template for amplifying target nucleic acids that comprise at least one STR. In some embodiments, each target nucleic acid comprises a single *i.e.* one STR. STR loci are found on almost every chromosome in the genome and may be amplified using a variety of polymerase chain reaction (PCR) primers. Tetranucleotide repeats have been preferred among forensic scientists due to their fidelity in PCR amplification, although some tri- and pentanucleotide repeats are also in use. A comprehensive listing of references, facts and sequence information on STRs, published PCR primers, common multiplex systems, and related population data are compiled in STRBase, which may be accessed via the World Wide Web at ibm4.carb.nist.gov:8800/dna/home.htm. Sequence information from GenBank® (http://www2.ncbi.nlm.nih.gov/cgi-bin/genbank) for commonly used STR loci is also accessible through STRBase. Commercial kits available for the analysis of STR loci generally provide all of the necessary reaction components and controls required for amplification. STR multiplex systems allow the simultaneous amplification of multiple nonoverlapping loci in a single reaction, substantially increasing throughput. With multicolor fluorescent detection, even overlapping loci can be multiplexed. The polymorphic nature of tandem repeated DNA sequences that are widespread throughout the human genome have made them important genetic markers for gene mapping studies, linkage analysis, and human identity testing. Because of the high polymorphism of STRs, most individuals will be heterozygous *i.e.* most people will possess two alleles (versions) of each-one inherited from each parent-with a different number of repeats. The PCR products comprising the STRs can be separated and detected using manual, semi-automated or automated methods. Semi-automated systems are gel-based and combine electrophoresis, detection and analysis into one unit. On a semiautomated system, gel assembly and sample loading are still manual processes; however, once samples are loaded onto the gel, electrophoresis, detection and analysis proceed automatically. Data collection occurs in "real time" as fluorescently labeled fragments migrate past the detector at a fixed point and can be viewed as they are collected. As the name implies, capillary electrophoresis is carried out in a microcapillary tube rather than between glass plates. Once samples, gel polymer and buffer are loaded onto the instrument, the capillary is filled with gel polymer and the sample is loaded automatically. Therefore, the non-maternally inherited fetal STR sequence will differ in the number of repeats from the maternal sequence. Amplification of these STR sequences can result in one or two major amplification products corresponding to the maternal alleles (and the maternally inherited fetal allele) and one minor product corresponding to the non-maternally inherited fetal allele. This technique was first reported in 2000 (Pertl et al., Human Genetics 106:45-49 [2002]) and has subsequently been developed using simultaneous identification of multiple different STR regions using real-time PCR (Liu et al., Acta Obset Gyn Scand 86:535-541 [2007]). Various sized PCR amplicons have been used to discern the respective size distributions of circulating fetal and maternal DNA species, and have shown that the fetal DNA molecules in the plasma of pregnant women are generally shorter than maternal DNA molecules (Chan et al., Clin Chem 50:8892 [2004]). Size fractionation of circulating fetal DNA has confirmed that the average length of circulating fetal DNA fragments is <300 bp, while maternal DNA has been estimated to be between about 0.5 and 1 Kb (Li et al., Clin Chem, 50: 1002-1011 [2004]). There is disclosed herein a method for determining the fraction of fetal nucleic acid in a maternal sample comprising determining the amount of copies of at least one fetal and one maternal allele at a polymorphic miniSTR site, which can be amplified to generate amplicons that are of lengths about the size of the circulating fetal DNA fragments e.g. less than about 250 base pairs. In one embodiment, the fetal fraction can be

determined by a method that comprises sequencing at least a portion of amplified polymorphic target nucleic acids each comprising a miniSTR. Fetal and maternal alleles at an informative STR site are discerned by their different lengths *i.e.* number of repeats, and the fetal fraction can be calculated as a percent ratio of the amount of fetal maternal alleles at that site. The method can use one or a combination of any number of informative miniSTRs to determine the fraction of fetal nucleic acid. For example, any one or a combination of any number of miniSTRs, for example the miniSTRs disclosed in Table 7 and Figures 4 and 5, can be used. In one embodiment, the fraction of fetal nucleic acid in a maternal sample is performed using a method that includes determining the number of copies of the maternal and fetal nucleic acid present in the maternal sample by amplifying at least one autosomal miniSTR chosen from CSF1PO, FGA, TH01, TPOX, vWA, D3S1358,D5S818, D7S820, D8S1179, D13S317, D16S539, D18S51, D21S11, Penta D, Penta E, D2S1338, D1S1677, D2S441, D4S2364, D10S1248, D14S1434, D22S1045, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D5S2500, D4S2408, D4S2364, D3S4529, D3S3053, D2S1776, D2S441, D1S1677, D1S1627, and D1GATA113. In another embodiment, the at least one autosomal miniSTR is the group ofminiSTRs CSF1PO, FGA, D13S317, D16S539, D18S51, D2S1338, D21S11, D2S1338 and D7S820. In one embodiment, the method comprises determining the number of copies of at least one fetal and at least one maternal allele at least at one polymorphic miniSTR that is amplified to generate amplicons that are less than about 300 bp, less than about 250 bp, less than about 200 bp, less than about 150 bp, less than about 100 bp, or less than about 50 bp. In another embodiment, the amplicons that are generated by amplifying the miniSTRs are less than about 300 bp. In another embodiment, the amplicons that are generated by amplifying the miniSTRs are less than about 250 bp. In another embodiment, the amplicons that are generated by amplifying the miniSTRs are less than about 200 bp. Amplification of the informative allele includes using miniSTR primers, which allow for the amplification of reduced-size amplicons to detect STR alleles that are less than about 500 bp, less than about 450 bp, less than about 400 bp, less than about 350 bp, less than about 300 base pairs (bp), less than about 250 bp, less than about 200 bp, less than about 150 bp, less than about 100 bp, or less than about 50 bp. The reduced-size amplicons generated using the miniSTR primers are known as miniSTRs that are identified according to the marker name corresponding to the locus to which they have been mapped. In one embodiment, the miniSTR primers include mini STR primers that have permitted the maximum size reduction in amplicon size for all 13 CODIS STR loci in addition to the D2S1338, Penta D, and pentaE found in commercially available STR kits (Butler et al., J Forensic Sci 48:1054-1064 [2003]), miniSTR loci that are unlinked to the CODIS markers as described by Coble and Butler (Coble and Butler, J Forensic Sci 50:43-53 [2005]), and other minSTRs that have been characterized at NIST. Information regarding the miniSTRs characterized at NIST is accessible via the world wide web at cstl.nist.gov/biotech/strbase/newSTRs.htm. Any one pair or a combination of two or more pairs of miniSTR primers can be used to amplify at least one miniSTR.

In one embodiment, exemplary primer sets that can be used to amplify STRs in maternal cfDNA samples include the primer sets provided in Example 9 and disclosed as SEQ ID Nos:113-196.

Gender identification (sex-typing) in commonly performed in conjunction with STR typing using PCR products generated from the Amelogenin gene that occurs on both the X- and Y-chromosome. Amelogenin is not an STR locus, but it produces X and Y chromosome specific PCR products. A commonly used PCR primer set first published by Sullivan et al. (1993) (Sullivan et al., BioTechniques 15:637-641 [1993]) targets a 6 bp deletion that occurs on the X-chromosome, which enables amplicons generated from the X- and Y-chromosome to be distinguished from one another when electrophoretic separation is performed to separate STR alleles. Most commercial STR kits utilize the Sullivan et al. (1993) primers or minor modifications. Since females are X,X, only a single peak is observed when testing female DNA whereas males, which possess both X and Y chromosomes, exhibit two peaks with a standard Amelogenin test. In one embodiment, the method to determine the fraction of fetal nucleic acid in a maternal sample comprises coamplifying Ameleogenin with at least one miniSTR. In another embodiment, the method does not comprise coamplifying Amelogenin with miniSTR loci.

Amplification of the target nucleic acids in the mixture of fetal and maternal nucleic acid *e.g.* cfDNA, is accomplished any method that uses PCR or variations of the method including but not limited to digital PCR, real time PCR (RT-PCR), TaqMan PCR System (Applied Biosystems), SNPlex or GenPlex methods, asymmetric PCR, helicase-dependent amplification, hot-start PCR, qPCR, solid phase PCR, and touchdown PCR. Alternatively, replication of target nucleic acid sequences can be obtained by enzyme-independent methods e.g. chemical solid-phase synthesis using the phosphoramidites. Amplification of the target sequences is accomplished using primer pairs each capable of amplifying a target nucleic acid sequence comprising the polymorphic site e.g. SNP, in a multiplex PCR reaction. Multiplex PCR reactions include combining at least 2, at least three, at least 3, at least 5, at least 10, at least 15, at least 20, at least 25, at least 30, at least 40 or more sets of primers in the same reaction to quantify the amplified target nucleic acids comprising at least two, at least three, at least 5, at least 10, at least 15, at least 20, at least 25, at least 30, at least 40 or more polymorphic sites in the same sequencing reaction. Any panel of primer sets can be configured to amplify at least one informative polymorphic sequence.

In step **140** of method **100 (Figure 1),** a portion or all of the amplified polymorphic sequences are used to

prepare a sequencing library for sequencing in a parallel fashion as described. In one embodiment, the library is prepared for sequencing-by-synthesis using Illumina's reversible terminator-based sequencing chemistry.

In step **140,** sequence information that is needed for determining fetal fraction is obtained using any of the known DNA sequencing methods. In one embodiment, the method described herein employs next generation sequencing technology (NGS) in which clonally amplified DNA templates or single DNA molecules are sequenced in a massively parallel fashion within a flow cell (*e.g.* as described in Volkerding et al Clin Chem 55:641-658 [2009]; Metzker M Nature Rev 11:31-46 [2010]). In addition to high-throughput sequence information, NGS provides digital quantitative information, in that each sequence read is a countable "sequence tag" representing an individual clonal DNA template or a single DNA molecule. This quantification allows NGS to expand the digital PCR concept of counting cell-free DNA molecules (Fan et al., Proc Natl Acad Sci U S A 105:16266-16271 [2008]; Chiu et al., Proc Natl Acad Sci U S A 2008;105:20458-20463 [2008]). The sequencing technologies of NGS include pyrosequencing, sequencing-by-synthesis with reversible dye terminators, sequencing by oligonucleotide probe ligation and real time sequencing.

Some of the sequencing technologies are available commercially, such as the sequencing-by-hybridization platform from Affymetrix Inc. (Sunnyvale, CA) and the sequencing-by-synthesis platforms from 454 Life Sciences (Bradford, CT), Illumina/Solexa (Hayward, CA) and Helicos Biosciences (Cambridge, MA), and the sequencing-by-ligation platform from Applied Biosystems (Foster City, CA), as described below. In addition to the single molecule sequencing performed using sequencing-by-synthesis of Helicos Biosciences, other single molecule sequencing technologies are encompassed by the method of the invention and include the SMRT™ technology of Pacific Biosciences, the Ion Torrent™ technology, and nanopore sequencing being developed for example, by Oxford Nanopore Technologies.

While the automated Sanger method is considered as a 'first generation' technology, Sanger sequencing including the automated Sanger sequencing, can also be employed by the method of the invention. Additional sequencing methods that comprise the use of developing nucleic acid imaging technologies e.g. atomic force microscopy (AFM) or transmission electron microspcopy (TEM), are also encompassed by the method of the invention. Exemplary sequencing technologies are described below.

In one embodiment, the DNA sequencing technology that is used in the method of the invention is the Helicos True Single Molecule Sequencing (tSMS) (e.g. as described in Harris T.D. et al., Science 320:106-109 [2008]). In the tSMS technique, a DNA sample is cleaved into strands of approximately 100 to 200 nucleotides, and a polyA sequence is added to the 3' end of each DNA strand. Each strand is labeled by the addition of a fluorescently labeled adenosine nucleotide. The DNA strands are then hybridized to a flow cell, which contains millions of oligo-T capture sites that are immobilized to the flow cell surface. The templates can be at a density of about 100 million templates/$cm^2$. The flow cell is then loaded into an instrument, e.g., HeliScope™ sequencer, and a laser illuminates the surface of the flow cell, revealing the position of each template. A CCD camera can map the position of the templates on the flow cell surface. The template fluorescent label is then cleaved and washed away. The sequencing reaction begins by introducing a DNA polymerase and a fluorescently labeled nucleotide. The oligo-T nucleic acid serves as a primer. The polymerase incorporates the labeled nucleotides to the primer in a template directed manner. The polymerase and unincorporated nucleotides are removed. The templates that have directed incorporation of the fluorescently labeled nucleotide are discerned by imaging the flow cell surface. After imaging, a cleavage step removes the fluorescent label, and the process is repeated with other fluorescently labeled nucleotides until the desired read length is achieved. Sequence information is collected with each nucleotide addition step.

In one embodiment, the DNA sequencing technology that is used in the method of the invention is the 454 sequencing (Roche) (*e.g.* as described in Margulies, M. et al. Nature 437:376-380 [2005]). 454 sequencing involves two steps. In the first step, DNA is sheared into fragments of approximately 300-800 base pairs, and the fragments are blunt-ended. Oligonucleotide adaptors are then ligated to the ends of the fragments. The adaptors serve as primers for amplification and sequencing of the fragments. The fragments can be attached to DNA capture beads, e.g., streptavidin-coated beads using, e.g., Adaptor B, which contains 5'-biotin tag. The fragments attached to the beads are PCR amplified within droplets of an oil-water emulsion. The result is multiple copies of clonally amplified DNA fragments on each bead. In the second step, the beads are captured in wells (pico-liter sized). Pyrosequencing is performed on each DNA fragment in parallel. Addition of one or more nucleotides generates a light signal that is recorded by a CCD camera in a sequencing instrument. The signal strength is proportional to the number of nucleotides incorporated. Pyrosequencing makes use of pyrophosphate (PPi) which is released upon nucleotide addition. PPi is converted to ATP by ATP sulfurylase in the presence of adenosine 5' phosphosulfate. Luciferase uses ATP to convert luciferin to oxyluciferin, and this reaction generates light that is discerned and analyzed.

In one embodiment, the DNA sequencing technology that is used in the method of the invention is the SOLiD technology (Applied Biosystems). In SOLiD sequencing-by-ligation, genomic DNA is sheared into fragments, and adaptors are attached to the 5' and 3' ends of the fragments to generate a fragment library. Alternatively, internal adaptors can be introduced by ligating adaptors to the 5' and 3' ends of the fragments, circularizing the fragments, digesting the circularized fragment to generate an internal adaptor, and attaching adaptors to the 5' and 3' ends of the resulting fragments to generate a mate-paired library. Next, clonal bead populations are prepared in microreactors containing

beads, primers, template, and PCR components. Following PCR, the templates are denatured and beads are enriched to separate the beads with extended templates. Templates on the selected beads are subjected to a 3' modification that permits bonding to a glass slide. The sequence can be determined by sequential hybridization and ligation of partially random oligonucleotides with a central determined base (or pair of bases) that is identified by a specific fluorophore. After a color is recorded, the ligated oligonucleotide is cleaved and removed and the process is then repeated.

In one embodiment, the DNA sequencing technology that is used in the method of the invention is the single molecule, real-time (SMRT™) sequencing technology of Pacific Biosciences. In SMRT sequencing, the continuous incorporation of dye-labeled nucleotides is imaged during DNA synthesis. Single DNA polymerase molecules are attached to the bottom surface of individual zero-mode wavelength identifiers (ZMW identifiers) that obtain sequence information while phospolinked nucleotides are being incorporated into the growing primer strand. A ZMW is a confinement structure which enables observation of incorporation of a single nucleotide by DNA polymerase against the background of fluorescent nucleotides that rapidly diffuse in an out of the ZMW (in microseconds). It takes several milliseconds to incorporate a nucleotide into a growing strand. During this time, the fluorescent label is excited and produces a fluorescent signal, and the fluorescent tag is cleaved off. Identification of the corresponding fluorescence of the dye indicates which base was incorporated. The process is repeated.

In one embodiment, the DNA sequencing technology that is used in the method of the invention is nanopore sequencing (*e.g.* as described in Soni GV and Meller A. Clin Chem 53: 1996-2001 [2007]). Nanopore sequencing DNA analysis techniques are being industrially developed by a number of companies, including Oxford Nanopore Technologies (Oxford, United Kingdom). Nanopore sequencing is a single-molecule sequencing technology whereby a single molecule of DNA is sequenced directly as it passes through a nanopore. A nanopore is a small hole, of the order of 1 nanometer in diameter. Immersion of a nanopore in a conducting fluid and application of a potential (voltage) across it results in a slight electrical current due to conduction of ions through the nanopore. The amount of current which flows is sensitive to the size and shape of the nanopore. As a DNA molecule passes through a nanopore, each nucleotide on the DNA molecule obstructs the nanopore to a different degree, changing the magnitude of the current through the nanopore in different degrees. Thus, this change in the current as the DNA molecule passes through the nanopore represents a reading of the DNA sequence.

In one embodiment, the DNA sequencing technology that is used in the method of the invention is the chemical-sensitive field effect transistor (chemFET) array (e.g., as described in U.S. Patent Application Publication No. 20090026082). In one example of the technique, DNA molecules can be placed into reaction chambers, and the template molecules can be hybridized to a sequencing primer bound to a polymerase. Incorporation of one or more triphosphates into a new nucleic acid strand at the 3' end of the sequencing primer can be discerned by a change in current by a chemFET. An array can have multiple chemFET sensors. In another example, single nucleic acids can be attached to beads, and the nucleic acids can be amplified on the bead, and the individual beads can be transferred to individual reaction chambers on a chemFET array, with each chamber having a chemFET sensor, and the nucleic acids can be sequenced.

In one embodiment, the DNA sequencing technology that is used in the method of the invention is the Halcyon Molecular's method that uses transmission electron microscopy (TEM). The method, termed Individual Molecule Placement Rapid Nano Transfer (IMPRNT), comprises utilizing single atom resolution transmission electron microscope imaging of high-molecular weight (150kb or greater) DNA selectively labeled with heavy atom markers and arranging these molecules on ultra-thin films in ultra-dense (3nm strand-to-strand) parallel arrays with consistent base-to-base spacing. The electron microscope is used to image the molecules on the films to determine the position of the heavy atom markers and to extract base sequence information from the DNA. The method is further described in PCT patent publication WO 2009/046445. The method allows for sequencing complete human genomes in less than ten minutes.

In one embodiment, the DNA sequencing technology is the Ion Torrent single molecule sequencing, which pairs semiconductor technology with a simple sequencing chemistry to directly translate chemically encoded information (A, C, G, T) into digital information (0, 1) on a semiconductor chip. In nature, when a nucleotide is incorporated into a strand of DNA by a polymerase, a hydrogen ion is released as a byproduct. Ion Torrent uses a high-density array of micro-machined wells to perform this biochemical process in a massively parallel way. Each well holds a different DNA molecule. Beneath the wells is an ion-sensitive layer and beneath that an ion sensor. When a nucleotide, for example a C, is added to a DNA template and is then incorporated into a strand of DNA, a hydrogen ion will be released. The charge from that ion will change the pH of the solution, which can be identified by Ion Torrent's ion sensor. The sequencer-essentially the world's smallest solid-state pH meter-calls the base, going directly from chemical information to digital information. The Ion personal Genome Machine (PGM™) sequencer then sequentially floods the chip with one nucleotide after another. If the next nucleotide that floods the chip is not a match. No voltage change will be recorded and no base will be called. If there are two identical bases on the DNA strand, the voltage will be double, and the chip will record two identical bases called. Direct identification allows recordation of nucleotide incorporation in seconds.

Other sequencing methods include digital PCR and sequencing by hybridization. Digital polymerase chain reaction (digital PCR or dPCR) can be used to directly identify and quantify nucleic acids in a sample. Digital PCR can

be performed in an emulsion. Individual nucleic acids are separated, e.g., in a microfluidic chamber device, and each nucleic can is individually amplified by PCR. Nucleic acids can be separated such there is an average of approximately 0.5 nucleic acids/well, or not more than one nucleic acid/well. Different probes can be used to distinguish fetal alleles and maternal alleles. Alleles can be enumerated to determine copy number. In sequencing by hybridization, the hybridization comprises contacting the plurality of polynucleotide sequences with a plurality of polynucleotide probes, wherein each of the plurality of polynucleotide probes can be optionally tethered to a substrate. The substrate might be flat surface comprising an array of known nucleotide sequences. The pattern of hybridization to the array can be used to determine the polynucleotide sequences present in the sample. In other embodiments, each probe is tethered to a bead, e.g., a magnetic bead or the like. Hybridization to the beads can be identified and used to identify the plurality of polynucleotide sequences within the sample.

In one embodiment, the method employs massively parallel sequencing of millions of DNA fragments using Illumina's sequencing-by-synthesis and reversible terminator-based sequencing chemistry (e.g. as described in Bentley et al., Nature 6:53-59 [2009]). Template DNA can be genomic DNA *e.g.* cfDNA. In some embodiments, genomic DNA from isolated cells is used as the template, and it is fragmented into lengths of several hundred base pairs. In other embodiments, cfDNA is used as the template, and fragmentation is not required as ctDNA exists as short fragments. For example fetal cfDNA circulates in the bloodstream as fragments of<300 bp, and maternal cfDNA has been estimated to circulate as fragments of between about 0.5 and 1 Kb (Li et al., Clin Chem, 50: 1002-1011 [2004]). Illumina's sequencing technology relies on the attachment of fragmented genomic DNA to a planar, optically transparent surface on which oligonucleotide anchors are bound. Template DNA is end-repaired to generate 5'-phosphorylated blunt ends, and the polymerase activity of Klenow fragment is used to add a single A base to the 3' end of the blunt phosphorylated DNA fragments. This addition prepares the DNA fragments for ligation to oligonucleotide adapters, which have an overhang of a single T base at their 3' end to increase ligation efficiency. The adapter oligonucleotides are complementary to the flow-cell anchors. Under limiting-dilution conditions, adapter-modified, single-stranded template DNA is added to the flow cell and immobilized by hybridization to the anchors. Attached DNA fragments are extended and bridge amplified to create an ultra-high density sequencing flow cell with hundreds of millions of clusters, each containing ~1,000 copies of the same template. The cluster amplified DNA molecules are sequenced using a robust four-color DNA sequencing-by-synthesis technology that employs reversible terminators with removable fluorescent dyes. High-sensitivity fluorescence identification is achieved using laser excitation and total internal reflection optics. Short sequence reads of about 20-40 bp *e.g.* 36 bp, are aligned against a repeat-masked reference genome and genetic differences are called using specially developed data analysis pipeline software. After completion of the first read, the templates can be regenerated in situ to enable a second read from the opposite end of the fragments. Thus, either single-end or paired end sequencing of the DNA fragments is used according to the method. Partial sequencing of DNA fragments present in the sample is performed, and sequence tags comprising reads of predetermined length e.g. 36 bp, that are mapped to a known reference genome are counted.

The length of the sequence read is associated with the particular sequencing technology. NGS methods provide sequence reads that vary in size from tens to hundreds of base pairs. In some embodiments of the method described herein, the sequence reads are about 20bp, about 25bp, about 30bp, about 35bp, about 40bp, about 45bp, about 50bp, about 55bp, about 60bp, about 65bp, about 70bp, about 75bp, about 80bp, about 85bp, about 90bp, about 95bp, about 100bp, about 110bp, about 120bp, about 130, about 140bp, about 150bp, about 200bp, about 250bp, about 300bp, about 350bp, about 400bp, about 450bp, about 500bp, about 550bp or about 600bp. It is expected that technological advances will enable single-end reads of greater than 500bp enabling for reads of greater than about 1000bp when paired end reads are generated. In one embodiment, the sequence reads are 36bp. Other sequencing methods that can be employed by the method of the invention include the single molecule sequencing methods that can sequence nucleic acids molecules >5000 bp. The massive quantity of sequence output is transferred by an analysis pipeline that transforms primary imaging output from the sequencer into strings of bases. A package of integrated algorithms performs the core primary data transformation steps: image analysis, intensity scoring, base calling, and alignment.

In one embodiment, partial sequencing of amplified target polymorphic nucleic acids is performed, and sequence tags comprising reads of predetermined length e.g. 36 bp, that map to a known reference genome are counted. Only sequence reads that uniquely align to a reference genome are counted as sequence tags. In one embodiment, the reference genome is an artificial target sequences genome that comprises the sequences of the polymorphic target nucleic acids *e.g.* SNPs. In one embodiment, the reference genome is an artificial SNP reference genome. In another r embodiment, the reference genome is an artificial STR reference genome. In yet another embodiment, the reference genome is an artificial tandem-STR reference genome. Artificial reference genomes can be compiled using the sequences of the target polymorphic nucleic acids. Artificial reference genomes can comprise polymorphic target sequence each comprising one or more different types of polymorphic sequences. For example, an artificial reference genome can comprise polymorphic sequences comprising SNP alleles and/or STRs. In one embodiment, the reference genome is the human reference genome NCBI36/hg18 sequence, which is available on the world wide web at genome.ucsc.edu/cgi-bin/hgGateway?org=Human&db=hg18&hgsid=166260105). Other sources of public sequence information include Gen-

Bank, dbEST, dbSTS, EMBL (the European Molecular Biology Laboratory), and the DDBJ (the DNA Databank of Japan). In another embodiment, the reference genome comprises the human reference genome NCBI36/hg18 sequence and an artificial target sequences genome, which includes the target polymorphic sequences e.g. a SNP genome. Mapping of the sequence tags is achieved by comparing the sequence of the mapped tag with the sequence of the reference genome to determine the chromosomal origin of the sequenced nucleic acid (e.g. cfDNA) molecule, and specific genetic sequence information is not needed. A number of computer algorithms are available for aligning sequences, including without limitation BLAST (Altschul et al., 1990), BLITZ (MPsrch) (Sturrock & Collins, 1993), FASTA (Person & Lipman, 1988), BOWTIE (Langmead et al., Genome Biology 10:R25.1-R25.10 [2009]), or ELAND (Illumina, Inc., San Diego, CA, USA). In one embodiment, one end of the clonally expanded copies of the plasma cfDNA molecules is sequenced and processed by bioinformatic alignment analysis for the Illumina Genome Analyzer, which uses the Efficient Large-Scale Alignment of Nucleotide Databases (ELAND) software. In embodiments of the method that comprise determining the presence or absence of an aneuploidy and fetal fraction using NGS sequencing methods, analysis of sequencing information for the determination of aneuploidy may allow for a small degree of mismatch (0-2 mismatches per sequence tag) to account for minor polymorphisms that may exist between the reference genome and the genomes in the mixed sample. Analysis of sequencing information for the determination of fetal fraction may allow for a small degree of mismatch depending on the polymorphic sequence. For example, a small degree of mismatch may be allowed if the polymorphic sequence is an STR. In cases when the polymorphic sequence is a SNP, all sequence that match exactly to either of the two alleles at the SNP site are counted first and filtered from the remaining reads, for which a small degree of mismatch may be allowed. Quantification of the number of sequence reads aligning to each chromosome for determining chromosomal aneuploidies can be determined as described herein, or using alternative analyses that employ normalizing the median number of sequence tags for a chromosome of interest to the median number of tags for each of the other autosomes (Fan et al., Proc Natl Acad Sci 105:16266-16271 [2008]), or that compare the number of unique reads aligning to each chromosome to the total number of reads aligning to all chromosomes to derive a percent genomic representation for each chromosome. A "z score" is generated to represent the difference between the percent genomic representation of the chromosome of interest and the mean percent representation for the same chromosome between a euploid control group, divided by the standard deviation (Chiu et al., Clin Chem 56:459-463 [2010]). In another embodiment, the sequencing information can be determined as described in U.S. Provisional Patent Application titled "Normalizing Biological Assays," docket no. 32047-768.101, filed January 19, 2010.

Analysis of sequencing information for the determination of fetal fraction may allow for a small degree of mismatch depending on the polymorphic sequence. For example, a small degree of mismatch may be allowed if the polymorphic sequence is an STR. In cases when the polymorphic sequence is a SNP, all sequences that match exactly to either of the two alleles at the SNP site are counted first and filtered from the remaining reads, for which a small degree of mismatch may be allowed. The present method for determining fetal fraction by sequencing of nucleic acids can be used in combination with other methods.

In step **160,** fetal fraction is determined based on the total number of tags that map to the first allele and the total number of tags that map to second allele at an informative polymorphic site e.g. a SNP, contained in a reference genome. For example, the reference genome is an artificial target sequence genome that encompasses the polymorphic sequences that comprise SNPs rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In one embodiment, the artificial reference genome includes the polymorphic target sequences of SEQ ID NOs:1-56 (see Example-3).

In another embodiment, the artificial genome is an artificial target sequence genome that encompasses polymorphic sequences that comprise tandem SNPs rs7277033-rs2110153; rs2822654-rsl882882; rs368657-rs376635; rs2822731-rs2822732; rs1475881-rs7275487; rs1735976-rs2827016; rs447340-rs2824097; rs418989-rs13047336; rs987980-rs987981; rs4143392-rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102- rs458848; rs961301-rs2830208; rs2174536-rs458076; rs11088023-rs1 1088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rsl2626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672.

In another embodiment, the artificial target genome encompasses polymorphic sequences that comprise STRs selected from CSF1PO, FGA, TH01, TPOX, vWA, D3S1358,D5S818, D7S820, D8S1179, D13S317, D16S539, D18S51,

D21S11, Penta D, Penta E, D2S1338, D1S1677, D2S441, D4S2364, D10S1248, D14S1434, D22S1045, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D5S2500, D4S2408, D4S2364, D3S4529, D3S3053, D2S1776, D2S441, D1S1677, D1S1627, and D1GATA113. The composition of the artificial target sequences genome will vary depending on the polymorphic sequences that are used for determining the fetal fraction. Accordingly, an artificial target sequences genome is not limited to the SNP, tandem SNP or STR sequences exemplified herein.

The informative polymorphic site *e.g.* SNP, is identified by the difference in the allelic sequences and the amount of each of the possible alleles. Fetal cfDNA is present at a concentration that is <10% of the maternal cfDNA. Thus, the presence of a minor contribution of an allele to the mixture of fetal and maternal nucleic acids relative to the major contribution of the maternal allele can be assigned to the fetus. Alleles that are derived from the maternal genome are herein referred to as major alleles, and alleles that are derived from the fetal genome are herein referred to as minor alleles. Alleles that are represented by similar levels of mapped sequence tags represent maternal alleles. The results of an exemplary multiplex amplification of target nucleic acids comprising SNPs and derived from a maternal plasma sample is shown in **Figure 2.** Informative SNPs are discerned from the single nucleotide change at a polymorphic site, and fetal alleles are discerned by their relative minor contribution to the mixture of fetal and maternal nucleic acids in the sample when compared to the major contribution to the mixture by the maternal nucleic acids. Accordingly, the relative abundance of fetal cfDNA in the maternal sample is determined as a parameter of the total number of unique sequence tags mapped to the target nucleic acid sequence on a reference genome for each of the two alleles of the predetermined polymorphic site. In one embodiment, the fraction of fetal nucleic acids in the mixture of fetal and maternal nucleic acids is calculated for each of the informative allele ($allele_x$) as follows:

$$\%\ \text{fetal fraction allele}_x = ((\sum \text{Fetal sequence tags for allele}_x) / (\sum \text{Maternal sequence tags for allele}_x)) \times 100$$

and fetal fraction for the sample is calculated as the average of the fetal fraction of all of the informative alleles. Optionally, the fraction of fetal nucleic acids in the mixture of fetal and maternal nucleic acids is calculated for each of the informative allele ($allele_x$) as follows:

$$\%\ \text{fetal fraction allele}_x = ((2 \text{ X } \sum \text{Fetal sequence tags for allele}_x) / (\sum \text{Maternal sequence tags for allele}_x)) \times 100,$$

to compensate for the presence of 2 fetal alleles, one being masked by the maternal background.

The percent fetal fraction is calculated for at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 40 or more informative alleles. In one embodiment, the fetal fraction is the average fetal fraction determined for at least 3 informative alleles.

**Figure 3,** shows a flowchart of alternate methods whereby fetal fraction can be determined from amplified target nucleic acids that have been combined with unamplified fetal and maternal cfDNA sample to allow for the simultaneous determination of fetal fraction and the presence or absence of fetal aneuploidy by enriching the maternal sample comprising fetal and maternal nucleic acids for polymorphic target nucleic acids. In one embodiment, the sample that is enriched is the plasma fraction of a blood sample (a). For example, a portion of an original maternal plasma sample is used for amplifying target nucleic acid sequences. Subsequently, some or all of the amplified product is combined with the remaining unamplified original plasma sample thereby enriching it (see Example 7). In another embodiment, the sample that is enriched is the sample of purified cfDNA that is extracted from plasma (b). For example, enrichment comprises amplifying the target nucleic acids that are contained in a portion of an original sample of purified mixture of fetal and maternal nucleic acids *e.g.* cfDNA that has been purified from a maternal plasma sample, and subsequently combining some or all of the amplified product with the remaining unamplified original purified sample (see Example 6). In yet another embodiment, the sample that is enriched is a sequencing library sample prepared from a purified mixture of fetal and maternal nucleic acids (c). For example, enrichment comprises amplifying the target nucleic acids that are contained in a portion of an original sample of purified mixture of fetal and maternal nucleic acids *e.g.* cfDNA that has been purified from a maternal plasma sample, preparing a first sequencing library of unamplified nucleic acid sequences, preparing a second sequencing library of amplified polymorphic target nucleic acids, and subsequently combining some or all of the second sequencing library with some or all of the first sequencing library (see Example 5). The amount of amplified product that is used to enrich the original sample is selected to obtain sufficient sequencing information for determining both the presence or absence of aneuploidy and the fetal fraction from the same sequencing run. At least about 3%, at least about 5%, at least about 7%, at least about 10%, at least about 15%, at least about 20%, at least

about 25%, at least about 30% or more of the total number of sequence tags obtained from sequencing are mapped to determine the fetal fraction. Sequencing of the library generated following any one of the methods depicted in **Figure 3,** provides sequence tags derived from the amplified target nucleic acids and tags derived from the original unamplified maternal sample. Fetal fraction is calculated from the number of tags mapped to an artificial reference genome, and the presence or absence of aneuploidy is determined from the number of tags that map to the subject genome *e.g.* human genome.

An alternative method for determining fetal fraction from amplified polymorphic target nucleic acids at step **130** of **Figure 1,** uses size separation of amplified polymorphic target nucleic acids comprising STRs (step **150** of **Figure 1).** As described above, the polymorphic character of a STR locus is due to variation in the number of tandemly repeated units between alleles. Because of the high polymorphism of the STRs most individuals will be heterozygous for STRs. Amplification of an STR will result in one or two PCR products in most samples. In samples obtained from pregnant women *e.g.* plasma samples, amplification of an STR will result in one or two major PCR products, which correspond to the one or two maternal alleles including one fetal maternally-inherited allele, and a third paternally-inherited fetal allele that is detected at an informative STR.

The STRs that are targeted for amplification are miniSTRs as described herein that are less than about 300 base pairs and that are amplified in a multiplex PCR reaction, which allows the simultaneous amplification of multiple loci in a single reaction. The primers are labeled with different fluorescent dyes each emitting fluorescence at a different wavelength *e.g.* 6FAM™, VIC™, NED™, and PET™, and the number of repeat units for each fluorescently tagged STR in the resulting PCR products is detected following their separation and accurate sizing that is achieved by slab or capillary electrophoresis. In one embodiment, capillary electrophoresis is used, and it can be performed in microfabricated channels or capillary arrays. Alternatively, methods utilizing mass spectrometry and microarray technology are used. Multiplex STR analysis can be performed to determine fetal fraction using commercially-available kits *e.g.* AmpFESTR® Identifiler® PCR Amplification Kit (Figure 4) and AmpFℓSTR®) MiniFiler® PCR Amplification Kit (Figure 5) (Applied Biosystems, Foster City, Calif.). The AmpFℓSTR® MiniFiler® PCR Amplification Kit was designed to amplify as miniSTRs eight of the largest sized loci in the AmpFℓSTR® Identifiler® PCR Amplification Kit. Together with the gender-identification locus Amelogenin, the nine-locus multiplex enables simultaneous amplification of loci of cfDNA samples (see Example 10).

In one embodiment, multiplex STR analysis for determining fetal fraction is performed by amplifying polymorphic target nucleic acids present in a maternal plasma sample that each comprise a miniSTR selected from CSF1PO, FGA, TH01, vWA, D3S1358, D5S818, D7S820, D8S1179, D13S317, D16S539, D18S51, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. In another embodiment, multiplex STR analysis for determining fetal fraction is performed by amplifying polymorphic target nucleic acids present in a maternal plasma sample for the panel of miniSTRs: CSF1PO, D13S317, D16S539, D18S51, D21S11, D2S1338, D7S820 and FGA. The miniSTRs can be located on the same or on different chromosomes. The method is a fetal gender-independent method. Therefore, in some embodiments, the miniSTRs are located on chromosomes other than the Y chromosome. In other embodiments, the miniSTRs are located on chromosomes other than chromosomes 13, 18, 21 or X *i.e.* chromosomes that might be involved in an aneuploidy.

Samples of maternal plasma often contain less than 100 pg of cfDNA. The low copy number DNA samples can fall below the sensitivity limitations of STR analysis methods. The intractable samples can be made amenable by increasing the number of starting cfDNA available for subsequent STR analysis by a whole genome amplification strategy. In one embodiment, the mixture of fetal and maternal nucleic acids can be preamplified before alleles are detected or quantified. For example, template cell-free DNA can be amplified by PCR. The nucleic acid can be amplified for about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 cycles. Nucleic acid can be amplified for about 1-10 cycles, about 1-20 cycles, about 1-30 cycles, about 1-40 cycles, about 5-15 cycles, about 5-20 cycles, about 5-30 cycles, about 5-40 cycles, about 10-15 cycles, about 10-20 cycles, about 10-30 cycles, about 10-40 cycles, about 20-30 cycles, about 20-40 cycles, or about 30-40 cycles. The amount of template nucleic acid that can be amplified can about 10-1000 pg, 25-1000 pg, 50-1000 pg, 100-1000, pg, 200-1000 pg, 300-1000 pg, 400-1000 pg, 500-1000 pg, 600-1000, pg, 700-1000 pg, or 800-1000 pg. Following preamplification, the nucleic acids can be diluted before alleles are detected or quantified. Preamplification can be used to increase the detection sensitivity of alleles in a sample, for example, a maternal sample (Example 11). In another embodiment, genotyping a polymorphism need not require a pre-amplification step. Any PCR-based amplification method can be used to preamplify the cfDNA. Amplification methods include but are not limited to whole genome amplification strategies including methods such as primer extension preamplification, degenerate oligonucleotide-primed PCR (DOP-PCR), low fragments from low quantities of DOP-PCR, improved primer extension preamplification PCR (IPEP PCR), and modified improved primer extension preamplification (mIPEP). Thus, in one embodiment, the method that is used for determining the fetal fraction in a maternal sample e.g. plasma sample, comprises preamplifying the

mixture of fetal and maternal nucleic acids present in the plasma cfDNA sample using a whole genome amplification method, amplifying a plurality of polymorphic nucleic acids in said mixture of fetal and maternal nucleic acids, wherein each of said at least one polymorphic nucleic acid comprises an STR; determining the amount of fetal and maternal STR alleles at least one polymorphic nucleic acid; and calculating the fetal fraction from the amount of fetal and maternal STR alleles. Following preamplification, multiplex STR analysis for determining fetal fraction is performed by amplifying polymorphic target nucleic acids present in a maternal plasma sample that each comprise a miniSTR selected from CSF1PO, FGA, TH01, vWA, D3S1358, D5S818, D7S820, D8S1179, D13S317, D16S539, D18S51, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. Alternatively, multiplex STR analysis for determining fetal fraction is performed by amplifying polymorphic target nucleic acids for the panel of miniSTRs: CSF1PO, D13S317, D16S539, D18S51, D21S11, D2S1338, D7S820 and FGA.

**Applications**

Methods described herein are applicable to diagnosis or prognosis of various disease conditions including, but not limited to, cancer, genetic disorders and infection. The fetal fraction of nucleic acid in a maternal sample can be used for determining a chromosomal abnormality. Examples of chromosomal abnormalities include, for example, aneuploidy, monosomy, trisomy, duplication, inversion, deletion, polyploidy, deletion of a part of a chromosome, addition, addition of a part of chromosome, insertion, a fragment of a chromosome, a region of a chromosome, chromosomal rearrangement, and translocation. For example, aneuploidy can refer to the occurrence of one or more extra or missing chromosomes in a sample.

Examples of fetal conditions that can be determined using the methods of the provided invention include, for example, Angleman syndrome (15q11.2-q13), cri-du-chat syndrome (5p-), DiGeorge syndrome and Velo-cardiofacial syndrome (22q11.2), Miller-Dieker syndrome (17 p13.3), Prader-Willi syndrome (15q11.2-q13), retinoblastoma (13q14), Smith-Magenis syndrome (17 p11.2), trisomy 13, trisomy 16, trisomy 18, trisomy 21 (Down's syndrome), triploidy, Williams syndrome (7q 11.23), and Wolf-Hirschhom syndrome (4p-). Examples of sex chromosome abnormalities that can be detected by methods described herein include, but are not limited to, Kallman syndrome (Xp22.3), steroid sulfate deficiency (STS) (Xp22.3), X-linked ichthyosis (Xp22.3), Klinefelter syndrome (XXY), fragile X syndrome, Turner syndrome metafemales or trisomy X, and monosomy X.

In one embodiment, fetal fraction information can be used to set thresholds and estimate minimum sample size in aneuploidy detection. Such use is described in Example 7 below. Fetal fraction information can be used in conjunction with sequencing information. For example, nucleic acids from a cell-free sample, for example a maternal plasma or serum sample, can be used to enumerate sequences in a sample. Sequences can be enumerated using any of the sequencing techniques described above. Knowledge of fetal fraction can be used to set "cutoff" thresholds to call "aneuploidy," "normal," or "marginal/no call" (uncertain) states. Then, calculations can be performed to estimate the minimum number of sequences required to achieve adequate sensitivity (i.e. probability of correctly identifying an aneuploidy state).

The determination of fetal fraction according to the method of the invention can be practiced in combination with any method used to determine the presence of absence of fetal aneuploidy in a maternal plasma sample. In addition to the method described herein for the determination of aneuploidy, the determination of fetal fraction by massively parallel sequencing can be used in conjunction with other methods for determining fetal aneuploidy, for example, according to the methods described in U.S. U.S. Patent Application Publication Nos. US 2007/0202525A1; US2010/0112575A1, US 2009/0087847A1; US2009/0029377A1; US 2008/0220422A1; US2008/0138809A1, US2008/0153090A1, and US Patent 7,645,576. The methods can also be combined with assays for determining other prenatal conditions associated with the mother and/or the fetus. For example, the method can be used in conjunction with prenatal analyses, for example, as described in U.S. Patent Application Publication Nos. US2010/0112590A1, US2009/0162842A1, US2007/0207466A1, and US2001/0051341A1.

The methods described can be applied to determine the fraction of any one population of nucleic acids in a mixture of nucleic acids contributed by different genomes. In addition to determining the fraction contributed to a sample by two individuals e.g. the different genomes are contributed by the fetus and the mother carrying the fetus, the methods can be used to determine the fraction of a genome in a mixture derived from two different cells of from one individual *e.g.* the genomes are contributed to the sample by aneuploid cancerous cells and normal euploid cells from the same subject.

**Compositions and Kits**

The present invention is also related to compositions and kits or reagent systems useful for practicing the

methods described herein.

The compositions disclosed herein can be included in kits for massively parallel sequencing mixtures of fetal and maternal nucleic acid molecules *e.g.* cfDNA, present in a maternal sample *e.g.* a plasma sample. The kits comprise a composition comprising at least one set of primers for amplifying at least one polymorphic target nucleic acid in said fetal and maternal nucleic acid molecules. Polymorphic target nucleic acids can comprise without limitation single nucleotide polymorphisms (SNPs), tandem SNPs, small-scale multi-base deletions or insertions, called IN-DELS (also called deletion insertion polymorphisms or DIPs), Multi-Nucleotide Polymorphisms (MNPs) Short Tandem Repeats (STRs), restriction fragment length polymorphism (RFLP), or a polymorphism comprising any other change of sequence in a chromosome. Sequencing methods utilizing the compositions disclosed herein are NGS methods of single nucleic acid molecules or clonally amplified nucleic acid molecules as described herein. The massively parallel sequencing methods of NGS include pyrosequencing, sequencing by synthesis with reversible dye terminators, real-time sequencing, or sequencing by oligonucleotide probe ligation.

In one embodiment, the compositions includes primers for amplifying polymorphic target nucleic acids that each comprise at least one SNP. In one embodiment, the at least one SNP is selected from SNPs rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005,and rs530022. Exemplary corresponding sets of primers for amplifying the SNPs are provided as SEQ ID NOs:57-112.

In another embodiment, the composition includes primers for amplifying polymorphic target nucleic acids that each comprise at least one tandem SNP. In one embodiment, the composition includes primers for amplifying tandem SNPs. In one embodiment, the composition includes primers for amplifying the tandem SNPs disclosed herein, and the composition comprises the corresponding exemplary primers of SEQ ID NOS:197-310.

In another embodiment, the composition includes primers for amplifying polymorphic target nucleic acids that each comprise at least one STR. Exemplary STRs include CSF1PO, FGA, TH01, TPOX, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627 and D1GATA113, which can be amplified by the corresponding sets of primers of SEQ ID NOs:1 13-196.

Kits can contain a reagent combination including the elements required to conduct an assay according to the methods disclosed herein. The reagent system is presented in a commercially packaged form, as a composition or admixture where the compatibility of the reagents will allow, in a test device configuration, or more typically as a test kit, i.e., a packaged combination of one or more containers, devices, or the like holding the necessary reagents, and preferably including written instructions for the performance of assays. The kit disclosed herein may be adapted for any configuration of assay and may include compositions for performing any of the various assay formats described herein. Kits for determining fetal fraction comprise compositions including primer sets for amplifying polymorphic nucleic acids present in a maternal sample as described and, where applicable, reagents for purifying cfDNA, are disclosed herein. In one embodiment, a kit designed to allow quantification of fetal and maternal polymorphic sequences *e.g.* STRs and/or SNPs and/or tandem SNPs, in a cfDNA plasma sample, includes at least one set of allele specific oligonucleotides specific for a selected SNP and/or region of tandem repeats. Preferably, the kit includes a plurality of primer sets to amplify a panel of polymorphic sequences. A kit can comprise other reagents and/or information for genotyping or quantifying alleles in a sample (e.g., buffers, nucleotides, instructions). The kits also include a plurality of containers of appropriate buffers and reagents.

The present invention is described in further detail in the following Examples which are not in any way intended to limit the scope of the invention as claimed. The attached Figures are meant to be considered as integral parts of the specification and description of the invention. The following examples are offered to illustrate, but not to limit the claimed invention.

## EXPERIMENTAL

### Example 1

### Determination of Fetal Fraction using Massively Parallel Sequencing: Sample Processing and cfDNA Extraction

Peripheral blood samples were collected from pregnant women in their first or second trimester of pregnancy and who were deemed at risk for fetal aneuploidy. Informed consent was obtained from each participant prior to the blood draw. Blood was collected before amniocentesis or chorionic villus sampling. Karyotype analysis was performed using the chorionic villus or amniocentesis samples to confirm fetal karyotype.

Peripheral blood drawn from each subject was collected in ACD tubes. One tube of blood sample (approximately 6-9 mL/tube) was transferred into one 15-mL low speed centrifuge tube. Blood was centrifuged at 2640 rpm, 4°C for 10 min using Beckman Allegra 6 R centrifuge and rotor model GA 3.8.

For cell-free plasma extraction, the upper plasma layer was transferred to a 15-ml high speed centrifuge tube and centrifuged at 16000 x g, 4°C for 10 min using Beckman Coulter Avanti J-E centrifuge, and JA-14 rotor. The two centrifugation steps were performed within 72 h after blood collection. Cell-free plasma comprising cfDNA was stored at -80°C and thawed only once before amplification of plasma cfDNA or for purification of cfDNA.

Purified cell-free DNA (cfDNA) was extracted from cell-free plasma using the QIAamp Blood DNA Mini kit (Qiagen) essentially according to the manufacturer's instruction. One milliliter of buffer AL and 100 μl of Protease solution were added to 1 ml of plasma. The mixture was incubated for 15 minutes at 56°C. One milliliter of 100% ethanol was added to the plasma digest. The resulting mixture was transferred to QIAamp mini columns that were assembled with VacValves and VacConnectors provided in the QIAvac 24 Plus column assembly (Qiagen). Vacuum was applied to the samples, and the cfDNA retained on the column filters was washed under vacuum with 750μl of buffer AW1, followed by a second wash with 750μl of buffer AW24. The column was centrifuged at 14,000 RPM for 5 minutes to remove any residual buffer from the filter. The cfDNA was eluted with buffer AE by centrifugation at 14,000 RPM, and the concentration determined using Qubit™ Quantitation Platform (Invitrogen).

## Example 2

## Determination of Fetal Fraction using Massively Parallel Sequencing: Preparation of Sequencing Libraries, Sequencing, and Analysis of Sequencing Data

### a. Preparation of Sequencing Libraries

All sequencing libraries *i.e.* target, primary and enriched libraries, were prepared from approximately 2 ng of purified cfDNA that was extracted from maternal plasma. Library preparation was performed using reagents of the NEBNext™ DNA Sample Prep DNA Reagent Set 1 (Part No. E6000L; New England Biolabs, Ipswich, MA) for Illumina® as follows. Because cell-free plasma DNA is fragmented in nature, no further fragmentation by nebulization or sonication was done on the plasma DNA samples. The overhangs of approximately 2 ng purified cfDNA fragments contained in 40μl were converted into phosphorylated blunt ends according to the NEBNext® End Repair Module by incubating in a 1.5ml microfuge tube the cfDNA with 5μl 10X phosphorylation buffer, 2μl deoxynucleotide solution mix (10 mM each dNTP), 1μl of a 1:5 dilution of DNA Polymerase I, 1μl T4 DNA Polymerase and 1μl T4 Polynucleotide Kinase provided in the NEBNext™ DNA Sample Prep DNA Reagent Set 1 for 15 minutes at 20°C. The enzymes were then heat inactivated by incubating the reaction mixture at 75°C for 5 minutes. The mixture was cooled to 4°C, and dA tailing of the blunt-ended DNA was accomplished using 10μl of the dA-tailing master mix containing the Klenow fragment (3' to 5' exo minus) (NEBNext™ DNA Sample Prep DNA Reagent Set 1), and incubating for 15 minutes at 37°C. Subsequently, the Klenow fragment was heat inactivated by incubating the reaction mixture at 75°C for 5 minutes. Following the inactivation of the Klenow fragment, 1μl of a 1:5 dilution of Illumina Genomic Adaptor Oligo Mix (Part No. 1000521; Illumina Inc., Hayward, CA) was used to ligate the Illumina adaptors (Non-Index Y-Adaptors) to the dA-tailed DNA using 4μl of the T4 DNA ligase provided in the NEBNext™ DNA Sample Prep DNA Reagent Set 1, by incubating the reaction mixture for 15 minutes at 25°C. The mixture was cooled to 4°C, and the adaptor-ligated cfDNA was purified from unligated adaptors, adaptor dimers, and other reagents using magnetic beads provided in the Agencourt AMPure XP PCR purification system (Part No. A63881; Beckman Coulter Genomics, Danvers, MA). Eighteen cycles of PCR were performed to selectively enrich adaptor-ligated cfDNA using Phusion ® High-Fidelity Master Mix (Finnzymes, Woburn, MA) and Illumina's PCR primers complementary to the adaptors (Part No. 1000537 and 1000537). The adaptor-ligated DNA was subjected to PCR (98°C for 30 seconds; 18 cycles of 98°C for 10 seconds, 65°C for 30 seconds, and 72°C for 30 seconds; final extension at 72°C for 5 minutes, and hold at 4°C) using Illumina Genomic PCR Primers (Part Nos. 100537 and 1000538) and the Phusion HF PCR Master Mix provided in the NEBNext™ DNA Sample Prep DNA Reagent Set 1, according to the manufacturer's instructions. The amplified product was purified using the Agencourt AMPure XP PCR purification system (Agencourt Bioscience Corporation, Beverly, MA) according to the manufacturer's instructions available at www.beckmangenomics.com/products/AMPureXPProtocol 000387v001.pdf. The purified amplified product was eluted in 40μl of Qiagen EB Buffer, and the concentration and size distribution of the amplified libraries was analyzed using the Agilent DNA 1000 Kit for the 2100 Bioanalyzer (Agilent technologies Inc., Santa Clara, CA).

### b. Sequencing

Sequencing of library DNA was performed using the Genome Analyzer II (Illumina Inc., San Diego, CA, USA) according to standard manufacturer protocols. Copies of the protocol for whole genome sequencing using Illumina/Solexa

technology may be found at BioTechniques.RTM. Protocol Guide 2007 Published December 2006: p 29, and on the world wide web at biotechniques.com/default.asp?page=protocol&subsection=article display&id=112378. The DNA library was diluted to 1nM and denatured. Library DNA (5pM) was subjected to cluster amplification according to the procedure described in Illumina's Cluster Station User Guide and Cluster Station Operations Guide, available on the world wide web at illumina.com/systems/genome analyzer/cluster_station.ilmn. The amplified DNA was sequenced using Illumina's Genome Analyzer II to obtain single-end reads of 36bp. Only about 30 bp of random sequence information are needed to identify a sequence as belonging to a specific human chromosome. Longer sequences can uniquely identify more particular targets. In the present case, a large number of 36 bp reads were obtained, covering approximately 10% of the genome.

**c. Analysis of sequencing data for the determination of fetal fraction**

Upon completion of sequencing of the sample, the Illumina "Sequencer Control Software" transferred image and base call files to a Unix server running the Illumina "Genome Analyzer Pipeline" software version 1.51. the 36bp reads were aligned to an artificial reference genome e.g. a SNP genome, using the BOWTIE program. The artificial reference genome was identified as the grouping of the polymorphic DNA sequences that encompass the alleles comprised in the polymorphic target sequences. For example, the artificial reference genome is a SNP genome comprising SEQ ID NOs: 1-56. Only reads that mapped uniquely to the artificial genome were used for the analysis of fetal fraction. Reads that matched perfectly to the SNP genome were counted as tags and filtered. Of the remaining reads, only reads having one or two mismatches were counted as tags and included in the analysis. Tags mapped to each of the polymorphic alleles were counted, and the fetal fraction was determined as a percent of the ratio of the number of tags mapped to the major allele *i.e.* maternal allele, and the number of tags mapped to the minor allele *i.e.* fetal allele.

**Example 3**

**Selection of Autosomal SNPs for the Determination of Fetal Fraction**

A set of 28 autosomal SNPs were selected from a list of 92 SNPs (Pakstis et al., Hum Genet 127:315-324 [2010]) and from Applied Biosystems by Life Technologies™ (Carlsbad, CA) at world wide web address appliedbiosystems.com, and validated for use in multiplexed PCR amplification. Primers were designed to hybridize to a sequence close to the SNPs site on the cfDNA to ensure that it be included in the 36 bp read generated from the massively parallel sequencing on the Illumina Analyzer GII, and to generate amplicons of sufficient length to undergo bridge-amplification during cluster formation. Thus, primers were designed to generate amplicons that were at least 110 bp, which when combined with the universal adaptors (Illumina Inc., San Diego, CA) used for cluster amplification, resulted in DNA molecules of at least 200bp. Primer sequences were identified, and primer sets *i.e.* forward and reverse primers, were synthesized by Integrated DNA Technologies (San Diego, CA), and stored as a 1μM solution to be used for amplifying polymorphic target sequences as described in Examples 4-7. Table 1 provides the RefSNP (rs) accession ID numbers, the primers used for amplifying the target cfDNA sequence, and the sequences of the amplicons comprising the possible SNP alleles that would be generated using the primers. The SNPs given in Table 1 were used for the simultaneous amplification of 13 target sequences in a multiplexed assay. The panel provided in Table 1 is an exemplary SNP panel. Fewer or more SNPs can be employed to enrich the fetal and maternal DNA for polymorphic target nucleic acids. Additional SNPs that can be used include the SNPs given in Table 2. The SNP alleles are shown in bold and are underlined. Other additional SNPs that can be used to determine fetal fraction according to the present method include rs315791, rs3780962, rs1410059, rs279844, rs38882, rs9951171, rs214955, rs6444724, rs2503107, rs1019029, rs1413212, rs1031825, rs891700, rs1005533, rs2831700, rs354439, rs1979255, rs1454361, rs8037429, and rs1490413, which have been analyzed for determining fetal fraction by TaqMan PCR, and are disclosed in US Provisional applications 61/296,358 and 61/360,837.

**TABLE 1**

| SNP Panel for the Determination of Fetal Fraction | | | | | |
|---|---|---|---|---|---|
| **SNP ID** | **Chr** | **Amplicon: Allele 1** | **Amplicon: Allele 2** | **Forward Primer Sequence, name and SEQ ID NO:** | **Reverse Primer Sequence, name and SEQ ID NO:** |
| rs560681 | 1 | CACATGCACAGCCA GCAACCCTGTCAGC AGGAGTTCCCACCA GTTTCTTTCTGAGAA CATCTGTTCAGGTTT CTCTCCATCTCT**A**TT TACTCAGGTCACAG GACCTTGGGG (SEQ ID NO:1) | CACATGCACAGCCA GCAACCCTGTCAGC AGGAGTTCCCACCA GTTTCTTTCTGAGAA CATCTGTTCAGGTTT CTCTCCATCTCT**G**TT TACTCAGGTCACAG GACCTTGGGG (SEQ ID NO:2) | CACATGCA CAGCCAGC AACCC (rs560681_C1 _1_F; SEQ ID NO:57) | CCCCAAGGTCC TGTGACCTGAG T (rs560681_C1_1_R ; SEQ ID NO:58) |
| rs109037 | 2 | TGAGGAAGTGAGGC TCAGAGGGTAAGAA ACTTTGTCACAGAGC TGGTGGTGAGGGTG GAGATTTTACACTCC CTGCCTCCCACACCA GTTTCTCC**A**GAGTGG AAAGACTTTCATCTC GCACTGGCA (SEQ ID NO:3) | TGAGGAAGTGAGGC TCAGAGGGTAAGAA ACTTTGTCACAGAGC TGGTGGTGAGGGTG GAGATTTTACACTCC CTGCCTCCCACACCA GTTTCTCC**G**GAGTGG AAAGACTTTCATCTC GCACTGGCA (SEQ ID NO:4) | TGAGGAAG TGAGGCTC AGAGGGT (rs110937_C2 _1_F ; SEQ ID NO:59) | TGCCAGTGCGA GATGAAAGTCT TT (rs110937_C2_1_R ; SEQ ID NO:60) |

(continued)

| SNP Panel for the Determination of Fetal Fraction | | | | | |
|---|---|---|---|---|---|
| **SNP ID** | **Chr** | **Amplicon: Allele 1** | **Amplicon: Allele 2** | **Forward Primer Sequence, name and SEQ ID NO:** | **Reverse Primer Sequence, name and SEQ ID NO:** |
| rs9866013 | 3 | GTGCCTTCAGAACCTTTGAGATCTGATTCTATTTTTAAAGCTTCTTAGAAGAGAGATTGCAAAGTGGGTTGTTTCTCTAGCCAGACAGGGCAGGCAAATAGGGGTGGCTGGTGGGATGGGA (SEQ ID NO:5) | GTGCCTTCAGAACCTTTGAGATCTGATTCTATTTTTAAAGCTTCTTAGAAGAGAGATTGCAAAGTGGGTTGTTTCTCTAGCCAGACAGGGCAGGTAAATAGGGGTGGCTGGTGGGATGGGA (SEQ ID NO:6) | GTGCCTTCAGAACCTTTGAGATCTGAT (rs9866013_C3_1_F; SEQ ID NO:61) | TCCCATCCCACCAGCCACCC (rs9866013_C3_1_R; SEQ ID NO:62) |
| rs13182883 | 5 | AGGTGTGTCTCTCTTTTGTGAGGGGAGGGGTCCCTTCTGGCCTAGTAGAGGGCCTGGCCTGCAGTGAGCATTCAAATCCTCAAGGAACAGGGTGGGGAGGTGGGACAAAGG (SEQ ID NO:7) | AGGTGTGTCTCTCTTTTGTGAGGGGAGGGGTCCCTTCTGGCCTAGTAGAGGGCCTGGCCTGCAGTGAGCATTCAAATCCTCGAGGAACAGGGTGGGGAGGTGGGACAAAGG (SEQ ID NO:8) | AGGTGTGTCTCTCTTTTGTGAGGGG (rs13182883_C5_1_F; SEQ ID NO:63) | CCTTTGTCCCACCTCCCCACC (rs13182883_C5_1_R; SEQ ID NO:64) |

(continued)

| SNP Panel for the Determination of Fetal Fraction | | | | | |
|---|---|---|---|---|---|
| SNP ID | Chr | Amplicon: Allele 1 | Amplicon: Allele 2 | Forward Primer Sequence, name and SEQ ID NO: | Reverse Primer Sequence, name and SEQ ID NO: |
| rs13218440 | 6 | CCTCGCCTACTGTGCTGTTTCTAACCATCATGCTTTTCCCTGAATCTCTTGAGTCTTTTTCTGCTGTGGACTGAAACTTGATCCTGAGATTCACCTCTAGTCCCTCTG**A**GCAGCCTCCTGGAATACTCAGCTGGGATGG (SEQ ID NO:9) | CCTCGCCTACTGTGCTGTTTCTAACCATCATGCTTTTCCCTGAATCTCTTGAGTCTTTTTCTGCTGTGGACTGAAACTTGATCCTGAGATTCACCTCTAGTCCCTCTG**G**GCAGCCTCCTGGAATACTCAGCTGGGATGG (SEQ ID NO:10) | CCTCGCCTACTGTGCTGTTTCTAACC (rs13218440_C6_1_F; SEQ ID NO:65) | CCATCCCAGCTGAGTATTCCAGGAG (rs13218440_C6_1_R; SEQ ID NO:66) |
| rs7041158 | 9 | AATTGCAATGGTGAGAGGTTGATGGTAAAATCAAACGGAACTTGTTATTTTGTCATTCTGATGGACTGGAACTGAGGATTTTCAATTTCCT**C**TCCAACCCAAGACACTTCTCACTGG (SEQ ID NO:11) | AATTGCAATGGTGAGAGGTTGATGGTAAAATCAAACGGAACTTGTTATTTTGTCATTCTGATGGACTGGAACTGAGGATTTTCAATTTCCT**T**TCCAACCCAAGACACTTCTCACTGG (SEQ ID NO:12) | AATTGCAATGGTGAGAGGTTGATGGT (SEQ ID NO:67) | CCAGTGAGAAGTGTCTTGGGTTGG (SEQ ID NO:68) |

(continued)

| SNP Panel for the Determination of Fetal Fraction | | | | | |
|---|---|---|---|---|---|
| **SNP ID** | **Chr** | **Amplicon: Allele 1** | **Amplicon: Allele 2** | **Forward Primer Sequence, name and SEQ ID NO:** | **Reverse Primer Sequence, name and SEQ ID NO:** |
| rs740598 | 10 | GAAATGCCTTCTCAG<br>GTAATGGAAGGTTA<br>TCCAAATATTTTTCG<br>TAAGTATTTCAAATA<br>GCAATGGCTCGTCTA<br>TGGTTAGTCTC**A**CAG<br>CCACATTCTCAGAAC<br>TGCTCAAACC (SEQ<br>ID NO:13) | GAAATGCCTTCTCAG<br>GTAATGGAAGGTTA<br>TCCAAATATTTTTCG<br>TAAGTATTTCAAATA<br>GCAATGGCTCGTCTA<br>TGGTTAGTCTC**G**CAG<br>CCACATTCTCAGAAC<br>TGCTCAAACC (SEQ<br>ID NO:14) | GAAATGCC<br>TTCTCAGGT<br>AATGGAAG<br>GT (SEQ ID<br>NO:69) | GGTTTGAGCAG<br>TTCTGAGAATG<br>TGGCT (SEQ ID<br>NO:70) |
| rs10773760 | 12 | ACCCAAAACACTGG<br>AGGGGCCTCTTCTCA<br>TTTTCGGTAGACTGC<br>AAGTGTTAGCCGTC<br>GGGACCAGCTTCTGT<br>CTGGAAGTTCGTCA<br>AATTGCAGTTA**A**GTC<br>CAAGTATGCCACAT<br>AGCAGATAAGGG<br>(SEQ ID NO:15) | ACCCAAAACACTGG<br>AGGGGCCTCTTCTCA<br>TTTTCGGTAGACTGC<br>AAGTGTTAGCCGTC<br>GGGACCAGCTTCTGT<br>CTGGAAGTTCGTCA<br>AATTGCAGTTA**G**GT<br>CCAAGTATGCCACA<br>TAGCAGATAAGGG<br>(SEQ ID NO:16) | ACCCAAAA<br>CACTGGAG<br>GGGCCT<br>(SEQ ID<br>NO:71) | CCCTTATCTGCT<br>ATGTGGCATAC<br>TTGG (SEQ ID<br>NO:72) |

(continued)

| SNP Panel for the Determination of Fetal Fraction | | | | | |
|---|---|---|---|---|---|
| SNP ID | Chr | Amplicon: Allele 1 | Amplicon: Allele 2 | Forward Primer Sequence, name and SEQ ID NO: | Reverse Primer Sequence, name and SEQ ID NO: |
| rs4530059 | 14 | GCACCAGAATTTAA ACAACGCTGACAAT AAATATGCAGTCGA TGATGACTTCCCAGA GCTCCAGAAGCAAC TCCAGCACAC**A**GAG AGGCGCTGATGTGC CTGTCAGGTGC (SEQ ID NO:17) | GCACCAGAATTTAA ACAACGCTGACAAT AAATATGCAGTCGA TGATGACTTCCCAGA GCTCCAGAAGCAAC TCCAGCACAC**G**GAG AGGCGCTGATGTGC CTGTCAGGTGC (SEQ ID NO:18) | GCACCAGA ATTTAAACA ACGCTGAC AA (SEQ ID NO:73) | GCACCTGACAG GCACATCAGCG (SEQ ID NO:74) |
| rs7205345 | 16 | TGACTGTATACCCCA GGTGCACCCTTGGGT CATCTCTATCATAGA ACTTATCTCACAGAG TATAAGAGCTGATTT CTGTGTCTGCCT**C**TC ACACTAGACTTCCAC ATCCTTAGTGC (SEQ ID NO:19) | TGACTGTATACCCCA GGTGCACCCTTGGGT CATCTCTATCATAGA ACTTATCTCACAGAG TATAAGAGCTGATTT CTGTGTCTGCCT**G**TC ACACTAGACTTCCAC ATCCTTAGTGC (SEQ ID NO:20) | TGACTGTAT ACCCCAGG TGCACCC (SEQ ID NO:75) | GCACTAAGGAT GTGGAAGTCTA GTGTG (SEQ ID NO:76) |

(continued)

| SNP Panel for the Determination of Fetal Fraction | | | | | |
|---|---|---|---|---|---|
| SNP ID | Chr | Amplicon: Allele 1 | Amplicon: Allele 2 | Forward Primer Sequence, name and SEQ ID NO: | Reverse Primer Sequence, name and SEQ ID NO: |
| rs8078417 | 17 | TGTACGTGGTCACCA GGGGACGCCTGGCG CTGCGAGGGAGGCC CCGAGCCTCGTGCCC CCGTGAAGCTTCAG CTCCCCTCCC<u>C</u>GGCT GTCCTTGAGGCTCTT CTCACACT (SEQ ID NO:21) | TGTACGTGGTCACCA GGGGACGCCTGGCG CTGCGAGGGAGGCC CCGAGCCTCGTGCCC CCGTGAAGCTTCAG CTCCCCTCCC<u>T</u>GGCT GTCCTTGAGGCTCTT CTCACACT (SEQ ID NO:22) | TGTACGTGG TCACCAGG GGACG (SEQ ID NO:77) | AGTGTGAGAAG AGCCTCAAGGA CAGC (SEQ ID NO:78) |
| rs576261 | 19 | CAGTGGACCCTGCT GCACCTTTCCTCCCC TCCCATCAACCTCTT TTGTGCCTCCCCCTC CGTGTACCACCTTCT CTGTCACCA<u>A</u>CCCTG GCCTCACAACTCTCT CCTTTGCCAC (SEQ ID NO:23) | CAGTGGACCCTGCT GCACCTTTCCTCCCC TCCCATCAACCTCTT TTGTGCCTCCCCCTC CGTGTACCACCTTCT CTGTCACCA<u>C</u>CCCTG GCCTCACAACTCTCT CCTTTGCCAC (SEQ ID NO:24) | CAGTGGAC CCTGCTGCA CCTT (SEQ ID NO:79) | GTGGCAAAGGA GAGAGTTGTGA GG (SEQ ID NO:80) |

(continued)

| SNP Panel for the Determination of Fetal Fraction | | | | | |
|---|---|---|---|---|---|
| SNP ID | Chr | Amplicon: Allele 1 | Amplicon: Allele 2 | Forward Primer Sequence, name and SEQ ID NO: | Reverse Primer Sequence, name and SEQ ID NO: |
| rs2567608 | 20 | CAGTGGCATAGTAG TCCAGGGGCTCCTCC TCAGCACCTCCAGC ACCTTCCAGGAGGC AGCAGCGCAGGCAG AGAACCCGCTGGAA G**A**ATCGGCGGAAGT TGTCGGAGAGG (SEQ ID NO:25) | CAGTGGCATAGTAG TCCAGGGGCTCCTCC TCAGCACCTCCAGC ACCTTCCAGGAGGC AGCAGCGCAGGCAG AGAACCCGCTGGAA G**G**ATCGGCGGAAGT TGTCGGAGAGG (SEQ ID NO:26) | CAGTGGCA TAGTAGTCC AGGGGCT (SEQ ID NO:81) | CCTCTCCGACA ACTTCCGCCG (SEQ ID NO:82) |

**TABLE 2**

| Additional SNPs for the Determination of Fetal Fraction | | | | | |
|---|---|---|---|---|---|
| **SNP ID** | **Chr** | **Amplicon: Allele 1** | **Amplicon: Allele 2** | **Forward Primer Sequence, name and SEQ ID NO:** | **Reverse Primer Sequence, name and SEQ ID NO:** |
| rs430046 | 16 | AGGTCTGGGGGCC GCTGAATGCCAAGC TGGGAATCTTAAAT GTTAAGGAACAAG GTCATACAATGAAT GGTGTGATGTAAAA GCTTGGGAGGTGAT TT**C**TGAGGGTAGGT GCTGGGTTTAATGG GAGGA (SEQ ID NO:27) | AGGTCTGGGGGCCGC TGAATGCCAAGCTGG GAATCTTAAATGTTA AGGAACAAGGTCATA CAATGAATGGTGTGA TGTAAAAGCTTGGGA GGTGATTT**T**TGAGGG TAGGTGCTGGGTTTA ATGGGAGGA (SEQ ID NO:28) | AGGTCTGG GGGCCGCT GAAT (rs430046_C1 _1_F; SEQ ID NO:83) | TCCTCCCATTA AACCCAGCACC T (rs430046_C1_1_R ; SEQ ID NO:84) |
| rs9951171 | 18 | ACGGTTCTGTCCTG TAGGGGAGAAAAG TCCTCGTTGTTCCT CTGGGATGCAACAT GAGAGAGCAGCAC ACTGAGGCTTTATG G**A**TTGCCCTGCCAC AAGTGAACAGG (SEQ ID NO:29) | ACGGTTCTGTCCTGT AGGGGAGAAAAGTCC TCGTTGTTCCTCTGGG ATGCAACATGAGAGA GCAGCACACTGAGGC TTTATGG**G**TTGCCCT GCCACAAGTGAACAG G (SEQ ID NO:30) | ACGGTTCTG TCCTGTAGG GGAGA (rs9951171_C 1_1_F; SEQ ID NO:85) | CCTGTTCACTTG TGGCAGGGCA (rs9951171_C1_1_ R; SEQ ID NO:86) |
| rs338882 | 5 | GCGCAGTCAGATG GGCGTGCTGGCGTC | GCGCAGTCAGATGGG CGTGCTGGCGTCTGT | GCGCAGTC AGATGGGC | TCCAGCCCTTG TCCCAAACGTG |

(continued)

| Additional SNPs for the Determination of Fetal Fraction | | | | | |
|---|---|---|---|---|---|
| SNP ID | Chr | Amplicon: Allele 1 | Amplicon: Allele 2 | Forward Primer Sequence, name and SEQ ID NO: | Reverse Primer Sequence, name and SEQ ID NO: |
| | | TGTCTTCTCTCTCTCCTGCTCTCTGGCTTCATTTTTCTCTCCTTCTGTCTCACCTTCTTTCGTGTGCCTGTGCA**C**ACACACGTTTGGGACAAGGGCTGGA (SEQ ID NO:31) | CTTCTCTCTCTCCTGCTCTCTGGCTTCATTTTTCTCTCCTTCTGTCTCACCTTCTTTCGTGTGCCTGTGCA**T**ACACACGTTTGGGACAAGGGCTGGA (SEQ ID NO:32) | GTGC (rs338882_C1_1_F; SEQ ID NO:87) | T (rs338882_C1_1_R; SEQ ID NO:88) |
| rs10776839 | 9 | GCCGGACCTGCGAAATCCCAAAATGCCAAACATTCCCGCCTCACATGATCCCAGAGAGAGGGGACCCAGTGTTCCCAGCTTGCAGCTGAGGAGCCCGAG**G**TTGCCGTCAGATCAGAGCCCCAGTTGCCCG (SEQ ID NO:33) | GCCGGACCTGCGAAATCCCAAAATGCCAAACATTCCCGCCTCACATGATCCCAGAGAGAGGGGACCCAGTGTTCCCAGCTTGCAGCTGAGGAGCCCGAG**T**TTGCCGTCAGATCAGAGCCCCAGTTGCCCG (SEQ ID NO:34) | GCCGGACCTGCGAAATCCCAA (rs10776839C1_1_F; SEQ ID NO:89) | CGGGCAACTGGGGCTCTGATC (rs10776839_C1_1_R; SEQ ID NO:90) |

(continued)

| Additional SNPs for the Determination of Fetal Fraction | | | | | |
|---|---|---|---|---|---|
| SNP ID | Chr | Amplicon: Allele 1 | Amplicon: Allele 2 | Forward Primer Sequence, name and SEQ ID NO: | Reverse Primer Sequence, name and SEQ ID NO: |
| rs9905977 | 17 | AGCAGCCTCCCTCGACTAGCTCACACTACGATAAGGAAAATTCATGAGCTGGTGTCCAAGGAGGGCTGGGTGACTCGTGGCTCAGTCAGCATCAAGATTCCTTTCGTCTTTCCCCTCTGCC (SEQ ID NO:35) | AGCAGCCTCCCTCGACTAGCTCACACTACGATAAGGAAAATTCATGAGCTGGTGTCCAAGGAGGGCTGGGTGACTCGTGGCTCAGTCAGCGTCAAGATTCCTTTCGTCTTTCCCCTCTGCC (SEQ ID NO:36) | AGCAGCCTCCCTCGACTAGCT (rs9905977_C1_1_F; SEQ ID NO:91) | GGCAGAGGGGAAAGACGAAAGGA (rs9905977_C1_1_R; SEQ ID NO:92) |
| rs1277284 | 4 | TGGCATTGCCTGTAATATACATAGCCATGGTTTTTTATAGGCAATTTAAGATGAATAGCTTCTAAACTATAGATAAGTTTCATTACCCCAGGAAGCTGAACTATAGCTACTTTACCCAAAATCATTAGAATGGTGCTT (SEQ ID NO:37) | TGGCATTGCCTGTAATATACATAGCCATGGTTTTTTATAGGCAATTTAAGATGAATAGCTTCTAAACTATAGATAAGTTTCATTACCCCAGGAAGCTGAACTATAGCTACTTTCCCCAAAATCATTAGAATGGTGCTT (SEQ ID NO:38) | TGGCATTGCCTGTAATATACATAG (rs1277284_C4_1_F; SEQ ID NO:93) | AAGCACCATTCTAATGATTTTGG (rs1277284_C4_1_R; SEQ ID NO:94) |

(continued)

| Additional SNPs for the Determination of Fetal Fraction | | | | | |
|---|---|---|---|---|---|
| **SNP ID** | **Chr** | **Amplicon: Allele 1** | **Amplicon: Allele 2** | **Forward Primer Sequence, name and SEQ ID NO:** | **Reverse Primer Sequence, name and SEQ ID NO:** |
| rs258684 | 7 | ATGAAGCCTTCCAC<br>CAACTGCCTGTATG<br>ACTCATCTGGGGAC<br>TTCTGCTCTATACT<br>CAAAGTGGCTTAGT<br>CACTGCCAATGTAT<br>TTCCATATGAGGGA<br>CGATGATTACTAAG<br>GAAATATAGAAAC<br>AACAACTGATC<br>(SEQ ID NO:39) | ATGAAGCCTTCCACC<br>AACTGCCTGTATGAC<br>TCATCTGGGGACTTC<br>TGCTCTATACTCAAA<br>GTGGCTTAGTCACTG<br>CCAATGTATTTCCAT<br>ATGAGGGACGGTGAT<br>TACTAAGGAAATATA<br>GAAACAACAACTGAT<br>C (SEQ ID NO:40) | ATGAAGCC<br>TTCCACCAA<br>CTG<br>(rs258684_C7<br>_1_F; SEQ ID<br>NO:95) | GATCAGTTGTT<br>GTTTCTATATTT<br>CCTT<br>(rs258684_C7_1_R<br>; SEQ ID NO:96) |
| rs1347696 | 8 | ACAACAGAATCAG<br>GTGATTGGAGAAA<br>AGATCACAGGCCTA<br>GGCACCCAAGGCTT<br>GAAGGATGAAAGA<br>ATGAAAGATGGAC<br>GGAACAAAATTAG<br>GACCTTAATTCTTT<br>GTTCAGTTCAG<br>(SEQ ID NO:41) | ACAACAGAATCAGGT<br>GATTGGAGAAAAGAT<br>CACAGGCCTAGGCAC<br>CCAAGGCTTGAAGGA<br>TGAAAGAATGAAAGA<br>TGGACGGAAGAAAAT<br>TAGGACCTTAATTCTT<br>TGTTCAGTTCAG (SEQ<br>ID NO:42) | ACAACAGA<br>ATCAGGTG<br>ATTGGA<br>(rs1347696_C<br>8_4_F; SEQ<br>ID NO:97) | CTGAACTGAAC<br>AAAGAATTAAG<br>GTC<br>(rs1347696_C8_4_<br>F; SEQ ID NO:98) |

(continued)

| Additional SNPs for the Determination of Fetal Fraction | | | | | |
|---|---|---|---|---|---|
| **SNP ID** | **Chr** | **Amplicon: Allele 1** | **Amplicon: Allele 2** | **Forward Primer Sequence, name and SEQ ID NO:** | **Reverse Primer Sequence, name and SEQ ID NO:** |
| rs508485 | 11 | TTGGGGTAAATTTTCATTGTCATATGTGGAATTTAAATATACCATCATCTACAAAGAATTCCACAGAGTTAAATATCTTAAGTTAAACACTTAAAATAAGTGTTTGCGTGATATTTTGATGA<u>C</u>AGATAAACAGAGTCTAATTCCCACCCC (SEQ ID NO:43) | TTGGGGTAAATTTTCATTGTCATATGTGGAATTTAAATATACCATCATCTACAAAGAATTCCACAGAGTTAAATATCTTAAGTTAAACACTTAAAATAAGTGTTTGCGTGATATTTTGATGA<u>T</u>AGATAAACAGAGTCTAATTCCCACCCC (SEQ ID NO:44) | TTGGGGTAAATTTTCATTGTCA (rs508485_C11_1_F; SEQ ID NO:99) | GGGGTGGGAATTAGACTCTG (rs508485_C11_1_R; SEQ ID NO100) |
| rs9788670 | 15 | TGCAATTCAAATCAGGAAGTATGACCAAAAGACAGAGATCTTTTTTGGATGATCCCTAGCCTAGCAAT | TGCAATTCAAATCAGGAAGTATGACCAAAAGACAGAGATCTTTTTTGGATGATCCCTAGCCTAGCAATGCCTGGC | TGCAATTCAAATCAGGAAGTATG (rs9788670_c15_2_F; SEQ | GCAACATCGAGGTTTGTCAG (rs9788670_c15_2_R; SEQ ID NO:102) |

(continued)

| Additional SNPs for the Determination of Fetal Fraction | | | | | |
|---|---|---|---|---|---|
| SNP ID | Chr | Amplicon: Allele 1 | Amplicon: Allele 2 | Forward Primer Sequence, name and SEQ ID NO: | Reverse Primer Sequence, name and SEQ ID NO: |
| | | GCCTGGCAGCCATGCAGGTGCAATGTCAACCTTAAATAATGTATTGCAAA**C**TCAGAGCTGACAAACCTCGATGTTGC (SEQ ID NO:45) | AGCCATGCAGGTGCAATGTCAACCTTAAATAATGTATTGCAAA**T**TCAGAGCTGACAAACCTCGATGTTGC (SEQ ID NO:46) | ID NO:101) | |
| rs8137254 | 22 | CTGTGCTCTGCGAATAGCTGCAGAAGTAACTTGGGGACCCAAAATAAAGCAGAATGCTAATGTCAAGTCCTGAGAACCAAGCCCTGGGACTCTGGTGCCATTT**C**GGATTCTCCATGAGCATGGT (SEQ ID NO:47) | CTGTGCTCTGCGAATAGCTGCAGAAGTAACTTGGGGACCCAAAATAAAGCAGAATGCTAATGTCAAGTCCTGAGAACCAAGCCCTGGGACTCTGGTGCCATTT**T**GGATTCTCCATGAGCATGGT (SEQ ID NO:48) | CTGTGCTCTGCGAATAGCTG (rs8137254_c22_2_F: SEQ ID NO:103) | ACCATGCTCATGGAGAATCC (rs8137254_c22_2_R; SEQ ID NO:104) |

(continued)

| Additional SNPs for the Determination of Fetal Fraction | | | | | |
|---|---|---|---|---|---|
| SNP ID | Chr | Amplicon: Allele 1 | Amplicon: Allele 2 | Forward Primer Sequence, name and SEQ ID NO: | Reverse Primer Sequence, name and SEQ ID NO: |
| rs3143 | 19 | TTTTTCCAGCCAACTCAAGGCCAAAAAAATTTCTTAATATAGTTATTATGCGAGGGGAGGGGAAGCAAAGGAGCACAGGTAGTCCACAGAATA<u>A</u>GACACAAGAAACCTCAAGCTGTG (SEQ ID NO:49) | TTTTTCCAGCCAACTCAAGGCCAAAAAAATTTCTTAATATAGTTATTATGCGAGGGGAGGGGAAGCAAAGGAGCACAGGTAGTCCACAGAATA<u>G</u>GACACAAGAAACCTCAAGCTGTG (SEQ ID NO:50) | TTTTTCCAGCCAACTCAAGG (rs3143_c19_2_F: SEQ ID NO:105) | CACAGCTTGAGGTTTCTTGTG (rs3143_c19_2_R; SEQ ID NO:106) |
| rs2182957 | 13 | TCTTCTCGTCCCCTAAGCAAACAACATCCGCTTGCTTCTGTCTGTGTAACCACAGTGAATGGGTGTGCACGCTTG<u>A</u>TGGGCCTCTGAGCCCCTGTTGCACAAACCAGAAA (SEQ ID NO:51) | TCTTCTCGTCCCCTAAGCAAACAACATCCGCTTGCTTCTGTCTGTGTAACCACAGTGAATGGGTGTGCACGCTTG<u>G</u>TGGGCCTCTGAGCCCCTGTTGCACAAACCAGAAA (SEQ ID NO:52) | TCTTCTCGTCCCCTAAGCAA (rs2182957_c13_1_F: SEQ ID NO:107) | TTTCTGGTTTGTGCAACAGG (rs2182957_c13_1_R; SEQ ID NO:108) |
| rs3739005 | 2 | CACATGGGGGCATTAAGAATCGCCCAGGGAGGAGGAGGGAGAACGCGTGCTTTT | CACATGGGGGCATTAAGAATCGCCCAGGGAGGAGGAGGGAGAACGCGTGCTTTTCACATT | CACATGGGGGCATTAAGAAT (rs3739005_c2 | ACATCGATGAGCACAAAAACAC (rs3739005_c2_2_R; SEQ ID |

(continued)

| Additional SNPs for the Determination of Fetal Fraction | | | | | |
|---|---|---|---|---|---|
| **SNP ID** | **Chr** | **Amplicon: Allele 1** | **Amplicon: Allele 2** | **Forward Primer Sequence, name and SEQ ID NO:** | **Reverse Primer Sequence, name and SEQ ID NO:** |
| | | CACATTTGCATTTG AATTTT**C**GAGTTCC CAGGATGTGTTTTT GTGCTCATCGATGT (SEQ ID NO:53) | TGCATTTGAATTTT**T**G AGTTCCCAGGATGTG TTTTTGTGCTCATCGA TGT (SEQ ID NO:54) | _2_F; SEQ ID NO:109) | NO:110) |
| rs530022 | 1 | GGGCTCTGAGGTGT GTGAAATAAAAAC AAATGTCCATGTCT GTCCTTTTATGGCA TTTTGGGACTTTAC ATTTCAAACATTTC AGACATGTATCACA ACACGA**A**GGAATA ACAGTTCCAGGGAT ATCT (SEQ ID NO:55) | GGGCTCTGAGGTGTG TGAAATAAAAACAAA TGTCCATGTCTGTCCT TTTATGGCATTTTGGG ACTTTACATTTCAAA CATTTCAGACATGTA TCACAACACGA**G**GGA ATAACAGTTCCAGGG ATATCT (SEQ ID NO:56) | GGGCTCTG AGGTGTGT GAAA (rs530022_c1_ 2_F; SEQ ID NO:111) | AGATATCCCTG GAACTGTTATT CC (rs530022_c1_2_R ; SEQ ID NO:112) |

**Example 4**

**Determination of Fetal Fraction by Massively Parallel Sequencing of a Target Library**

To determine the fraction of fetal cfDNA in a maternal sample, target polymorphic nucleic acid sequences each comprising a SNP were amplified and used for preparing a target library for sequencing in a massively parallel fashion.

cfDNA was extracted as described in Example 1. A target sequencing library was prepared as follows. cfDNA contained in 5μl of purified cfDNA was amplified in a reaction volume of 50g1 containing 7.5μl of a 11μM primer mix (Table 1), 10μl of NEB 5X Mastermix and 27 μl water. Thermal cycling was performed with the Gene Amp9700 (Applied Biosystems) using the following cycling conditions: incubating at 95°C for 1 minute, followed by 20-30 cycles at 95°C for 20 seconds, 68°C for 1 minute, and 68°C for 30s, which was followed by a final incubation at 68°C for 5 minutes. A final hold at 4°C was added until the samples were removed for combining with the unamplified portion of the purified cfDNA sample. The amplified product was purified using the Agencourt AMPure XP PCR purification system (Part No. A63881; Beckman Coulter Genomics, Danvers, MA). A final hold at 4°C was added until the samples were removed for preparing the target library. The amplified product was analyzed with a 2100 Bioanalyzer (Agilent Technologies, Sunnyvale, CA), and the concentration of amplified product determined. A sequencing library of amplified target nucleic acids was prepared as described in Example 2, and was sequenced in a massively parallel fashion using sequencing-by-synthesis with reversible dye terminators and according to the Illumina protocol (BioTechniques.RTM. Protocol Guide 2007 Published December 2006: p 29, and on the world wide web at biotechniques.com/default.asp?page=protocol&subsection=article_display&id=I 12378). Analysis and counting of tags mapped to a reference genome consisting of 26 sequences (13 pairs each representing two alleles) comprising a SNP *i.e.* SEQ ID NO:1-26 was performed as described.

Table 3 provides the tag counts obtained from sequencing the target library, and the calculated fetal fraction derived from sequencing data.

**TABLE 3**

| Determination of Fetal Fraction by Massively Parallel Sequencing of a Library of Polymorphic Nucleic Acids | | |
|---|---|---|
| **SNP** | **SNP TAG COUNTS** | **Fetal Fraction (%)** |
| rs10773760.1\|Chr.12\|length=128\|allele=A | 236590 | 1.98 |
| rs10773760.2\|Chr.12\|length=128\|allele=A | 4680 | |
| rs13182883.1\|Chr.5\|length=111\|allele=A | 3607 | 4.99 |
| rs13182883.2\|Chr.5\|length=111\|allele=G | 72347 | |
| rs4530059.1\|Chr.14\|length=110\|allele=A | 3698 | 1.54 |
| rs4530059.1\|Chr.14\|length=110\|allele=G | 239801 | |
| rs8078417.1\|Chr.17\|length=110\|allele=C | 1E+06 | 3.66 |
| rs8078417.2\|Chr.17\|length=110\|allele=T | 50565 | |
| **Fetal Fraction (Mean±S.D.) = 12.4±6.6** | | |

The results show that polymorphic nucleic acid sequences each comprising at least one SNP can be amplified from cfDNA derived from a maternal plasma sample to construct a library that can be sequenced in a massively parallel fashion to determine the fraction of fetal nucleic acids in the maternal sample.

**Example 5**

**Determination of Fetal Fraction Following Enrichment of Fetal and Maternal Nucleic Acids in a cfDNA Sequencing Library Sample.**

To enrich the fetal and maternal cfDNA contained in a primary sequencing library constructed using purified fetal and maternal cfDNA., a portion of a purified cfDNA sample was used for amplifying polymorphic target nucleic acid sequences, and for preparing a sequencing library of amplified polymorphic target nucleic acids, which was used to enrich the fetal and maternal nucleic acid sequences comprised in the primary library.

The method corresponds to workflow 3 diagrammed in Figure 3. A target sequencing library was prepared from a portion of the purified cfDNA as described in Example 2. A primary sequencing library was prepared using the remaining

portion of the purified cfDNA as described in Example 2. Enrichment of the primary library for the amplified polymorphic nucleic acids comprised in the target library was obtained by diluting the primary and the target sequencing libraries to 10nM, and combining the target library with the primary library at a ratio of 1:9 to provide an enriched sequencing library. Sequencing of the enriched library and analysis of the sequencing data was performed as described in Example 2.

Table 4 provides the number of sequence tags that mapped to the SNP genome for the informative SNPs identified from sequencing an enriched library derived from plasma samples of pregnant women each carrying a T21, a T13, a T18 and a monosomy X fetus, respectively. Fetal fraction was calculated as follows:

% fetal fraction $allele_x = ((\Sigma Fetal\ sequence\ tags\ for\ allele_x) / (\Sigma Maternal\ sequence\ tags\ for\ allele_x)) \times 100$

Table 4 also provides the number of the sequence tags mapped to the human reference genome. Tags mapped to the human reference genome were used to determine the presence or absence of aneuploidy using the same plasma sample that was utilized for determining the corresponding fetal fraction. Method for using sequence tags counts for determining aneuploidy are described in U.S. Provisional Applications 61/407,017 and 61/455,849778.

**TABLE** 4

| **Determination of Fetal Fraction by Massively Parallel Sequencing of an Enriched Library of Polymorphic Nucleic Acids** | | | |
|---|---|---|---|
| **Sample ID (karyotype)** | **SNP** | **SNP TAG COUNTS** | **FETAL FRACTION (%)** |
| 11409 (47, XY+21) | rs13182883.1\|Chr.5\|length=111\|allele=A | 261 | 4.41 |
| | rs13182883.2\|Chr.5\|length= 111lallele=G | 5918 | |
| | rs740598.1\|Chr.10\|length=114\|allele=A | 5545 | 7.30 |
| | rs740598.2\|Chr.10\|length=114\|allele=G | 405 | |
| | rs8078417.1\|Chr.17\|length=110\|allele=C | 8189 | 6.74 |
| | rs8078417.2\|Chr.17\| length=110\|allele=T | 121470 | |
| | rs576261.1\|Chr.19\|length=114\|allele=A | 58342 | 7.62 |
| | rs576261.2\|Chr.19\|length=114\|allele=C | 4443 | |
| **Fetal Fraction (Mean±S.D.) = 6.5±1.5** | | | |
| **Sample ID** | | | |
| 95133 (47,XX+18) | rs1109037.1\|Chr.2\|length=126\|allele=A | 12229 | 2.15 |
| | rs1109037.2\|Chr.2\|length=126\|allele=A | 263 | |
| | rs13218440.1\|Chr.6\|length=139\|allele=A | 55949 | 3.09 |
| | rs13218440.2\|Chr.6\|length=139\|allele=G | 1729 | |
| | rs7041158.1\|Chr.9\|length=117\|allele=C | 7281 | 4.12 |
| | rs7041158.2\|Chr.9\|length=117\|allele=T | 300 | |
| | rs7205345.1\|Chr.16\|length=116\|allele=C | 53999 | 2.14 |
| | rs7205345.2\|Chr.16\|length=116\|allele=G | 1154 | |

(continued)

| Fetal Fraction (Mean±S.D.) = 2.9±0.9 | | | |
|---|---|---|---|
| **Sample ID** | | | |
| 51236 (46,XY+13) | rs13218440.1\|Chr.6\|length=139\|allele=A | 1119 | 1.65 |
| | rs13218440.2\|Chr.6\|length=139\|allele=G | 67756 | |
| | rs560681.1\|Chr.1\|length=111\|allele=A | 14123 | 5.18 |
| | rs560681.2\|Chr.1\|length=111\|allele=G | 732 | |
| | rs7205345.1\|Chr.16\|length=116\|allele=C | 18176 | 1.63 |
| | rs7205345.2\|Chr.16\|length=116\|allele=G | 296 | |
| | rs9866013.1\|Chr.3\|length=121\|allele=C | 117 | 2.33 |
| | rs9866013.2\|Chr.3\|length=121\|allele=T | 5024 | |
| **Fetal Fraction (Mean±S.D.) = 2.7±1.7** | | | |
| **Sample ID** | | | |
| 54430 (45,XO) | rs1109037.1\|Chr.2\|length=126\|allele=A | 19841 | 1.80 |
| | rs1109037.2\|Chr.21\|length=126\|allele=G | 357 | |
| | rs9866013.1\|Chr.3\|lenth=121\|allele=C | 12931 | 3.81 |
| | rs9866013.2\|Chr.3\|length=121\|allele=T | 493 | |
| | rs7041158.1\|Chr.9\|length=117\|allele=C | 2800 | 4.25 |
| | rs7041158.2\|Chr.9\|length=117\|allele=T | 119 | |
| | rs740598.1\|Chr.10\|length=114\|allele=A | 12903 | 4.87 |
| | rs740598.2\|Chr.1\|length=114\|allele=G | 628 | |
| | rs10773760.1\|Chr.12\|length=128\|allele=A | 46324 | 4.65 |
| | rs10773760.2\|Chr.12\|length=128\|allele=G | 2154 | |
| **Fetal Fraction (Mean±S.D.) = 3.9±1.2** | | | |

**Example 6**

**Determination of Fetal Fraction by Massively Parallel Sequencing: Enrichment of Fetal and Maternal Nucleic Acids for Polymorphic Nucleic Acids in a Purified cfDNA S**ample.

To enrich the fetal and maternal cfDNA contained in a purified sample of cfDNA extracted from a maternal plasma sample, a portion of the purified cfDNA was used for amplifying polymorphic target nucleic acid sequences each comprising one SNP chosen from the panel of SNPs given in Table 5.

The method corresponds to workflow 2 diagrammed in Figure 3. Cell-free plasma was obtained from a maternal blood sample, and cfDNA was purified from the plasma sample as described in Example 1. The final concentration was determined to be 92.8pg/μl. cfDNA contained in 5μl of purified cfDNA was amplified in a reaction volume of 50μl containing 7.5μl of a luM primer mix (Table 1), 10μl of NEB 5X Mastermix and 27 μl water. Thermal cycling was performed with the Gene Amp9700 (Applied Biosystems). Using the following cycling conditions: incubating at 95°C for 1 minute, followed by 30 cycles at 95°C for 20 seconds, 68°C for 1 minute, and 68°C for 30s, which was followed by a final incubation at 68°C for 5 minutes. A final hold at 4°C was added until the samples were removed for combining with the unamplified portion of the purified cfDNA sample. The amplified product was purified using the Agencourt AMPure XP PCR purification system (Part No. A63881; Beckman Coulter Genomics, Danvers, MA), and the concentration quantified using the Nanodrop 2000 (Thermo Scientific, Wilmington, DE). The purified amplification product was diluted 1:10 in water and 0.9 μl (371pg) added to 40μl of purified cfDNA sample to obtain a 10% spike. The enriched fetal and maternal cfDNA present in the purified cfDNA sample was used for preparing a sequencing library, and was sequenced as described in Example 2.

Table 5 provides the tag counts obtained for each of chromosomes 21, 18, 13, X and Y *i.e.* sequence tag

density, and the tag counts obtained for the informative polymorphic sequences contained in the SNP reference genome *.i.e.* SNP tag density. The data show that sequencing information can be obtained from sequencing a single library constructed from a purified maternal cfDNA sample that has been enriched for sequences comprising SNPs to simultaneously determine the presence or absence of aneuploidy and the fetal fraction. The presence or absence of aneuploidy was determined using the number of tags mapped to chromosomes as described in U.S. Provisional Applications 61/407,017 and 61/455,849. In the example given, the data show that the fraction of fetal DNA in plasma sample AFR105 was quantifiable from the sequencing results of five informative SNPs and determined to be 3.84%. Sequence tag densities are provided for chromosomes 21, 13, 18, X and Y.

The example shows that the enrichment protocol provides the requisite tag counts for determining aneuploidy and fetal fraction from a single sequencing process.

**TABLE 5**

| Determination of Fetal Fraction by Massively Parallel Sequencing: Enrichment of Fetal and Maternal Nucleic Acids for Polymorphic Nucleic Acids in a Purified cfDNA sample | | | | | |
|---|---|---|---|---|---|
| **Aneuploidy** | | | | | |
| | Chromosome 21 | Chromosome 18 | Chromosome 13 | Chromosome X | Chromosome Y |
| Sequence Tag Density | 178763 | 359529 | 388204 | 572330 | 2219 |
| Karyotype | Unaffected | Unaffected | Unaffected | Unaffected | Unaffected |
| **Fetal Fraction** | | | | | |
| **SNP** | | **SNP TAG DENSITY** | | **FETAL FRACTION (%)** | |
| rs10773760.1\|Chr.12\|length=128\|allele=A | | 18903 | | 2.81 | |
| rs10773760.2\|Chr.12\|length=128\|allele=G | | 532 | | | |
| rs 1109037.1\|Chr.2\|length=126\|allele=A | | 347 | | 5.43 | |
| rs1109037.2\|Chr.2\|length=126\|allele=G | | 6394 | | | |
| rs2567608.1\|Chr.20\|length=110\|allele=A | | 94503 | | 1.74 | |
| rs2567608.2\|Chr.20\|length=110\|allele=G | | 1649 | | | |
| rs7041158.1\|Chr.9\|length=117\|allele=C | | 107 | | 5.61 | |
| rs7041158.2\|Chr.9\|length=117\|allele=T | | 6 | | | |
| rs8078417.1\|Chr.17\|length=110\|allele=C | | 162668 | | 3.61 | |
| rs8078417.2\|Chr.171\|length=1\|allele=T | | 5877 | | | |
| **Fetal Fraction (Mean±S.D.) = 3.8±1.7** | | | | | |

**Example 7**

**Determination of Fetal Fraction by Massively Parallel Sequencing: Enrichment of Fetal and Maternal Nucleic Acids for Polymorphic Nucleic Acids in a Plasma Sample.**

To enrich the fetal and maternal cfDNA contained in an original plasma sample derived from a pregnant woman, a portion the original plasma sample was used for amplifying polymorphic target nucleic acid sequences each comprising one SNP chosen from the panel of SNPs given in Table 1, and a portion of the amplified product was combined with the remaining original plasma sample.

The method corresponds to workflow 2 diagrammed in Figure 3. cfDNA contained in 15μl of cell-free plasma was amplified in a reaction volume of 50μl containing 9ul of a 1 μM mixture of primers (15 plexTable 1), 1μl of Phusion blood DNA polymerase, 25ul of the 2X Phusion blood PCR buffer containing deoxynucleotide triphosphates (dNTPs: dATP, dCTP, dGTP and dTTP). Thermal cycling was performed with the Gene Amp9700 (Applied Biosystems) using the following cycling conditions: incubating at 95°C for 3 minutes, followed by 35 cycles at 95°C for 20 seconds, 55°C for 30s, and 70°C for 1 minute, which was followed by a final incubation at 68°C for 5 minutes. A final hold at 4°C was added until the samples were removed for combining with the unamplified portion of the cell-free plasma. The amplified product was diluted 1:2 with water and analyzed using the Bioanalyzer. An additional 3μl of amplified product was diluted with 11.85 μl of water to obtain a final concentration of 2ng/μl. 2.2μl of the diluted amplified product was combined with the remaining plasma sample. The enriched fetal and maternal cfDNA present in the plasma sample was purified as described in Example 1, and used for preparing a sequencing library. Sequencing and analysis of the sequencing data was performed as described in Example 2.

The results are given in Table 6. In the example given, the data show that the fraction of fetal DNA in plasma sample SAC2517 was quantifiable from the sequencing results of one informative SNP and determined to be 9.5%. In the example given, sample SAC2517 was shown by karyotyping to be unaffected for aneuploidies of chromosomes 21, 13, 18, X and Y. Sequence tag densities are provided for chromosomes 21, 13, 18, X and Y. The presence or absence of aneuploidy was determined using tag counts as described in U.S. Provisional Applications 61/407,017 and 61/455,849.

The example demonstrates that enriching the mixture of fetal and maternal cfDNA present in a plasma sample for nucleic acid sequences that comprise at least one informative SNP can be used to provide the requisite sequence and SNP tag counts for determining aneuploidy and fetal fraction from a single sequencing process by massively parallel sequencing a library prepared from cfDNA contained in a plasma sample that is enriched for polymorphic nucleic acids.

**TABLE 6**

| **Determination of Fetal Fraction by Massively Parallel Sequencing: Enrichment of Fetal and-Maternal Nucleic Acids for Polymorphic Nucleic Acids Comprising a SNP in a Plasma Sample** | | | | | |
|---|---|---|---|---|---|
| **Aneuploidy** | | | | | |
| | Chromosome 21 | Chromosome 18 | Chromosome 13 | Chromosome X | Chromosome Y |
| Sequence Tag Density | 183851 | 400582 | 470526 | 714055 | 2449 |
| Karyotype | Unaffected | Unaffected | Unaffected | Unaffected | Unaffected |
| **Fetal Fraction** | | | | | |
| **SNP** | | **TAG COUNTS** | **FETAL FRACTION (%)** | | |
| rs10773760. 1\|Chr.12\|length=128\|allele=A | | 8536 | **9.5** | | |
| rs10773760.2\|Chr. 12\|length=128\|allele=G | | 89924 | | | |

**Example 8**

**Determination of Fetal Fraction by Massively Parallel Sequencing of Samples Comprising Amplified Polymorphic Sequences: Tandem SNPs**

To determine the fraction of fetal cfDNA in a maternal sample, target polymorphic nucleic acid sequences each

comprising a pair of tandem SNPs are amplified and used for preparing a target library for sequencing in a massively parallel fashion. Pairs of tandem SNPs can be selected from rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980- rs987981; rs4143392- rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102- rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The primers used for amplifying the target sequences comprising the tandem SNPs are designed to encompass both SNP sites. For example, the forward primer is designed to encompass the first SNP, and the reverse primer is designed to encompass the second of the tandem SNP pair *i.e.* each of the SNP sites in the tandem pair is encompassed within the 36 bp generated by the sequencing method. Paired-end sequencing can be used to identify all sequences encompassing the tandem SNP sites. Exemplary sets of primers that are used to amplify the tandem SNPs disclosed herein are rs7277033-rs2110153_F:

TCCTGGAAACAAAAGTATT (SEQ ID NO:197) and rs7277033-rs2110153_R: AACCTTACAACAAAGCTAGAA (SEQ ID NO:198), set rs2822654-rs1882882_F: ACTAAGCCTTGGGGATCCAG (SEQ ID NO:199) and rs2822654-rs1882882_R: TGCTGTGGAAATACTAAAAGG (SEQ ID NO:200), set rs368657-rs376635_F:CTCCAGAGGTAATCCTGTGA (SEQ ID NO:201) and rs368657-rs376635_R:TGGTGTGAGATGGTATCTAGG (SEQ ID NO:202), rs2822731-s2822732-F:GTATAATCCATGAATCG (SEQ ID NO:203) and rs2822731-rs2822732_R:TTCAAATTGTATATAAGAGAGT (SEQ ID NO:204), rs1475881-rs7275487_F:GCAGGAAAGTTATTTTTAAT (SEQ ID NO:205) and rs1475881-rs7275487_R:TGCTTGAGAAAGCTAACACTT (SEQ ID NO:206), rs1735976-rs2827016F:CAGTGTTTGGAAATTGTCTG (SEQ ID NO:207) and rs1735976-rs2827016 R:GGCACTGGGAGATTATTGTA (SEQ ID NO:208), rs447349-rs2824097_F:TCCTGTTGTTAAGTACACAT (SEQ ID NO:209) and rs447349-rs2824097_R:GGGCCGTAATTACTTTTG (SEQ ID NO:210), rs418989-rs13047336-F:ACTCAGTAGGCACTTTGTGTC (SEQ ID NO:211) and rs418989-rs13047336_R:TCTTCCACCACACCAATC (SEQ ID NO:212), rs987980-rs987981_F:TGGCTTTTCAAAGGTAAAA (SEQ ID NO:213) and rs987980- rs987981_R: GCAACGTTAACATCTGAATTT (SEQ ID NO:214), rs4143392- rs4143391_F: rs4143392- rs4143391 (SEQ ID NO:215) and rs4143392-rs4143391_R:ATTTTATATGTCATGATCTAAG (SEQ ID NO:216), rs1691324-ras13050434_F: AGAGATTACAGGTGTGAGC (SEQ ID NO:217) and rs1691324- rs13050434_R: ATGATCCTCAACTGCCTCT (SEQ ID NO:218), rs1 1909758-rs9980111_F: TGAAACTCAAAAGAGAAAAG (SEQ ID NO:219) and rs11909758-rs9980111_R: ACAGATTTCTACTTAAAATT (SEQ ID NO:220), rs2826842-rs232414_F: TGAAACTCAAAAGAGAAAAG (SEQ ID NO:221) and rs2826842-rs232414_R: ACAGATTTCTACTTAAAATT (SEQ ID NO:22), rs2826842-rs232414_F: GCAAAGGGGTACTCTATGTA (SEQ ID NO:223) and rs2826842-rs232414_R: TATCGGGTCATCTTGTTAAA (SEQ ID NO:224), rs1980969-rs1980970_F: TCTAACAAAGCTCTGTCCAAAA (SEQ ID NO:225) and rs1980969-rsl980970_R: CCACACTGAATAACTGGAACA (SEQ ID NO:226), rs9978999-rs9979175_F: GCAAGCAAGCTCTCTACCTTC (SEQ ID NO:227) and rs9978999-rs9979175_R: TGTTCTTCCAAAATTCACATGC (SEQ ID NO:228), rs1034346-rs12481852_F: ATTTCACTATTCCTTCATTTT (SEQ ID NO:229) and rs1034346-rs12481852_R: TAATTGTTGCACACTAAATTAC (SEQ ID NO:230), rs4817013-rs7277036_F: AAAAAGCCACAGAAATCAGTC (SEQ ID NO:231) and rs4817013-rs7277036_R: TTCTTATATCTCACTGGGCATT (SEQ ID NO:232), rs9981121-rs2829696_F: GGATGGTAGAAGAGAAGAAAGG (SEQ ID NO:233) and rs9981121-rs2829696_R: GGATGGTAGAAGAGAAGAAAGG (SEQ ID NO:234), rs455921-rs2898102_F: TGCAAAGATGCAGAACCAAC (SEQ ID NO:235) and rs455921-rs2898102_R: TTTTGTTCCTTGTCCTGGCTGA (SEQ ID NO:236), rs2898102- rs458848]: TGCAAAGATGCAGAACCAAC (SEQ ID NO:237) and rs2898102- rs458848_R: GCCTCCAGCTCTATCCAAGTT (SEQ ID NO:238), rs961301-rs2830208_F: CCTTAATATCTTCCCATGTCCA (SEQ ID NO:239) and rs961301-rs2830208_R: ATTGTTAGTGCCTCTTCTGCTT (SEQ ID NO:240), rs2174536-rs458076_F: GAGAAGTGAGGTCAGCAGCT (SEQ ID NO:241) and rs2174536-rs458076_R:

TTTCTAAATTTCCATTGAACAG (SEQ ID NO:242), rs11088023-rs11088024_F: GAAATTGGCAATCTGATTCT (SEQ ID NO:243) and rs11088023-rs11088024_R: CAACTTGTCCTTTATTGATGT (SEQ ID NO:244), rs1011734-rs1011733_F: CTATGTTGATAAAACATTGAAA (SEQ ID NO:245) and rs1011734-rs1011733_R: GCCTGTCTGGAATATAGTTT (SEQ ID NO:246), rs2831244-rs9789838_F: CAGGGCATATAATCTAAGCTGT (SEQ ID NO:247) and rs2831244-rs9789838_R: CAATGACTCTGAGTTGAGCAC (SEQ ID NO:248), rs8132769-rs2831440_F: ACTCTCTCCCTCCCCTCT (SEQ ID NO:249) and rs8132769-rs2831440_R: TATGGCCCCAAAACTATTCT (SEQ ID NO:250), rs8134080-rs2831524_F: ACAAGTACTGGGCAGATTGA (SEQ ID NO:251) and rs8134080-rs2831524_R: GCCAGGTTTAGCTTTCAAGT (SEQ ID NO:252), rs4817219-rs4817220_F: TTTTATATCAGGAGAAACACTG (SEQ ID NO:253) and rs4817219-rs4817220_R: CCAGAATTTTGGAGGTTTAAT (SEQ ID NO:254), rs2250911-rs2250997_F: TGTCATTCCTCCTTTATCTCCA (SEQ ID NO:255) and rs2250911-rs2250997_R: TTCTTTTGCCTCTCCCAAAG (SEQ ID NO:256), rs2831899-rs2831900_F: ACCCTGGCACAGTGTTGACT (SEQ ID NO:257) and rs2831899-rs2831900_R: TGGGCCTGAGTTGAGAAGAT (SEQ ID NO:258), rs2831902-rs2831903_F: AATTTGTAAGTATGTGCAACG (SEQ ID NO:259) and rs2831902-rs2831903_R: TTTTTCCCATTTCCAACTCT (SEQ ID NO:260), rs11088086-rs2251447-F: AAAAGATGAGACAGGCAGGT (SEQ ID NO:261) and rs11088086-rs2251447_R: ACCCCTGTGAATCTCAAAAT (SEQ ID NO:262), rs2832040-rs11088088_F: GCACTTGCTTCTATTGTTTGT (SEQ ID NO:263) and rs2832040-rsl 1088088_R: CCCTTCCTCTCTTCCATTCT (SEQ ID NO:264), rs2832141-rs2246777_F: AGCACTGCAGGTA (SEQ ID NO:265) and rs2832141-rs2246777_R: ACAGATACCAAAGAACTGCAA (SEQ ID NO:266), rs2832959-rs9980934_F: TGGACACCTTTCAACTTAGA (SEQ ID NO:267) and rs2832959 -rs9980934_R: GAACAGTAATGTTGAACTTTTT (SEQ ID NO:268), rs2833734-rs2833735_F: TCTTGCAAAAAGCTTAGCACA (SEQ ID NO:269) and rs2833734-rs2833735_R: AAAAAGATCTCAAAGGGTCCA (SEQ ID NO:270), rs933121-rs933122_F: GCTTTTGCTGAACATCAAGT (SEQ ID NO:271) and rs933121-rs933122_R: CCTTCCAGCAGCATAGTCT (SEQ ID NO:272), rs2834140-rs12626953_F: AAATCCAGGATGTGCAGT (SEQ ID NO:273) and rs2834140-rs12626953_R: ATGATGAGGTCAGTGGTGT (SEQ ID NO:274), rs2834485-rs3453_F: CATCACAGATCATAGTAAATGG (SEQ ID NO:275) and rs2834485-rs3453_R: AATTATTATTTTGCAGGCAAT (SEQ ID NO:276), rs9974986-rs2834703_F: CATGAGGCAAACACCTTTCC (SEQ ID NO:277) and rs9974986-rs2834703_ R: GCTGGACTCAGGATAAAGAACA (SEQ ID NO:278), rs2776266-rs2835001_F: TGGAAGCCTGAGCTGACTAA (SEQ ID NO:279) and rs2776266-rs2835001_R:CCTTCTTTTCCCCCAGAATC (SEQ ID NO:280), rs1984014-rs1984015-F:TAGGAGAACAGAAGATCAGAG (SEQ ID NO:281) and rs1984014-rs1984015_R:AAAGACTATTGCTAAATGCTTG (SEQ ID NO:282), rs7281674-rs2835316_F: TAAGCGTAGGGCTGTGTGTG (SEQ ID NO:283) and rs7281674-rs2835316_R: GGACGGATAGACTCCAGAAGG (SEQ ID NO:284), rs13047304-rs13047322_F: GAATGACCTTGGCACTTTTATCA (SEQ ID NO:285) and rs13047304-rs13047322_R: AAGGATAGAGATATACAGATGAATGGA (SEQ ID NO:286), rs2835735-rs2835736_F: CATGCACCGCGCAAATAC (SEQ ID NO:287) and rs2835735-rs2835736_R: ATGCCTCACCCACAAACAC (SEQ ID NO:288), rs13047608-rs2835826_F: TCCAAGCCCTTCTCACTCAC (SEQ ID NO:289) and rs13047608-rs2835826_R: CTGGGACGGTGACATTTTCT (SEQ ID NO:290), rs2836550-rs2212596_F: CCCAGGAAGAGTGGAAAGATT (SEQ ID NO:291) and rs2836550-rs2212596_R: TTAGCTTGCATGTACCTGTGT (SEQ ID NO:292), rs2836660-rs2836661_F: AGCTAGATGGGGTGAATTTT (SEQ ID NO:293) and _R: TGGGCTGAGGGGAGATTC (SEQ ID NO:294), rs465612-rs8131220_F: ATCAAGCTAATTAATGTTATCT (SEQ ID NO:295) and rs465612-rs8131220 R: AATGAATAAGGTCCTCAGAG (SEQ ID NO:296), rs9980072-rs8130031_F:TTTAATCTGATCATTGCCCTA (SEQ ID NO:297) and rs9980072-rs8130031_R: AGCTGTGGGTGACCTTGA (SEQ ID NO:298), rs418359-rs2836926_F: TGTCCCACCATTGTGTATTA (SEQ ID NO:299) and rs418359-rs2836926_R: TCAGACTTGAAGTCCAGGAT (SEQ ID NO:300), rs7278447-rs7278858_F: GCTTCAGGGGTGTTAGTTTT (SEQ ID NO:301) and rs7278447-rs7278858_R: CTTTGTGAAAAGTCGTCCAG (SEQ ID NO:302), rs385787-rs367001_F:CCATCATGGAAAGCATGG (SEQ ID NO:303) and rs385787-rs367001_R: TCATCTCCATGACTGCACTA (SEQ ID NO:304), rs367001-rs386095_F: GAGATGACGGAGTAGCTCAT (SEQ ID NO:305) and rs367001-rs3860958R: CCCAGCTGCACTGTCTAC (SEQ ID NO:306), rs2837296-rs2837297_F: TCTTGTTCCAATCACAGGAC (SEQ ID NO:307) and rs2837296-rs2837297_R: ATGCTGTTAGCTGAAGCTCT (SEQ ID NO:308), and rs2837381-rs4816672_F: TGAAAGCTCCTAAAGCAGAG (SEQ ID NO:309) andrs2837381-rs4816672_R:TTGAAGAGATGTGCTATCAT (SEQ ID NO:310). Polynucleotide sequences *e.g.* GC clamp sequences, can be included

to ensure specific hybridization of AT-rich primers (Ghanta *et al.,* PLOS ONE 5(10): doi10.1371/journal.pone.0013184 [2010], available on the world wide web at plosone.org). An example of a GC clamp sequence that can be included either 5' of the forward primer or 3' of the reverse primer is GCCGCCTGCAGCCCGCGCCCCCCGTGCCCCCGCCCCGCCGCCGGCCCGGGCGCC (SEQ ID NO:311).

Polymorphic sequences can be used alone or in combination with unamplified cfDNA to determine either fetal fraction or the presence or absence of aneuploidy and fetal fraction in a maternal sample as described for polymorphic SNP sequences. Sample preparation and enrichment of cfDNA sequencing library, a purified cfDNA sample, and a plasma sample is performed according to the method described in Examples 5, 6, and 7, respectively. All sequencing libraries are prepared as described in Example 2a., and sequencing is performed as described in Example 2b. Analysis of the sequencing data for the determination of fetal aneuploidy is performed as described in Example 5. Concomitant to the analysis for determining aneuploidy, the sequencing data is analyzed to determine the fetal fraction as follows. Following the transfer of the image and base call files to the Unix server running the Illumina "Genome Analyzer Pipeline" software version 1.51 as described in Example 3a., the 36bp reads are aligned to a 'tandem SNP genome' using the BOWTIE program. The tandem SNP genome is identified as the grouping of the DNA sequences that encompass the alleles of the 58 tandem SNP pairs disclosed above. Only reads that mapped uniquely to the tandem SNP genome are used for the analysis of fetal fraction. Reads that match perfectly to the tandem SNP genome are counted as tags and filtered. Of the remaining reads, only reads having one or two mismatches are counted as tags and included in the analysis. Tags mapped to each of the tandem SNP alleles are counted, and the fetal fraction is determined essentially as described in Example 6 above but accounting for tags mapped to the two tandem SNP alleles x and y present on each of the amplified polymorphic target nucleic acid sequences that are amplified to enrich the samples *i.e.*

$$\%\ \text{fetal fraction allele}_{x+y} = ((\sum \text{Fetal sequence tags for allele}_{x+y}) / (\sum \text{Maternal sequence tags for allele}_{x+y})) \times 100$$

Only informative tandem SNPs are used to determine the fetal fraction.

Optionally, the fraction of fetal nucleic acids in the mixture of fetal and maternal nucleic acids is calculated for each of the informative allele (allele$_{x+y}$) as follows:

$$\%\ \text{fetal fraction allele}_{x+y} = ((2 \text{ X } \sum \text{Fetal sequence tags for allele}_{x+y}) / (\sum \text{Maternal sequence tags for allele}_{x+y})) \times 100,$$

to compensate for the presence of 2 sets of tandem fetal alleles, one being masked by the maternal background.

The percent fetal fraction is calculated for at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, or more informative sets of tandem alleles. In one embodiment, the fetal fraction is the average fetal fraction determined for at least 3 informative sets of tandem alleles.

**Example 9**

**Determination of Fetal Fraction by Massively Parallel Sequencing of Samples Comprising Amplified Polymorphic Sequences: STRs**

To determine the fraction of fetal cfDNA in a maternal sample, target polymorphic nucleic acid sequences each comprising an STR are amplified and used for preparing a target library for sequencing in a massively parallel fashion.

Peripheral blood samples are obtained from pregnant subjects, and cfDNA is purified from the plasma fraction as described in Examples1 and 2 STRs that are amplified are chosen from the codis and non-codis STRs disclosed in Table 7, and amplification of the polymorphic STR sequences is obtained using the corresponding sets of primers provided. For example, the STRs listed in Table 7 are amplified using the corresponding primers (SEQ ID NOs: 113-197), and the amplified product is used to generate a target sequencing library. The STR target sequencing library is prepared as described for the preparation of the SNP target library as described in Example 8. STRs CSF1PO, D13S317, D16S539, D18S51, D21S11, D2S1338D7S820, and FGA have been analyzed previously for determining fetal fraction, and are disclosed in US Provisional applications 61/296,358 and 61/360,837.

**TABLE 7**

| CODIS and NON-CODIS miniSTRs | | | | |
|---|---|---|---|---|
| **STR Locus (Marker Name)** | **Chromosome Location** | **Size Range (bp)** | **GenBank Accession** | **Primer Sequences (Forward/Reverse)** |
| **Codis miniSTR loci*** | | | | |
| CSF1PO | 5q33.1 | 89-129 | X14720 | ACAGTAACTGCCTTCATAGATAG (CSF1PO_F; SEQ ID NO: 113)<br>GTGTCAGACCCTGTTCTAAGTA (CSF1PO_R; SEQ ID NO:114) |
| FGA | 4q31.3 | 125-281 | M64982 | AAATAAAATTAGGCATATTTACAAGC (FGA_F; SEQ ID NO: 115)<br>GCTGAGTGATTTGTCTGTAATTG(FGA_R; SEQ ID NO: 116) |
| TH01 | 11p15.5 | 51-98 | D00269 | CCTGTTCCTCCCTTATTTCCC(TH01_F; SEQ ID NO: 117)<br>GGGAACACAGACTCCATGGTG(TH01_R; SEQ ID NO: 118) |
| TPOX | 2p25.3 | 65-101 | M68651 | CTTAGGGAACCCTCACTGAATG(TPOX_F ; SEQ ID NO:119)<br>GTCCTTGTCAGCGTTTATTTGC(TPOX_R; SEQ ID NO: 120) |
| vWA | 12p13.31 | 88-148 | M25858 | AATAATCAGTATGTGACTTGGATTGA(v WA_F; SEQ ID NO:121)<br>ATAGGATGGATGGATAGATGGA(vWA_R ; SEQ ID NO: 122) |
| D3S1358 | 3p21.31 | 72-120 | NT_005997 | CAGAGCAAGACCCTGTCTCAT(D3S1358_ F; SEQ ID NO: 123)<br>TCAACAGAGGCTTGCATGTAT(D3 S 1358_ R; SEQ ID NO: 124) |
| D5S818 | 5q23.2 | 81-117 | AC008512 | GGGTGATTTTCCTCTTTGGT(D5S818_F; SEQ ID NO: 125)<br>AACATTTGTATCTTTATCTGTATCCTTAT TTAT(D5S818_R; SEQ ID NO: 126) |
| D7S820 | 7q21.11 | 136-176 | AC004848 | GAACACTTGTCATAGTTTAGAACGAAC( D7S 820_F; SEQ ID NO: 127)<br>TCATTGACAGAATTGCACCA(D7S820_R; SEQ ID NO: 128) |
| D8S1179 | 8q24.13 | 86-134 | AF216671 | TTTGTATTTCATGTGTACATTCGTATC(D 7S820_F; SEQ ID NO: 129)<br>ACCTATCCTGTAGATTATTTTCACTGTG( D7S 820_R; SEQ ID NO: 130) |
| D13S317 | 13q31.1 | 88-132 | AL353628 | TCTGACCCATCTAACGCCTA(D13S317_F; SEQ ID NO:131)<br>CAGACAGAAAGATAGATAGATGATTGA( D13 S317_R; SEQ ID NO:132) |
| D16S539 | 16q24.1 | 81-121 | AC024591 | ATACAGACAGACAGACAGGTG(D 16S539 _F; SEQ ID NO:133) |

(continued)

| CODIS and NON-CODIS miniSTRs | | | | |
|---|---|---|---|---|
| **STR Locus (Marker Name)** | **Chromosome Location** | **Size Range (bp)** | **GenBank Accession** | **Primer Sequences (Forward/Reverse)** |
| **Codis miniSTR loci*** | | | | |
| | | | | GCATGTATCTATCATCCATCTCT(D16S53 9_R; SEQ ID NO:134) |
| D18S51 | 18q21.33 | 113-193 | AP001534 | TGAGTGACAAATTGAGACCTT(D18S51_F ; SEQ ID NO:135)<br>GTCTTACAATAACAGTTGCTACTATT(D1 8S51_R; SEQ ID NO:136) |
| D21S11 | 21q21.1 | 153-221 | AP000433 | ATTCCCCAAGTGAATTGC(D21S 11_F; SEQ ID NO: 137)<br>GGTAGATAGACTGGATAGATAGACGA(D 21S11_R; SEQ ID NO:138) |
| D2S1338 | 2q35 | 90-142 | AC01036 | TGGAAACAGAAATGGCTTGG(D2S1338_F ; SEQ ID NO:139)<br>GATTGCAGGAGGGAAGGAAG(D2S1338_ R; SEQ ID NO: 140) |
| Penta D | 21q22.3 | 94-167 | AP001752 | GAGCAAGACACCATCTCAAGAA(Penta D_F; SEQ ID NO:141)<br>GAAATTTTACATTTATGTTTATGATTCTC T(Penta D_R; SEQ ID NO:142) |
| Penta E | 15q26.2 | 80-175 | AC027004 | GGCGACTGAGCAAGACTC(Penta E _F; SEQ ID NO: 143)<br>GGTTATTAATTGAGAAAACTCCTTACA( Penta E _R; SEQ ID NO: 144) |
| **Non-Codis miniSTR loci*** | | | | |
| D22S1045 | 22q12.3 | 82-115 | AL022314 (17) | ATTTTCCCCGATGATAGTAGTCT(D22S10 45_F; SEQ ID NO:145)<br>GCGAATGTATGATTGGCAATATTTTT(D2 2S1045_R; SEQ ID NO:146) |
| D20S1082 | 20q13.2 | 73 - 101 | AL158015 | ACATGTATCCCAGAACTTAAAGTAAAC( D20S 1082_F; SEQ ID NO:147)<br>GCAGAAGGGAAAATTGAAGCTG(D20S 10 82_R; SEQ ID NO:148) |
| D20S482 | 20p13 | 85 - 126 | AL121781 (14) | CAGAGACACCGAACCAATAAGA(D20S48 2_F; SEQ ID NO:149)<br>GCCACATGAATCAATTCCTATAATAAA( D20S 482_R; SEQ ID NO:150) |
| D18S853 | 18p11.31 | 82 - 104 | AP005130 (11) | GCACATGTACCCTAAAACTTAAAAT(D 18 S853_F; SEQ ID NO:151)<br>GTCAACCAAAACTCAACAAGTAGTAA(D 18S853_R; SEQ ID NO:152) |
| D17S1301 | 17q25.1 | 114 - 139 | AC016888 (12) | AAGATGAAATTGCCATGTAAAAATA(D1 7S1301_F; SEQ ID NO:153) |

(continued)

| Non-Codis miniSTR loci* | | | | |
|---|---|---|---|---|
| | | | | GTGTGTATAACAAAATTCCTATGATGG( D17S 1301_R; SEQ ID NO:154) |
| D17S974 | 17p13.1 | 114 - 139 | AC034303 (10) | GCACCCAAAACTGAATGTCATA(D17S97 4_F; SEQ ID NO:155) GGTGAGAGTGAGACCCTGTC(D17S974_R ; SEQ ID NO:156) |
| D14S1434 | 14q32.13 | 70 - 98 | AL121612 (13) | TGTAATAACTCTACGACTGTCTGTCTG(D 14S1434_F; SEQ IDNO:157) GAATAGGAGGTGGATGGATGG(D 14S 143 4_R; SEQ ID NO:158) |
| D12ATA63 | 12q23.3 | 76 - 106 | AC009771 (13) | GAGCGAGACCCTGTCTCAAG(D 12ATA63 _F; SEQ ID NO:159) GGAAAAGACATAGGATAGCAATTT(D 12 ATA63_R; SEQ ID NO:160) |
| D11S4463 | 11q25 | 88 - 116 | AP002806 (14) | TCTGGATTGATCTGTCTGTCC(D 11S4463_ F; SEQ ID NO:161) GAATTAAATACCATCTGAGCACTGAA(D 11S4463_R; SEQ ID NO:162) |
| D10S1435 | 10p15.3 | 82 - 139 | AL354747 (11) | TGTTATAATGCATTGAGTTTTATTCTG(D 10S1435_F; SEQ ID NO:163) GCCTGTCTCAAAAATAAAGAGATAGAC A(D10S1435_R; SEQ ID NO:164) |
| D10S1248 | 10q26.3 | 79 - 123 | AL391869 (13) | TTAATGAATTGAACAAATGAGTGAG(D1 OS1248_F; SEQ ID NO:165) GCAACTCTGGTTGTATTGTCTTCAT(D 1 0S 1248_R; SEQ ID NO:166) |
| D9S2157 | 9q34.2 | 71 - 107 | AL162417 (10) | CAAAGCGAGACTCTGTCTCAA(D9S2157_ F; SEQ ID NO:167) GAAAATGCTATCCTCTTTGGTATAAAT( D9S2 157_R; SEQ ID NO:168) |
| D9S1122 | 9q21.2 | 93 - 125 | AL161789 (12) | GGGTATTTCAAGATAACTGTAGATAGG( D9S1122_F; SEQ ID NO:168) GCTTCTGAAAGCTTCTAGTTTACC(D9S 11 22_R; SEQ ID NO:170) |
| D8S1115 | 8p11.21 | 63 - 96 | AC090739 (9) | TCCACATCCTCACCAACAC(D8S1115_F; SEQ ID NO:171) GCCTAGGAAGGCTACTGTCAA(D8S 1115_ R; SEQ ID NO:172) |
| D6S1017 | 6p21.1 | 81 - 110 | AL035588 (10) | CCACCCGTCCATTTAGGC(D6S1017_F; SEQ ID NO:173) GTGAAAAAGTAGATATAATGGTTGGTG( D6S 1017_R; SEQ ID NO:174) |
| D6S474 | 6q21 | 107 - 136 | AL357514 (17) | GGTTTTCCAAGAGATAGACCAATTA(D6S 474_F; SEQ ID NO:175) GTCCTCTCATAAATCCCTACTCATATC(D 6S474_R; SEQ ID NO:176) |

(continued)

| Non-Codis miniSTR loci* | | | | |
|---|---|---|---|---|
| D5S2500 | 5q11.2 | 85 - 126 | AC008791 (17) | CTGTTGGTACATAATAGGTAGGTAGGT( D5S 2500_F; SEQ ID NO:177) GTCGTGGGCCCCATAAATC(D5S2500_R; SEQ ID NO:178) |
| D4S2408 | 4p15.1 | 85 - 109 | AC 110763 (9) | AAGGTACATAACAGTTCAATAGAAAGC( D4S 2408_F; SEQ ID NO:179) GTGAAATGACTGAAAAATAGTAACCA(D 4S2408_R; SEQ ID NO:180) |
| D4S2364 | 4q22.3 | 67 - 83 | AC022317 (9) | CTAGGAGATCATGTGGGTATGATT(D4S2 364U_F; SEQ ID NO:181) GCAGTGAATAAATGAACGAATGGA(D4S 2364_R; SEQ ID NO:182) |
| D3S4529 | 3p12.1 | 111 - 139 | AC117452 (13) | CCCAAAATTACTTGAGCCAAT(D3S452_F ; SEQ ID NO:183) GAGACAAAATGAAGAAACAGACAG(D3 S 452_R; SEQ ID NO:184) |
| D3S3053 | 3q26.31 | 84 - 108 | AC069259 (9) | TCTTTGCTCTCATGAATAGATCAGT(D3S 3053_F; SEQ ID NO:185) GTTTGTGATAATGAACCCACTCAG(D3 S3 053_R; SEQ ID NO:186) |
| D2S1776 | 2q24.3 | 127 - 161 | AC009475 (11) | TGAACACAGATGTTAAGTGTGTATATG( D2S1 776_F; SEQ ID NO:187) GTCTGAGGTGGACAGTTATGAAA(D2S 17 76_R; SEQ ID NO:188) |
| D2S441 | 2p14 | 78 - 110 | AC079112 (12) | CTGTGGCTCATCTATGAAAACTT(D2S441 F; SEQ ID NO:189) GAAGTGGCTGTGGTGTTATGAT(D2S441 R; SEQ ID NO:190) |
| D1S1677 | 1q23.3 | 81 - 117 | AL513307 (15) | TTCTGTTGGTATAGAGCAGTGTTT(D 1 S 16 77_F; SEQ ID NO:191) GTGACAGGAAGGACGGAATG(D 1S1677_ R; SEQ ID NO: 192) |
| D1S1627 | 1p21.1 | 81 - 100 | AC093119 (13) | CATGAGGTTTGCAAATACTATCTTAAC( D1S1 627_F; SEQ ID NO:193) GTTTTAAT'ITTCTCCAAATCTCCA(D 1 S 16 27_R; SEQ ID NO:194) |
| D1GATA113 | 1p36.23 | 81 - 105 | Z97987 (11) | TCTTAGCCTAGATAGATACTTGCTTCC(D 1GATA113_F; SEQ ID NO:195) GTCAACCTTTGAGGCTATAGGAA(D1GA TA113_R; SEQ ID NO:196) |
| *(Butler *et al.,* J Forensic Sci 5:1054-1064; Hill *et al.,* Poster #44- 17th International Symposium on Human Identification - 2006) | | | | |

Sequencing of the library enriched for polymorphic STR sequences is performed using a NGS technology *e.g.* sequencing by synthesis. Sequence reads of lengths that encompass the STRs *e.g.* miniSTRs of at least 100 bp, to a reference STR genome consisting of the polymorphic sequences which were amplified in the sample. Informative STR alleles are identified by differences in the length of the repeats, and the number of STR sequence tags are counted, and

used to determine the fetal fraction. Optionally, amplification of the polymorphic STR sequences is performed to enrich a plasma sample, a purified cfDNA. sample or a cfDNA sequencing library sample, as described in Examples 5, 6, and 7, respectively.

**Example 10**

**Determination of Fetal Fraction by Capillary Electrophoresis of Polymorphic Sequences Comprising STRs**

To determine fetal fraction in maternal samples comprising fetal and maternal cfDNA, peripheral blood samples were collected from volunteer pregnant women carrying either male or female fetuses. Peripheral blood samples were obtained and processed to provide purified cfDNA as described in Example 1

Ten microliters of cfDNA samples were analyzed using the AmpFISTR® MiniFiler™ PCR amplification kit (Applied Biosystems, Foster City, CA) according to the manufacturer's instructions. Briefly, cfDNA contained in 10 μl was amplified in a reaction volume of 25 μl containing 5μL fluorescently labeled primers (AmpF/STR® MiniFiler™ Primer Set), and the AmpF/STR® MiniFiler™ Master Mix, which includes AmpliTaq Gold® DNA polymerase and associated buffer, salt (1.5 mM MgC12), and 200 μM deoxynucleotide triphosphates (dNTPs: dATP, dCTP, dGTP and dTTP). The fluorescently labeled primers are forward primers that are labeled with 6FAM™, VIC™, NED™, and PET™ dyes. Thermal cycling was performed with the Gene Amp9700 (Applied Biosystems) using the following cycling conditions: incubating at 95°C for 10 minutes, followed by 30 cycles at 94°C for 20 seconds, 59°C for 2 minute, and 72°C for 1 minute, which was followed by a final incubation at 60°C for 45 minutes. A final hold at 4°C was added until the samples were removed for analysis. The amplified product was prepared by diluting 1ul of amplified product in 8.7ul Hi-DiTM formamide (Applied Biosystems) and 0.3 μl GeneScanTM-500 LIZ_ internal size standard (Applied Biosystems), and analyzed with an ABI PRISM3130x1 Genetic Analyzer (Applied Biosystems) using Data Collection HID_G5_POP4 (Applied Biosystems), and a 36-cm capillary array. All genotyping was performed with GeneMapper_ID v3.2 software (Applied Biosystems) using manufacturer provided allelic ladders and bins and panels.

All genotyping measurement were performed on the Applied Biosystems 3130*xl* Genetic Analyzer, using a ±0.5-nt "window" around the size obtained for each allele to allow for detection and correct assignment of alleles. Any sample allele whose size was outside the ±0.5-nt window was determined to be OL *i.e.* "Off Ladder". OL alleles are alleles of a size that is not represented in the AmpF/STR® MiniFiler™ Allelic Ladder or an allele that does not correspond to an allelic ladder, but whose size is just outside a window because of measurement error. The minimum peak height threshold of >50 RFU was set based on validation experiments performed to avoid typing when stochastic effects are likely to interfere with accurate interpretation of mixtures. The calculation of fetal fraction is based on averaging all informative markers. Informative markers are identified by the presence of peaks on the electropherogram that fall within the parameters of preset bins for the STRs that are analyzed.

Calculations of fetal fraction were performed using the average peak height for major and minor alleles at every STR locus determined from triplicate injections. The rules applied to the calculation are:

1. off-ladder allele (OL) data for alleles are not included in the calculation; and
2. only peak heights derived from >50 RFU (relative fluorescence units) are included in the calculation
3. if only one bin is present the marker is deemed non-informative; and
4. if a second bin is called but the peaks of the first and second bins are within 50-70% of their relative fluorescence units (RFU) in peak height, the minority fraction is not measured and the marker is deemed not informative.

The fraction of the minor allele for any given informative marker is calculated by dividing the peak height of the minor component by the sum of the peak height for the major component, and expressed as a percent was first calculated for each informative locus as

$$\text{fetal fraction} = (\sum \text{peak height of minor allele} / \sum \text{peak height of major allele(s)}) \times 100,$$

The fetal fraction for a sample comprising two or more informative STRs, would be calculated as the average of the fetal fractions calculated for the two or more informative markers.

Table 8 provides the data obtained from analyzing cfDNA of a subject pregnant with a male fetus.

**TABLE 8**

| Fetal Fraction Determined in cfDNA of a Pregnant Subject by Analysis of STRs | | | | | | | | |
|---|---|---|---|---|---|---|---|---|
| STR | Allele 1 | Allele 2 | Allele 3 | Allele1 Height | Allele 2 Height | Allele 3 Height | Fetal Fraction | Fetal Fraction (Mean/STR) |
| AMEL | X | Y | | 3599 | 106 | | 2.9 | |
| AMEL | X | Y | | 3602 | 110 | | 3.1 | |
| AMEL | X | Y | | 3652 | 109 | | 3.0 | 3.0 |
| CSF1PO | 11 | 12 | | 2870 | 2730 | | | |
| CSF1PO | 11 | 12 | | 2924 | 2762 | | | |
| CSF1PO | 11 | 12 | | 2953 | 2786 | | | |
| D13S317 | 11 | 12 | | 2621 | 2588 | | | |
| D13S317 | 11 | 12 | | 2680 | 2619 | | | |
| D13S317 | 11 | 12 | | 2717 | 2659 | | | |
| D16S539 | 9 | 11 | | 1056 | 1416 | | | |
| D16S539 | 9 | 11 | | 1038 | 1394 | | | |
| D16S539 | 9 | 11 | | 1072 | 1437 | | | |
| D18S51 | 13 | 15 | | 2026 | 1555 | | | |
| D18S51 | 13 | 15 | | 2006 | 1557 | | | |
| D18S51 | 13 | 15 | | 2050 | 1578 | | | |
| D21S11 | 28 | 31.2 | | 2450 | 61 | | 2.5 | |
| D21S11 | 28 | 31.2 | | 2472 | 62 | | 2.5 | |
| D21S11 | 28 | 31.2 | | 2508 | 67 | | 2.7 | 2.6 |
| D2S1338 | 20 | 23 | | 3417 | 3017 | | | |
| D2S1338 | 20 | 23 | | 3407 | 3020 | | | |
| D2S1338 | 20 | 23 | | 3493 | 3055 | | | |
| D7S820 | 9 | 12 | 13 | 2373 | 178 | 1123 | 5.1 | |
| D7S820 | 9 | 12 | 13 | 2411 | 181 | 1140 | 5.1 | |
| D7S820 | 9 | 12 | 13 | 2441 | 182 | 1156 | 5.1 | 5.1 |
| FGA | 17.2 | 22 | 25 | 68 | 1140 | 896 | 3.3 | |
| FGA | 17.2 | 22 | 25 | 68 | 1144 | 909 | 3.1 | |
| FGA | 17.2 | 22 | 25 | 68 | 1151 | 925 | 3.3 | 3.2 |
| **Fetal Fraction = 3.5** | | | | | | | | |

The results show that cfDNA can be used for determining the presence or absence of fetal DNA as indicated by the detection of a minor component at one or more STR alleles, for determining the percent fetal fraction, and for determining fetal gender as indicated by the presence or absence of the Amelogenin allele.

**Example 11**

**Preamplification of cfDNA for Determining Fetal Fraction by Capillary Electrophoresis of Polymorphic Sequences Comprising STRs**

To improve the sensitivity of the STR assay in detecting and quantifying the STR alleles in the minor contributor of the cfDNA sample, the number of starting genomes in the artificial samples was increased by a modified whole genome

amplification strategy.

Peripheral blood samples were collected and processed as described in Example 2. Cell-free DNA was extracted from 1 ml cell-free plasma using the Roche MagNA Pure Compact Nucleic Acid Isolation Kit I - Large Volume (Roche Applied Science, IN) using the MagNA Pure Compact Instrument, and eluted in 50 μl of elution buffer. Ten microliters of the extracted cfDNA were used to quantify the cfDNA, and the remainder was stored (see storage instructions WI0035 Clinical Sample Storage). The concentration of the plasma extracted cfDNA was determined by fluorescence-based quantitation with UV absorbance measurements using the Qubit™ Quantitation Platform (Invitrogen).

The concentration of cfDNA quantified in plasma samples prepared using the MagnaPure Nucleic Acid Isolation Kit I from 16 pregnant subjects was determined to range between 20 and 100 pg/μl. As the fetal component of plasma cfDNA is known to contribute 3-10% of the total plasma cfDNA, artificial plasma samples were created by spiking aliquots of cfDNA derived from plasma of female volunteer subjects with cfDNA extracted from plasma of male volunteer subjects to mimic the ratios of fetal to maternal cfDNA found in the pregnant subjects. Artificial samples were created to contain 200-1000pg of extracted female cfDNA that was spiked with 45-150 pg of extracted male cfDNA in a total volume of 10 μl. Each artificial sample was spiked to contain 3%, 5% and 10% male cfDNA.

Artificial samples having concentrations of total cfDNA of less than approximately 50 pg/μl, were preamplified using the modified improved primer extension amplification PCR (mIPEP) amplification according to the method of Hanson and Ballantyne, (Hanson and Ballantyne, Analytical Biochem 346:246-257 [2005]) as follows. Ten microliters of spiked plasma cfDNA were amplified in a 25 μl reaction volume containing 1mM dNTPs, 2.5mM $MgCl_2$ (Applied Biosystems), 1X Expand High Fidelity Buffer (No.3), 10.5U Expand High Fidelity Enzyme Mix (Roche Diagnostics), and 40 μM PEP primer (5' -NNNNNNNNNNNNNNN-3', Qiagen). The amplification was performed in a GeneAmp PCR System 9700 Thermocycler under the following conditions: (1) 20 and 30 cycles of 94°C for 1 minute, 37°C for 2 minutes, and 0.1°C/s ramp to 55°C for 4 minutes. The amplification product was purified using a Qiagen column. The concentration of the amplification product was determined using the Qubit™ Quantitation Platform as described above. STR analysis was performed as described in Example 9 above, except that only peak heights >100 RFU were included in the calculations.

The results are shown in Tables 9, 10 and 11. The results provided in Table 9 show that the cfDNA contained in 10μl cfDNA of artificial samples ART23 and ART24 having a starting concentration of cfDNA of 46.2 and 50.2 pg/μl, respectively, was amplified by approximately 5 and 10 fold following 20 and 30 cycles of PCR amplification, respectively.

These data indicate that a pre-amplification of cfDNA using the mIPEP method provided enhanced levels of total cfDNA rendering the level of the minor component more amenable to the STR analysis.

**TABLE 9**

| **Preamplification with mIPEP** | | | |
|---|---|---|---|
| SAMPLE | cfDNA without mIPEP (pg/μl) | cfDNA with mIPEP:20 PCR cycles (pg/50μl) | cfDNA with mIPEP:30 PCR cycles (pg/50μl) |
| ART23 | 46.2 | 2265 | 4125 |
| ART24 | 50.2 | 2085 | 3875 |

Table 10 shows triplicate measurements profiling 9 loci of the cfDNA of spiked samples ART23 and ART24 following the mIPEP procedure with 20 and 30 cycles of amplification, as described above.

The data in Table 11 indicate that pre-amplification of cfDNA enables the detection and quantification of the minor component at most loci tested in artificially mixed samples having a starting cfDNA concentration that would otherwise not permit an accurate analysis of the minor STR alleles.

**TABLE 10**

| mIPEP Preamplification and Detection of Minor Component | | | | | | | | |
|---|---|---|---|---|---|---|---|---|
| | | **ART23 (453pg) mIPEP amplified 20 cycles** | **ART23 (82 5pg) mIPEP amplified 20 cycles** | **ART23 (462pg) Extracted unamplified cfDNA** | | **ART24 (417pg) mIPEP amplified 30 cycles** | **ART24 (775pg) mIPEP amplified 30 cycles** | **ART24 (502pg) Extracted unamplified cfDNA** |
| **STR Locus** | **Allele** | **Allele Height** | **Allele Height** | **Allele Height** | **Allele** | **Allele Height** | **Allele Height** | **Allele Height** |
| **AMEL** | X/Y | 291/95 | 397/170 | 535/832 | X/Y | 695/359 | 1878/1148 | 1564/1959 |
| **AMEL** | X/Y | 425/147 | 428/188 | 675/1048 | X/Y | 1216/619 | 1551/954 | 1573/1943 |
| **AMEL** | X/Y | 267/94 | 455/203 | 664/1043 | X/Y | 718/363 | 1479/924 | 1621/2024 |
| **CSF1PO** | 10/11 | 800/979 | 725/1009 | 1429/1325 | 11/12 | 2029/1317 | 4159/2317 | 2990/3083 |
| **CSF1PO** | 10/11 | 1147/1432 | 789/1102 | 1779/1650 | 11/12 | 3449/2223 | **3460/113/1890** | 2996/3118 |
| **CSF1PO** | 10/11 | 729/906 | 831/1162 | 1783/1657 | 11/12 | 2006/1309 | 3362/1840 | 3072/3183 |
| **D13S317** | 12 | 743 | 515 | 1229 | 11 | 955 | 1490 | 3634 |
| **D13S317** | 12 | 1079 | 563 | 1534 | 11 | 1631 | 1198 | 3631 |
| **D13S317** | 12 | 668 | 583 | 1520 | 11 | 968 | 1170 | 3795 |
| **D16S539** | 9/10 | 239/140 | 370/466 | 835/676 | 10/11 | 513/512 | 1173/1472 | 1678/973 |
| **D16S539** | 9/10 | 347/203 | 64*(OL)/3 91/489 | 1046/864 | 10/11 | 859/870 | **973/1212** | 1730/999 |
| **D16S539** | 9/10 | 227/134 | 441/515 | 1055/860 | 10/11 | 530/513 | 960/1183 | 1784/1044 |
| **D18S51** | 14/15 | 359/464 | 363/220 | 785/541 | 12/18 | 1044/576 | 1840/786 | 2559/1507 |
| | | 512/645 | 391/226 | 999/672 | 12/18 | 1769/994 | 1511/643 | 2565/1469 |
| | | 313/402 | 409/245 | 994/685 | 12/18 | 1033/567 | 1496/631 | 2643/1523 |
| **D21S11** | 29/32 | 103/104 | 114/173 | 605/413 | 31.2 | 381 | 661 | 3276 |
| | | 149/153 | 130/182 | 759/523 | 31.2 | 650 | 536 | 3028 |
| | | 85/86 | 131/196 | 760/525 | 31.2 | 380 | 520 | 3282 |
| **D2S1338** | 18/20 | 572/383 | 428/363 | 1116/1013 | 19/20 | 1066/433 | 2315/1243 | 2962/2968 |
| | | 827/553 | 454/386 | 1428/1279 | 19/20 | 1821/757 | 1901/101 | 2942/2942 |

(continued)

| mIPEP Preamplification and Detection of Minor Component | | | | | | | | |
|---|---|---|---|---|---|---|---|---|
| | | ART23 (453pg) mIPEP amplified 20 cycles | ART23 (82 5pg) mIPEP amplified 20 cycles | ART23 (462pg) Extracted unamplified cfDNA | | ART24 (417pg) mIPEP amplified 30 cycles | ART24 (775pg) mIPEP amplified 30 cycles | ART24 (502pg) Extracted unamplified cfDNA |
| | | 530/351 | 482/408 | 1431/1275 | 19/20 | 1063/444 | 1859/1012 | 3072/3067 |
| **D7S820** | 11/12 | 262/167 | 149/270 | 557/627 | 11/12 | 256/138 | 520/322 | 1550/1548 |
| | | 62/366/23 1 | 162/292 | 699/775 | 11/12 | 448/236 | 419/258 | 1484/1466 |
| | | 224/146 | 169/307 | 689/779 | 11/12 | 253/141 | 406/250 | 1579/1573 |
| **FGA** | 21/23 | 263/146 | 181/88 | 596/365 | 22/24 | 228/244 | 375/429 | 1272/1064 |
| | | 384/215 | 191/92 | 762/450 | 22/24 | 409/425 | 303/345 | 1221/1023 |
| | | 230/136 | 202/102 | 749/456 | 22/24 | 232/250 | 297/348 | 1298/1087 |
| *"OL" means "Off Ladder measurement" | | | | | | | | |

**TABLE 11**

| Fetal Fraction Determined in a Sample Following Preamplification Using mIPEP | | | | | | |
|---|---|---|---|---|---|---|
| **STR marker** | **Allele 1/ Height** | **Allele 2/ Height** | **Allele 3/ Height** | **Allele 4/ Height** | **Percent minor fraction/STR - minor >100 RFU** | **Percent minor fraction/STR - minor <100 RFU** |
| Amelogenin | X/2799 | Y/207 | | | | |
| Amelogenin | X/2751 | Y/198 | | | | |
| Amelogenin | X/3109 | Y/232 | | | | |
| | **X/2886** | **Y/212** | | | **7** | |
| CSF1PO | 10/2377 | 11/1869 | 12/508 | | | |
| CSF1PO | 10/2299 | 11/1814 | 12/498 | | | |
| CSF1PO | 10/2616 | 11/206 | 12/562 | | | |
| | **10/2431** | **11/1917** | **12/523** | | **12** | |
| D13S317 | 10/1232 | 11/1600 | 13/186 | | | |
| D13S317 | 10/1208 | 11/1548 | 13/182 | | | |
| D13S317 | 10/1386 | 11/1758 | 13/212 | | | |
| | **10/1275** | **11/1635** | **13/193** | | **12** | |
| D16S539 | 11/757 | 12/933 | | | | |
| D16S539 | 11/729 | 12/885 | | | | |
| D16S539 | 11/836 | 12/1031 | | | | |
| | **11/774** | **12/950** | | | **12** | |
| D18S51 | <u>OL/80</u> | 14/3137 | 15/371 | | | |
| D18S51 | <u>11/73</u> | 14/3082 | 15/362 | | | |
| D18S51 | <u>OL/83</u> | 14/3488 | 15/413 | | | |
| | **<u>OL</u>** | **14/3236** | **15/382** | | | |
| D21S11 | 29/953 | 30/941 | | | | |
| D21S11 | 29/921 | 30/908 | | | | |
| D21S11 | 29/1046 | 30/1045 | | | | |
| | **29/973** | **30/965** | | | | |
| D2S1338 | 17/461 | 18/366 | 20/2280 | 24/1760 | | |
| D2S1338 | 17/460 | 18/360 | 20/2240 | 24/1712 | | |
| D2S1338 | 17/508 | 18/409 | 20/2563 | 24/1971 | | |
| | **17/476** | **18/378** | **20/2361** | **24/1814** | **20** | |
| D7S820 | 8/1409 | 9/60 | 12/1059 | | | |
| D7S820 | 8/1380 | 9/60 | 12/1036 | | | |
| D7S820 | 8/1561 | 9/69 | 12/1166 | | | |
| | **8/1450** | **9/63** | **12/1087** | | | **2** |
| FGA | 19/825 | 21/850 | 25/279 | | | |
| FGA | 19/807 | 21/841 | 25/265 | | | |
| FGA | 19/913 | 21/958 | 25/306 | | | |

(continued)

| Fetal Fraction Determined in a Sample Following Preamplification Using mIPEP | | | | | | |
|---|---|---|---|---|---|---|
| **STR marker** | **Allele 1/ Height** | **Allele 2/ Height** | **Allele 3/ Height** | **Allele 4/ Height** | **Percent minor fraction/STR - minor >100 RFU** | **Percent minor fraction/STR - minor <100 RFU** |
| | **19/848** | **21/883** | **25/283** | | **16** | |
| % fetal fraction > 100RFU for minor allele →12 | | | | | **12** | |
| % fetal fraction including < 100RFU for minor allele →11 | | | | | | **11** |

**Example 12**

**Correlation of Fetal Fraction Determined by Analysis of Fetal and Maternal SNPs and STRs**

To verify that the calculated fetal fraction *i.e.* fraction of minor nucleic acid component, determined using the SNP and STR assay as described in the preceding Examples provided an accurate measurement of the fetal fraction, the percent fetal fraction of cfDNA in the plasma from the same pregnant subjects was compared.

Peripheral blood samples were obtained from 48 volunteer subjects, 24 of the subjects were pregnant with male fetuses, and 24 were pregnant with female fetuses. cfDNA was prepared as described in Example 1, Fetal fraction using SNPs was determined by massively parallel sequencing by synthesis as described in Example 5, and fetal fraction using STRs was determined using capillary electrophoresis as described in Example 10

The results shown in **Figure 6** indicate that a positive correlation exists between the fraction determined using the STR assay and the fraction determined using the SNP sequencing. These data further validate the use of polymorphic sequences comprising STRs or SNPs for determining the fraction of fetal cfDNA in a plasma sample.

**Example 13**

**Use of Fetal Fraction to Set Thresholds and Estimate Minimum Sample Size in Aneuploidy Detection**

Counts of sequence matches to different chromosomes are manipulated to generate a score which will vary with chromosomal copy number that can be interpreted to identify chromosomal amplification or deletion. For example, such a score could be generated by comparing the relative amount of a sequence tags on a chromosome undergoing copy number changes to a chromosome known to be a euploid. Examples of scores that can be used to identify amplification or deletion include but are not limited to: counts for the chromosome of interest divided by counts of another chromosome from the same experimental run, the counts for the chromosome of interest divided by the total number of counts from the experimental run, comparison of counts from the sample of interest versus a separate control sample. Without loss of generality, it can be assumed that scores will increase as copy number increases. Knowledge of fetal fraction can be used to set "cutoff' thresholds to call "aneuploidy", "normal", or "marginal" (uncertain) states. Then, calculations are performed to estimate the minimum number of sequences required to achieve adequate sensitivity (i.e. probability of correctly identifying an aneuploidy state).

**Figure 7** is a plot of two different populations of scores. The x-axis is score and the y-axis is frequency. Scores on samples of chromosomes without aneuploidy can have a distribution shown in **Figure 7A. Figure 7B** illustrates a hypothetical distribution of a population of scores on samples with an amplified chromosome. Without loss of generality, the graphs and equations show the case of a univariate score where the aneuploidy condition represents an amplification of copy number. Multivariate cases and/or reduction/deletion abnormalities are simple extensions or rearrangements of the given descriptions and are intend to fall within the scope of this art.

The amount of "overlap" between the populations can determine how well normal and aneuploidy cases can be discriminated. In general, increasing fetal fraction, ff, increases discrimination power by separating the two population centers (by moving "C2," the "Center of Aneuploidy Scores", and increasing "d," causing the populations to overlap less. Furthermore, an increase in the absolute value of the magnitude, m, (for example having four copies of the chromosome instead of a trisomy) of the amplification will also increase separation of population centers leading to higher power (i.e. higher probability of correctly identifying aneuploidy states).

Increasing the number of sequences generated, N, reduces standard deviations "sdevA" and/or "sdevB," the spread of the two populations of scores, which also causes the populations to overlap less.

### Setting Thresholds and Estimating Sample Size

The following procedure can be used to set "c", the critical value for calling "aneuploidy", "normal", or "marginal" (uncertain) states. Without loss of generality, one sided statistical tests are used below.

First, an acceptable false positive rate, FP (sometimes also called "type I error" or "specificity"), is decided, which is the probability of a false positive or falsely calling aneuploidy. For example, FP can be at least, or about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, or 0.1.

Second, the value of "c" can be determined by solving the equation: FP = integral from c to infinity of (fl (x)dx).

$$\mathrm{FP} = \int_{c}^{\infty} f1(x)dx \qquad \text{(Equation 1)}$$

Once a critical value, c, has been determined, the minimum number sequences required to achieve a certain TP = True positive rate can be estimated. The true positive rate can be, for example, about 0.5, 0.6, 0.7, 0.8, or 0.9. In one embodiment, the true positive rate can be 0.8. In other words, N is the minimum number of sequences required to identify aneuploidy 100*TP percent of the time. N = minimum number such that TP = integral from c to infinity of f2(x,ff)dx > 0.8. N is determined by solving

$$\min_{N} s.t.\{TB \geq \int_{c}^{\infty} f2(x,N)dx\} \qquad \text{(Equation 2)}$$

In classical statistical tests f1 and f2 are often F, non-central F distributions (a special case of t and non-central t distributions) although that is not a necessary condition for this application.

### Setting "Levels" of Thresholds to Give More Control of Errors

Thresholds can also be set in stages using the above methods. For example, a threshold can be set for high confidence calling of "aneuploidy", say ca, using FP 0.001 and a "marginal" threshold, say cb, using FP 0.05. In this case if Score, S:

| | |
|---|---|
| (S > ca) | then call "Trisomy" |
| (cb > S <= ca) | then call "Marginal" |
| (S < cb) | then call "Normal" |

### Some Trivial Generalizations Falling Within Scope of this Art

Different values for thresholds such as TP, FP, etc can be used. Procedures can be run in any order. For example, one can start with N and solve for c, etc. Distributions can depend on ff so that f1(x,N,ff), f2(x,N,ff), and/or other variables. The above integral equations can be solved by reference to tables or by iterative computer methods. A non-centrality parameter can be estimated and power can be read from standard statistical tables. Statistical power and sample sizes may be derived from calculation or estimation of expected mean squares. Closed form theoretical distributions such as f, t, non-central t, normal, etc. or estimates (kernel or other) can be used to model the distributions f1, f2. Empirical threshold setting and parameter selection using Receiver Operator Characteristic Curves (ROC) can be used and collated with fetal fraction. Various estimates of distribution spread (variance, mean absolute deviation, inter quartile range, etc.) may be used. Various estimates of distribution center (mean, median, etc.) can be used. Two sided as opposed to one sided statistical tests can be used. The simple hypothesis test can be reformulated as linear or non-linear regression. Combinatorial methods, simulation (e.g., monte carlo), maximization (e.g., expectation maximization), iterative, or other methods can be used independently or in conjunction with the above to establish statistical power or thresholds.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention.

## Claims

1. A method for determining the fraction of fetal nucleic acids in a maternal blood sample comprising a mixture of fetal and maternal genomic DNA, wherein said fetal and maternal genomic DNA is cell-free DNA (cfDNA), said method comprising:

   (a) amplifying a plurality of polymorphic target nucleic acids in said mixture,
   wherein each amplified polymorphic target nucleic acid comprises at least one polymorphic site, using primer pairs each capable of amplifying a target nucleic acid sequence comprising a polymorphic site in a multiplex PCR reaction, to generate a panel of amplified polymorphic sites that contains a sufficient number of polymorphic sites such that at least 25 are informative polymorphic sites;
   (b) performing massively parallel sequencing of at least a portion of the amplified product obtained in (a), wherein said sequencing comprises providing a plurality of sequence tags for said panel of amplified polymorphic sites; and
   (c) using said plurality of sequence tags to:

      (i) identify at least one informative polymorphic site in said panel of amplified polymorphic sites, wherein said at least one informative polymorphic site is identified by the difference in the allelic sequences at each polymorphic site and the number of sequence tags mapped to each of the possible alleles at each polymorphic site, and
      (ii) determine said fraction of fetal nucleic acids by using the total number of tags that map to a first allele at an identified informative polymorphic site and the total number of tags that map to a second allele at the identified informative polymorphic site to calculate said fraction of fetal nucleic acids.

2. The method of Claim 1, wherein each of said plurality of polymorphic target nucleic acids comprises at least one single nucleotide polymorphism (SNP).

3. The method of Claim 1, wherein each of said plurality of polymorphic target nucleic acid comprises at least one short tandem repeat (STR).

4. The method of Claim 1, wherein said massively parallel sequencing is:

   (a) sequencing-by-synthesis with reversible dye terminators; or
   (b) sequencing-by-ligation.

5. The method of Claim 1, wherein said sequencing:

   (a) is single molecule sequencing; or
   (b) comprises an amplification.

6. The method of Claim 1, wherein said maternal sample is plasma or serum.

7. The method of Claim 2, wherein said at least one SNP is a single SNP selected from rs560681, ras1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022.

8. The method of Claim 2, wherein said at least one SNP is a tandem SNP selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989- rs13047336; rs987980- rs987981; rs4143392-rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102- rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs1 1088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736;

rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672.

**9.** The method of Claim 3, wherein said at least one STR is selected from CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113.

**10.** The method of Claim 1, wherein said method is a fetal gender-independent method.

**11.** The method of Claim 1, wherein said plurality of polymorphic nucleic acids are located on a:

(a) plurality of different chromosomes; or
(b) chromosome other than chromosome 13, 18, 21, X or Y.

**12.** The method of Claim 1, wherein said plurality of polymorphic nucleic acids comprises at least 25, at least 30 or at least 40 polymorphic sites.

**13.** The method of Claim 1, wherein said panel of amplified polymorphic sites contains a sufficient number of polymorphic sites such that at least 30, or at least 40, are informative polymorphic sites.

**14.** The method of any preceding claim, wherein said method is combined with:

(a) a method used to determine the presence or absence of fetal aneuploidy in the maternal blood sample; or
(b) an assay for determining the presence or absence of a prenatal condition associated with the fetus.

**15.** The method of claim 14(a), wherein said fetal aneuploidy is a monosomy or trisomy.

**Patentansprüche**

**1.** Verfahren zur Bestimmung der Fraktion von fetalen Nucleinsäuren in einer maternalen Blutprobe, umfassend eine Mischung aus fetaler und maternaler genomer DNA, wobei die fetale und maternale genome DNA zellfreie DNA (cfDNA) ist, wobei das Verfahren umfasst:

(a) Amplifizieren einer Vielzahl von polymorphen Zielnucleinsäuren in der Mischung, wobei jede amplifizierte polymorphe Zielnucleinsäure mindestens eine polymorphe Stelle umfasst, unter Verwendung von Primerpaaren, die jeweils in der Lage sind, eine Zielnucleinsäuresequenz, die eine polymorphe Stelle umfasst, in einer Multiplex-PCR-Reaktion zu amplifizieren, um eine Auswahl von amplifizierten polymorphen Stellen zu generieren, die eine ausreichende Anzahl an polymorphen Stellen enthält, so dass mindestens 25 informative polymorphe Stellen sind;
(b) Durchführen von massiver Parallelsequenzierung von mindestens einem Teil des in (a) erhaltenen amplifizierten Produkts, wobei das Sequenzieren das Bereitstellen einer Vielzahl von Sequenz-Tags für die Auswahl von amplifizierten polymorphen Stellen umfasst;
(c) Verwenden der Vielzahl von Sequenz-Tags zum:

(i) Identifizieren von mindestens einer informativen polymorphen Stelle in der Auswahl der amplifizierten polymorphen Stellen, wobei die mindestens eine informative polymorphe Stelle durch den Unterschied in den allelen Sequenzen von jeder polymorphen Stelle und die Anzahl der Sequenz-Tags, die jedem der möglichen Allelen von jeder polymorphen Stelle zugeordnet sind, identifiziert wird, und
(ii) Bestimmen der Fraktion der fetalen Nucleinsäuren, indem die Gesamtanzahl der Tags, die einem ersten Allel an einer identifizierten informativen polymorphen Stelle zugeordnet sind, und die Gesamtanzahl der Tags, die einem zweiten Allel an der identifizierten informativen polymorphen Stelle zugeordnet sind, verwendet werden, um die Fraktion der fetalen Nucleinsäuren zu berechnen.

**2.** Verfahren nach Anspruch 1, wobei jede der Vielzahl von polymorphen Zielnucleinsäuren mindestens einen Einzelnucleotidpolymorphismus (SNP) umfasst.

3. Verfahren nach Anspruch 1, wobei jede der Vielzahl von polymorphen Zielnucleinsäuren mindestens ein Short Tandem-Repeat (STR) umfasst.

4. Verfahren nach Anspruch 1, wobei die massive Parallelsequenzierung

(a) Sequenzierung-durch-Synthese mit reversiblen Farbstoffterminatoren oder
(b) Sequenzierung-durch-Ligation ist.

5. Verfahren nach Anspruch 1, wobei die Sequenzierung

(a) Einzelmolekülsequenzierung ist oder
(b) eine Amplifizierung umfasst.

6. Verfahren nach Anspruch 1, wobei die maternale Probe Plasma oder Serum ist.

7. Verfahren nach Anspruch 2, wobei das mindestens eine SNP ein Einzel-SNP ausgewählt aus rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005 und rs530022 ist.

8. Verfahren nach Anspruch 2, wobei das mindestens eine SNP ein Tandem-SNP ausgewählt aus den Tandem-SNP-Paaren rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980-rs987981; rs4143392-rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959-rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297 und rs2837381-rs4816672 ist.

9. Verfahren nach Anspruch 3, wobei das mindestens eine STR ausgewählt ist aus CSF1P0, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627 und D1GATA113.

10. Verfahren nach Anspruch 1, wobei das Verfahren ein vom Geschlecht des Fetus unabhängiges Verfahren ist.

11. Verfahren nach Anspruch 1, wobei die Vielzahl von polymorphen Nucleinsäuren

(a) auf einer Vielzahl von unterschiedlichen Chromosomen oder
(b) auf einem anderen Chromosom als Chromosom 13, 18, 21, X oder Y lokalisiert ist.

12. Verfahren nach Anspruch 1, wobei die Vielzahl der polymorphen Nucleinsäuren mindestens 25, mindestens 30 oder mindestens 40 polymorphe Stellen umfasst.

13. Verfahren nach Anspruch 1, wobei die Auswahl an amplifizierten polymorphen Stellen eine ausreichende Anzahl an polymorphen Stellen enthält, so dass mindestens 30 oder mindestens 40 informative polymorphe Stellen sind.

14. Verfahren nach einem der vorhergehenden Ansprüche, wobei das Verfahren kombiniert wird mit

(a) einem Verfahren, das zur Bestimmung der Anwesenheit oder Abwesenheit von fetaler Aneuploidie in der maternalen Blutprobe verwendet wird, oder
(b) einem Assay zur Bestimmung der Anwesenheit oder Abwesenheit eines pränatalen Zustands, der mit dem Fetus im Zusammenhang steht.

**15.** Verfahren nach Anspruch 14(a), wobei die fetale Aneuploidie eine Monosomie oder Trisomie ist.

## Revendications

**1.** Procédé de détermination de la fraction d'acides nucléiques foetaux dans un échantillon de sang maternel comprenant un mélange d'ADN génomique fatal et maternel, dans lequel ledit ADN génomique fatal et maternel est de l'ADN sans cellules (ADNcf), ledit procédé comprenant .

(a) l'amplification d'une pluralité d'acides nucléiques cibles polymorphes dans ledit mélange, chaque acide nucléique cible polymorphe amplifié comprenant au moins un site polymorphe, au moyen de paires d'amorces, chacune étant capable d'amplifier une séquence d'acide nucléique cible comprenant un site polymorphe dans une réaction de PCR multiplexe, afin de générer un panel de sites polymorphes amplifiés qui contient un nombre suffisant de sites polymorphes tel qu'au moins 25 qui sont des sites polymorphes informatifs ;
(b) la conduite d'un séquençage massif en parallèle d'au moins une partie du produit amplifié obtenu dans (a), ledit séquençage comprenant la fourniture d'une pluralité d'étiquettes de séquence pour ledit panel de sites polymorphes amplifiés ; et
(c) l'utilisation de ladite pluralité d'étiquettes de séquence pour :

(i) identifier au moins un site polymorphe informatif dans ledit panel de sites polymorphes amplifiés, ledit au moins un site polymorphe informatif étant identifié par la différence des séquences alléliques à chaque site polymorphe et du nombre d'étiquettes de séquence associée à chacun des allèles possibles à chaque site polymorphe, et
(ii) déterminer ladite fraction d'acides nucléiques foetaux au moyen du nombre total d'étiquettes qui correspondent à un premier allèle à un site polymorphe informatif identifié et le nombre total d'étiquettes qui correspondent à un deuxième allèle au site polymorphe informatif identifié pour calculer ladite fraction d'acides nucléiques foetaux.

**2.** Procédé de la revendication 1, dans lequel chacun de ladite pluralité d'acides nucléiques cibles polymorphes comprend au moins un polymorphisme de simple nucléotide (SNP).

**3.** Procédé de la revendication 1, dans lequel chacun de ladite pluralité d'acides nucléiques cibles polymorphes comprend au moins une répétition en tandem courte (STR).

**4.** Procédé de la revendication 1, dans lequel ledit séquençage massif en parallèle est :

(a) un séquençage par synthèse avec des terminateurs de colorant réversible ; ou
(b) un séquençage par ligature.

**5.** Procédé de la revendication 1, dans lequel ledit séquençage :

(a) est un séquençage de molécule unique ; ou
(b) comprend une amplification.

**6.** Procédé de la revendication 1, dans lequel ledit échantillon maternel est du plasma ou du sérum.

**7.** Procédé de la revendication 2, dans lequel ledit au moins un SNP est un SNP unique choisi parmi rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005 et rs530022.

**8.** Procédé de la revendication 2, dans lequel ledit au moins un SNP est un SNP en tandem choisi parmi les paires de SNP en tandem rs7277033-rs2110153 ; rs2822654-rs1882882 ; rs368657-rs376635 ; rs2822731-rs2822732 ;

rs1475881-rs7275487 ; rs1735976-rs2827016 ; rs447340-rs2824097 ; rs418989-rs13047336 ; rs987980-rs987981 ; rs4143392-rs4143391 ; rs1691324-rs13050434 ; rs11909758-rs9980111 ; rs2826842-rs232414 ; rs1980969-rs1980970 ; rs9978999-rs9979175 ; rs1034346-rs12481852 ; rs7509629-rs2828358 ; rs4817013-rs7277036 ; rs9981121-rs2829696 ; rs455921-rs2898102 ; rs2898102-rs458848 ; rs961301-rs2830208 ; rs2174536-rs458076 ; rs11088023-rs11088024 ; rs1011734-rs1011733 ; rs2831244-rs9789838 ; rs8132769-rs2831440 ; rs8134080-rs2831524 ; rs4817219-rs4817220 ; rs2250911-rs2250997 ; rs2831899-rs2831900 ; rs2831902-rs2831903 ; rs11088086-rs2251447 ; rs2832040-rs11088088 ; rs2832141-rs2246777 ; rs2832959-rs9980934 ; rs2833734-rs2833735 ; rs933121-rs933122 ; rs2834140-rs12626953 ; rs2834485-rs3453 ; rs9974986-rs2834703 ; rs2776266-rs2835001 ; rs1984014-rs1984015 ; rs7281674-rs2835316 ; rs13047304-rs13047322 ; rs2835545-rs4816551 ; rs2835735-rs2835736 ; rs13047608-rs2835826 ; rs2836550-rs2212596 ; rs2836660-rs2836661 ; rs465612-rs8131220 ; rs9980072-rs8130031 ; rs418359-rs2836926 ; rs7278447-rs7278858 ; rs385787-rs367001 ; rs367001-rs386095 ; rs2837296-rs2837297 ; et rs2837381-rs4816672.

**9.** Procédé de la revendication 3, dans lequel ladite au moins une STR est choisie parmi CSF1PO, FGA, TH01, vWA, D3S1358, D5S818, D7S820, D8S1179, D13S317, D16S539, D18S51, D21S11, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, et D1GATA113.

**10.** Procédé de la revendication 1, **caractérisé en ce que** ledit procédé est un procédé indépendant du sexe fatal.

**11.** Procédé de la revendication 1, dans lequel ladite pluralité d'acides nucléiques polymorphes sont situés suer :

(a) une pluralité de chromosomes différents ; ou
(b) un chromosome autre que le chromosome 13, 18, 21, X ou Y.

**12.** Procédé de la revendication 1, dans lequel ladite pluralité d'acides nucléiques polymorphes comprend au moins 25, au moins 30 ou au moins 40 sites polymorphes.

**13.** Procédé de la revendication 1, dans lequel ledit panel de sites polymorphes amplifiés contient un nombre suffisant de sites polymorphes tels qu'au moins 30, ou au moins 40, qui sont des sites polymorphes informatifs.

**14.** Procédé de l'une quelconque des revendications précédentes, **caractérisé en ce que** ledit procédé est combiné avec :

(a) un procédé utilisé pour déterminer la présence ou l'absence d'aneuploïdie foetale dans l'échantillon de sang maternel ; ou
(b) un essai pour déterminer la présence ou l'absence d'une affection prénatale associée au foetus.

**15.** Procédé de la revendication 14(a), dans lequel ladite aneuploïdie foetale est une monosomie ou une trisomie.

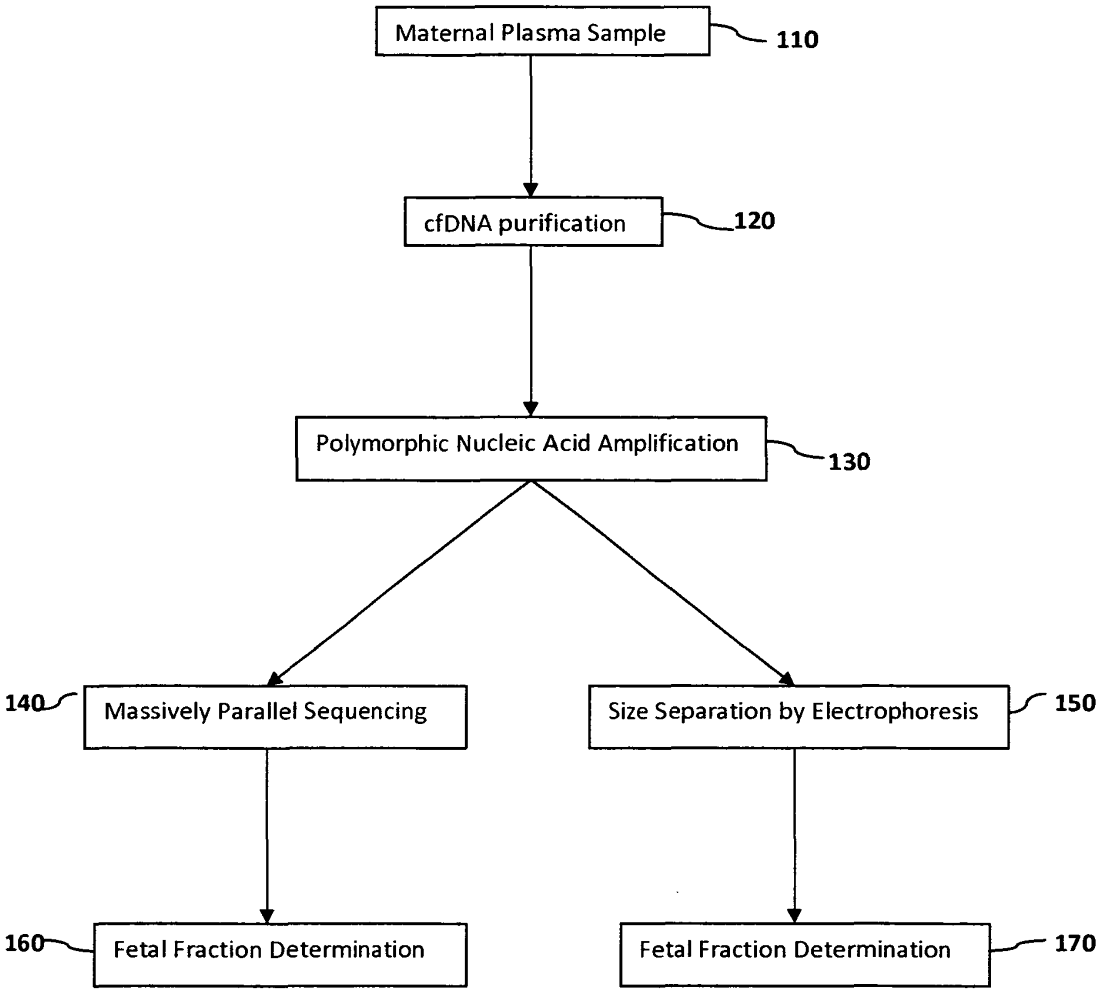
Maternal Plasma Sample
110
cfDNA purification
120
Polymorphic Nucleic Acid Amplification
130
140
Massively Parallel Sequencing
Size Separation by Electrophoresis
150
160
Fetal Fraction Determination
Fetal Fraction Determination
170

FIGURE 1

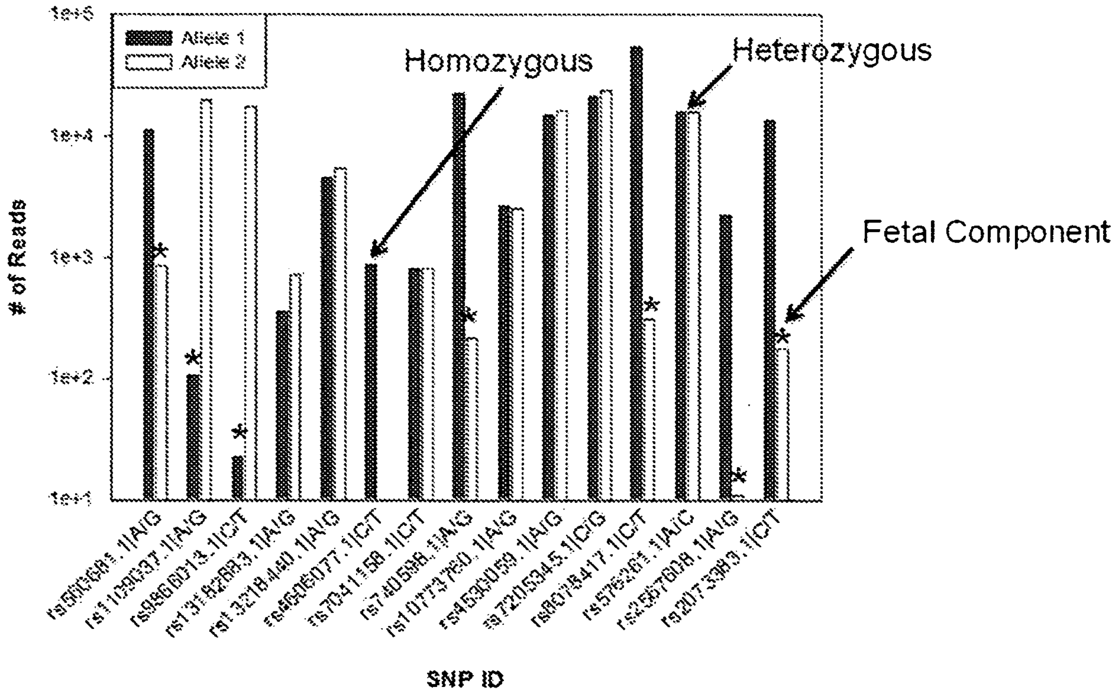
Allele 1
Allele 2
Homozygous
Heterozygous
Fetal Component
1e+5
1e+4
1e+3
1e+2
1e+1
# of Reads
SNP ID

FIGURE 2

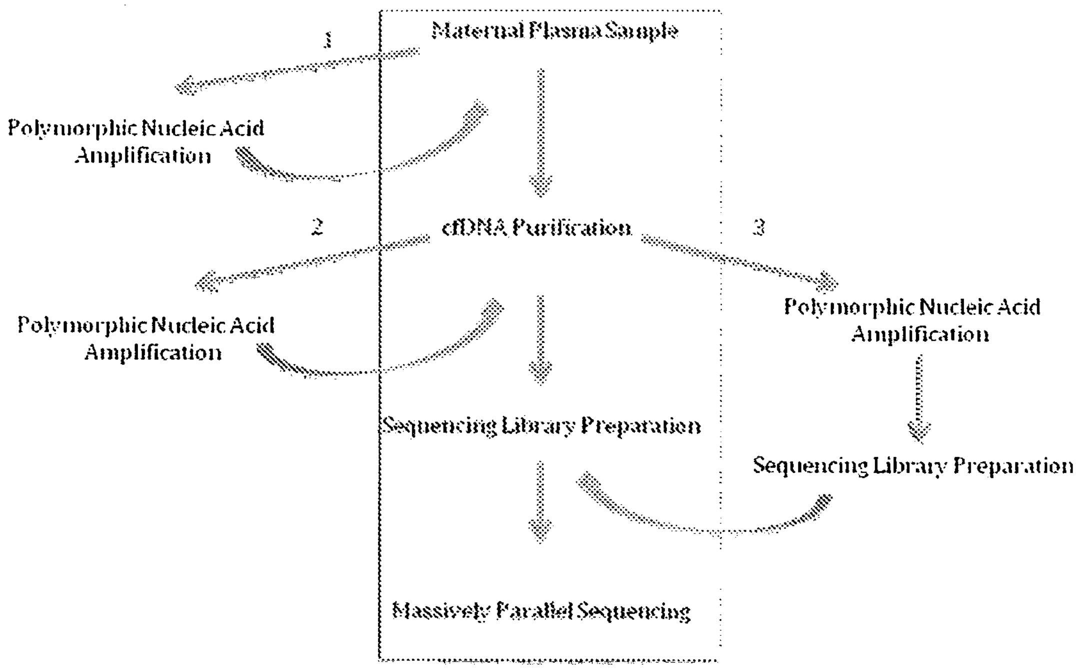
1
Maternal Plasma Sample
Polymorphic Nucleic Acid
Amplification
2
cfDNA Purification
3
Polymorphic Nucleic Acid
Amplification
Polymorphic Nucleic Acid
Amplification
Sequencing Library Preparation
Sequencing Library Preparation
Massively Parallel Sequencing

FIGURE 3

| Amplified Loci: Locus Designation | Chromosome Location | Alleles Included in Identifiler Allelic Ladder | Dye Label | Control DNA 9947A |
|---|---|---|---|---|
| D8S1179 | 8 | 8, 9 10, 11, 12, 13, 14, 15, 16, 17, 18, 19 | 6-FAM | 13a |
| D21S11 | 21q11.2-q21 | 24, 24.2, 25, 26, 27, 28, 28.2, 29, 29.2, 30, 30.2, 31, 31.2, 32, 32.2, 33, 33.2, 34, 34.2, 35, 35.2, 36, 37, 38 | | 30b |
| D7S820 | 7q11.21-22 | 6, 7, 8, 9, 10, 11, 12, 13, 14, 15 | | 10, 11 |
| CSF1PO | 5q33.3-34 | 6, 7, 8, 9, 10, 11, 12, 13, 14, 15 | | 10, 12 |
| D3S1358 | 3p | 12, 13, 14, 15, 16, 17, 18, 19 | VIC | 14, 15 |
| TH01 | 11p15.5 | 4, 5, 6, 7, 8, 9, 9.3, 10, 11, 13.3 | | 8, 9.3 |
| D13S317 | 13q22-31 | 8, 9, 10, 11, 12, 13, 14, 15 | | 11c |
| D16S539 | 16q24-qter | 5, 8, 9, 10, 11, 12,13, 14, 15 | | 11, 12 |
| D2S1338 | 2q35-37.1 | 15, 16, 17, 18, 19, 20, 21, 22, 23, 24, 25, 26, 27, 28 | | 19, 23 |
| D19S433 | 19q12-13.1 | 9, 10, 11, 12, 12.2, 13, 13.2, 14, 14.2, 15, 15.2, 16, 16.2, 17, 17.2 | NED | 14, 15 |
| vWA | 12p12-pter | 11,12, 13, 14, 15, 16, 17, 18, 19, 20, 21, 22, 23, 24 | | 17, 18 |
| TPOX | 2p23-2per | 6, 7, 8, 9, 10, 11, 12, 13 | | 8d |
| D18S51 | 18q21.3 | 7, 9, 10, 10.2, 11, 12, 13, 13.2, 14, 14.2, 15, 16, 17, 18, 19, 20, 21, 22, 23, 24, 25, 26, 27 | | 15, 19 |
| Amelogenin | X: p22.1-22.3<br>Y: p11.2 | X, Y | PET | X |
| D5S818 | 5q21-31 | 7, 8, 9, 10, 11, 12, 13, 14, 15, 16 | | 11e |
| FGA | 4q28 | 17, 18, 19, 20, 21, 22, 23, 24, 25, 26, 26.2, 27, 28, 29, 30, 30.2, 31.2, 32.2, 33.2, 42.2, 43.2, 44.2, 45.2, 46.2, 47.2, 48.2, 50.2, 51.2 | | 23, 24 |

## FIGURE 4

| Amplified Loci: Locus Designation | Chromosome Location | Alleles Included in Identifiler Allelic Ladder | Dye Label | Control DNA 9947A |
|---|---|---|---|---|
| D8S1179 | 8 | 8, 9 10, 11, 12, 13, 14, 15, 16, 17, 18, 19 | 6-FAM | 13a |
| D21S11 | 21q11.2-q21 | 24, 24.2, 25, 26, 27, 28, 28.2, 29, 29.2, 30, 30.2, 31, 31.2, 32, 32.2, 33, 33.2, 34, 34.2, 35, 35.2, 36, 37, 38 | | 30b |
| D7S820 | 7q11.21-22 | 6, 7, 8, 9, 10, 11, 12, 13, 14, 15 | | 10, 11 |
| CSF1PO | 5q33.3-34 | 6, 7, 8, 9, 10, 11, 12, 13, 14, 15 | | 10, 12 |
| D3S1358 | 3p | 12, 13, 14, 15, 16, 17, 18, 19 | VIC | 14, 15 |
| TH01 | 11p15.5 | 4, 5, 6, 7, 8, 9, 9.3, 10, 11, 13.3 | | 8, 9.3 |
| D13S317 | 13q22-31 | 8, 9, 10, 11, 12, 13, 14, 15 | | 11c |
| D16S539 | 16q24-qter | 5, 8, 9, 10, 11, 12,13, 14, 15 | | 11, 12 |
| D2S1338 | 2q35-37.1 | 15, 16, 17, 18, 19, 20, 21, 22, 23, 24, 25, 26, 27, 28 | | 19, 23 |
| D19S433 | 19q12-13.1 | 9, 10, 11, 12, 12.2, 13, 13.2, 14, 14.2, 15, 15.2, 16, 16.2, 17, 17.2 | NED | 14, 15 |
| vWA | 12p12-pter | 11,12, 13, 14, 15, 16, 17, 18, 19, 20, 21, 22, 23, 24 | | 17, 18 |
| TPOX | 2p23-2per | 6, 7, 8, 9, 10, 11, 12, 13 | | 8d |
| D18S51 | 18q21.3 | 7, 9, 10, 10.2, 11, 12, 13, 13.2, 14, 14.2, 15, 16, 17, 18, 19, 20, 21, 22, 23, 24, 25, 26, 27 | | 15, 19 |
| Amelogenin | X: p22.1-22.3<br>Y: p11.2 | X, Y | PET | X |
| D5S818 | 5q21-31 | 7, 8, 9, 10, 11, 12, 13, 14, 15, 16 | | 11e |
| FGA | 4q28 | 17, 18, 19, 20, 21, 22, 23, 24, 25, 26, 26.2, 27, 28, 29, 30, 30.2, 31.2, 32.2, 33.2, 42.2, 43.2, 44.2, 45.2, 46.2, 47.2, 48.2, 50.2, 51.2 | | 23, 24 |

**FIGURE 5**

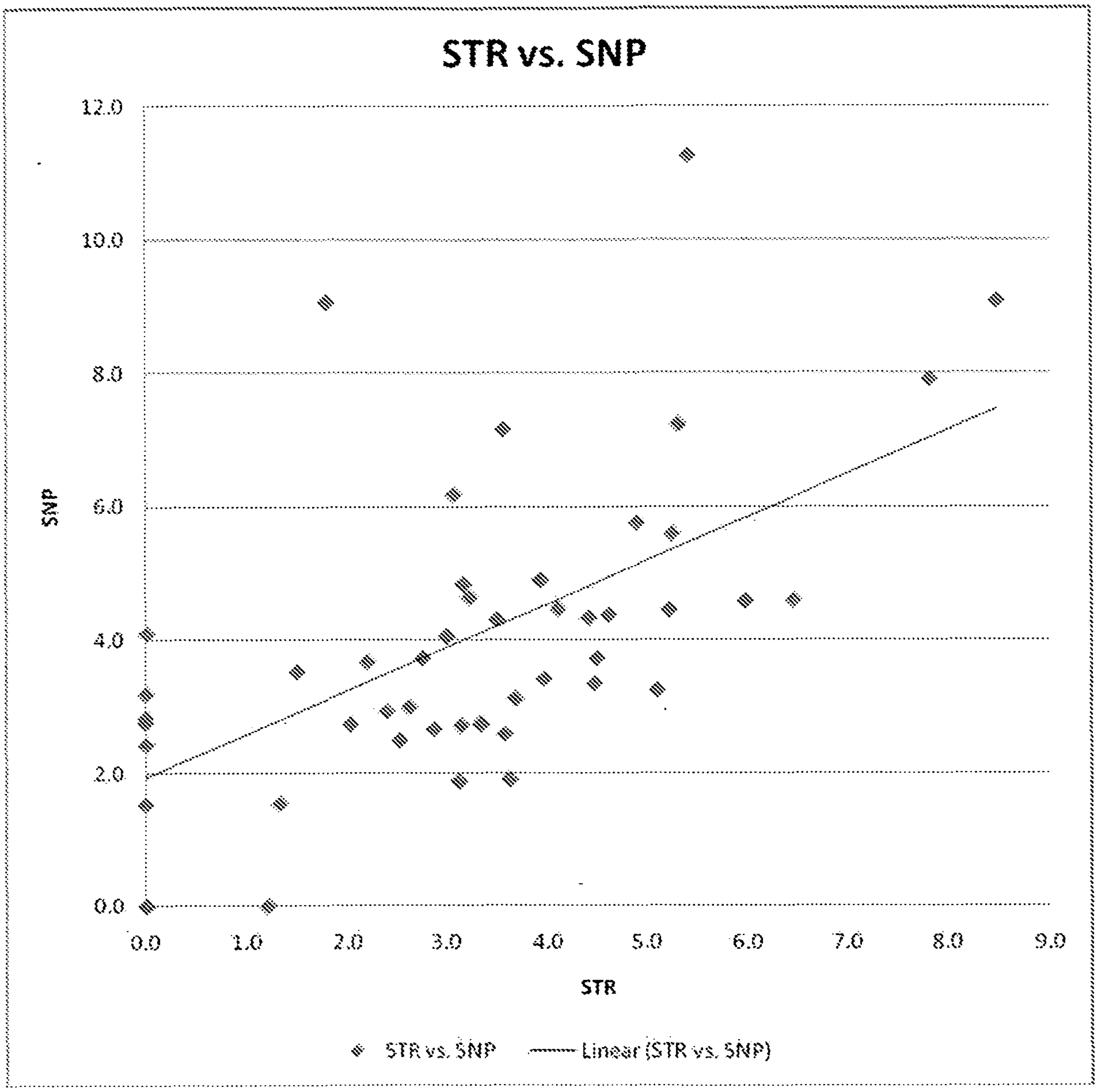
STR vs. SNP
12.0
10.0
8.0
6.0
4.0
2.0
0.0
SNP
0.0
1.0
2.0
3.0
4.0
5.0
6.0
7.0
8.0
9.0
STR
STR vs. SNP
Linear (STR vs. SNP)

FIGURE 6

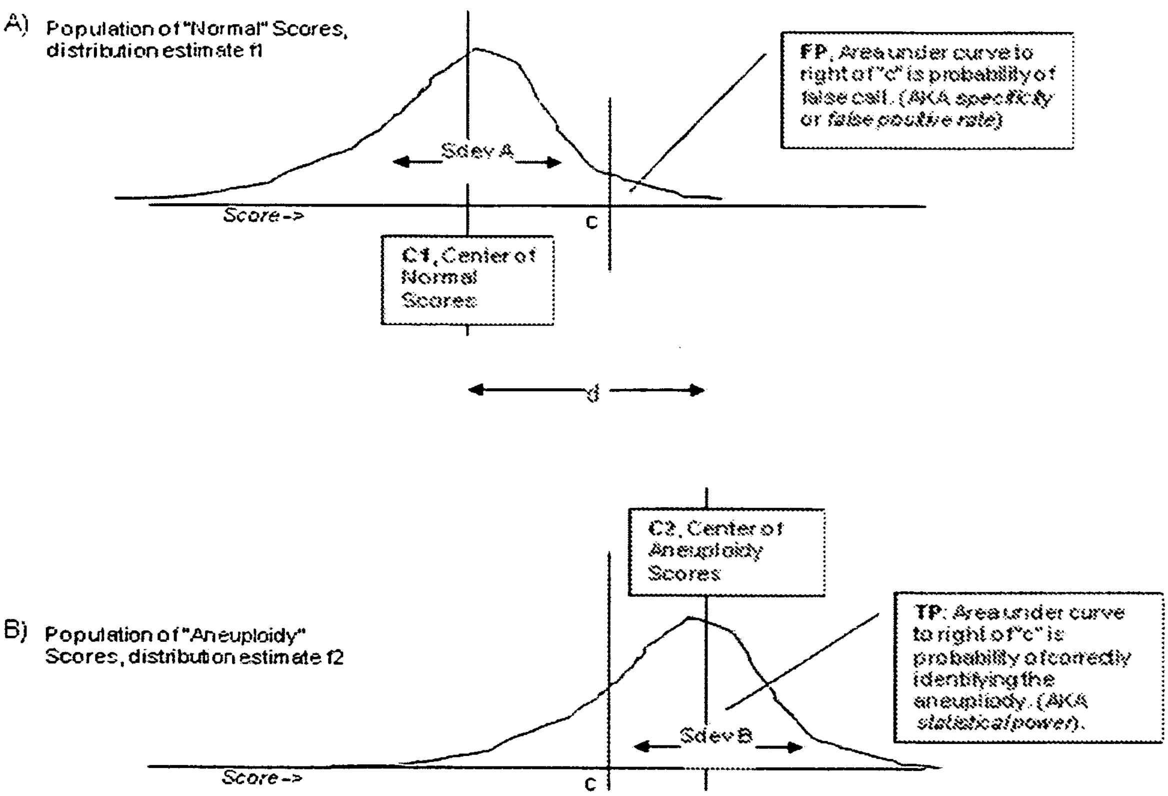
A) Population of "Normal" Scores, distribution estimate f1
FP, Area under curve to right of "c" is probability of false call. (AKA specificity or false positive rate)
Sdev A
Score->
c
C1, Center of Normal Scores
d
C2, Center of Aneuploidy Scores
B) Population of "Aneuploidy" Scores, distribution estimate f2
TP: Area under curve to right of "c" is probability of correctly identifying the aneuploidy. (AKA statistical power).
Sdev B
Score->
c

**FIGURE 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20080070792 A
- US 20050282293 A
- US 20050224351 A
- US 20050065735 A
- US 20090280492 A
- US 20080113358 A
- US 20080026390 A
- US 20080050739 A
- US 20080220422 A
- US 20080138809 A
- US 20090026082 A
- WO 2009046445 A
- US 20070202525 A1
- US 20100112575 A1
- US 20090087847 A1
- US 20090029377 A1
- US 20080220422 A1
- US 20080138809 A1
- US 20080153090 A1
- US 7645576 B
- US 20100112590 A1
- US 20090162842 A1
- US 20070207466 A1
- US 20010051341 A1
- US 61296358 B
- US 61360837 B
- US 61407017 A
- US 61455849778 A
- US 61455849 A
- US 61455849 B


### Non-patent literature cited in the description


- **HUANG et al.** *Methods in Molecular Biology,* 2008, vol. 444, 203-208
- **BIANCHI et al.** *Am J Hum Genet,* 1997, vol. 61, 822-829
- **BABOCHKINA et al.** *Haematologica,* 2005, vol. 90, 740-745
- **WRIGHT C.F. ; BURTON H.** *Human Reproduction Update,* 2009, vol. 15 (1), 139-151
- **LO et al.** *Clin Chem,* 1999, vol. 45, 1747-1751
- **DAHLLAN et al.** *Lancet,* 2007, vol. 369, 474-481
- **LI et al.** *Clin Chem,* 2004, 1002-1011
- **FAN et al.** *Proc Natl Acad Sci,* 2008, vol. 105, 16266-16271
- **KOIDE et al.** *Prenatal Diagnosis,* 2005, vol. 25, 604-607
- **CHEN et al.** *Nature Med.,* 1996, vol. 2, 1033-1035
- **LO et al.** *Lancet,* 1997, vol. 350, 485-487
- **PAKSTIS.** *Hum Genet,* 2010, vol. 127, 315-324
- *Nature,* 2003, vol. 426, 789-796
- **PERTL et al.** *Human Genetics,* 2002, vol. 106, 45-49
- **LIU et al.** *Acta Obset Gyn Scand,* 2007, vol. 86, 535-541
- **CHAN et al.** *Clin Chem,* 2004, vol. 50, 8892
- **LI et al.** *Clin Chem,* 2004, vol. 50, 1002-1011
- **BUTLER et al.** *J Forensic Sci,* 2003, vol. 48, 1054-1064
- **COBLE ; BUTLER.** *J Forensic Sci,* 2005, vol. 50, 43-53
- **SULLIVAN et al.** *BioTechniques,* 1993, vol. 15, 637-641
- **VOLKERDING et al.** *Clin Chem,* 2009, vol. 55, 641-658
- **METZKER M.** *Nature Rev,* 2010, vol. 11, 31-46
- **FAN et al.** *Proc Natl Acad Sci U S A,* 2008, vol. 105, 16266-16271
- **CHIU et al.** *Proc Natl Acad Sci U S A,* 2008, vol. 105, 20458-20463
- **HARRIS T.D. et al.** *Science,* 2008, vol. 320, 106-109
- **MARGULIES, M. et al.** *Nature,* 2005, vol. 437, 376-380
- **SONI GV ; MELLER A.** *Clin Chem,* 2007, vol. 53, 1996-2001
- **BENTLEY et al.** *Nature,* 2009, vol. 6, 53-59
- **LANGMEAD et al.** *Genome Biology,* 2009, vol. 10, R25.1-R25.10
- **CHIU et al.** *Clin Chem,* 2010, vol. 56, 459-463
- **PAKSTIS et al.** *Hum Genet,* 2010, vol. 127, 315-324
- *BioTechniques.RTM. Protocol Guide,* December 2006, 29
- **HANSON ; BALLANTYNE.** *Analytical Biochem,* 2005, vol. 346, 246-257